# DEMANDE DE BREVET EUROPEEN

(11) **EP 1 378 544 A2**
(43) Date de publication de la demande: **07.01.2004**
(21) Numéro de dépôt: 03291678.5
(22) Date de dépôt: 04.07.2003
(51) Int. Cl.: C09B 50/10, C07D 213/77, C07D 233/88, C07D 237/22, C07D 237/20, C07D 401/14, C07D 213/87

(54) **Composés tétraazapentaméthiniques et leur application pour la teinture des fibres kératiniques**

(30) Priorité: 05.07.2002 FR 0208494
(71) Demandeur: L'OREAL, 75008 Paris (FR)
(72) Inventeur: Burgaud, Hervé, 77230 Dammartin en Goele (FR); David, Hervé, 94340 Joinville le Pont (FR); Pereira, Rui, 77600 Bussy Saint Georges (FR); Belcour-Castro, Béatrice, 94340 Joinville le Pont (FR)
(74) Mandataire: Casalonga, Axel

(57) **Abrégé**

La présente invention concerne l'utilisation en tant que colorant direct d'au moins un composé tétraazapentaméthinique, une composition tinctoriale comprenant au moins un tel composé, ainsi qu'un procédé de teinture des fibres kératiniques, en particulier des fibres kératiniques humaines et plus particulièrement des cheveux, dans lequel on applique au moins une composition comprenant un tel composé. L'invention concerne aussi des composés tétraazapentaméthiniques de formule I' :

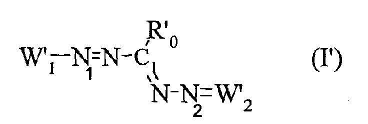

Elle a enfin pour objet un procédé de décoloration de tels composés, ainsi que la décoloration de fibres kératiniques préalablement teintes avec ces derniers.

## Description

La présente invention concerne le domaine de la teinture des fibres kératiniques, elle concerne plus particulièrement l'utilisation en tant que colorant direct d'au moins un composé tétraazapentaméthinique, une composition tinctoriale comprenant au moins un tel composé, ainsi qu'un procédé de teinture des fibres kératiniques dans lequel on applique au moins une composition comprenant un tel composé.

La présente invention concerne encore des nouveaux composés tétraazapentaméthiniques, ainsi que leur procédé de préparation.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, donnent naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

Ce procédé de coloration d'oxydation consiste à appliquer sur les fibres kératiniques, des bases d'oxydation ou un mélange de bases d'oxydation et de coupleurs avec un agent oxydant, par exemple de l'eau oxygénée, à laisser pauser, puis à rincer les fibres. Les colorations qui en résultent sont permanentes, puissantes, et résistantes aux agents extérieurs, notamment à la lumière, aux intempéries, aux lavages, à la transpiration et aux frottements. Généralement mis en oeuvre à pH basique, ce procédé permet d'obtenir une teinture et simultanément un éclaircissement de la fibre qui se traduit en pratique par la possibilité d'obtenir une coloration finale plus claire que la couleur d'origine. En outre, l'éclaircissement de la fibre a pour effet avantageux d'engendrer une couleur unie dans le cas des cheveux gris, et dans le cas de cheveux naturellement pigmentés, de faire ressortir la couleur, c'est-à-dire de la rendre plus visible.

Il est aussi connu de teindre les fibres kératiniques par une coloration directe. Le procédé classiquement utilisé en coloration directe consiste à appliquer sur les fibres kératiniques des colorants directs qui sont des molécules colorées et colorantes ayant une affinité pour les fibres, à laisser pauser, puis à rincer les fibres.

I1 est connu par exemple d'utiliser des colorants directs nitrés benzèniques, anthraquinoniques, nitropyridiniques, azoïques, xanthéniques, acridiniques, aziniques ou triarylméthaniques.

Il en résulte des colorations particulièrement chromatiques qui sont cependant temporaires ou semi-permanentes à cause de la nature des liaisons entre les colorants directs et la fibre kératinique. Ces interactions font que la désorption des colorants de la surface et/ou du coeur de la fibre se fait facilement. Les colorations présentent généralement une faible puissance tinctoriale et une mauvaise tenue aux lavages ou à la transpiration. Ces colorants directs sont en outre généralement sensibles à la lumière car la résistance du chromophore vis-à-vis des attaques photochimiques est faible, ce qui conduit à un affadissement de la coloration des cheveux dans le temps. En outre, la sensibilité de ces colorants à la lumière dépend de leur répartition uniforme ou en aggrégats dans et/ou sur la fibre kératinique.

Il est connu d'utiliser des colorants directs en combinaison avec des agents oxydants. Cependant, les colorants directs sont généralement sensibles à l'action des agents oxydants tels que l'eau oxygénée, ce qui les rendent généralement difficilement utilisables dans les compositions de teinture directe éclaircissante à base d'eau oxygénée et d'un agent alcalinisant, ou dans des compositions de teinture d'oxydation à base de colorants d'oxydation.

Ainsi, il a été proposé dans les demandes de brevets FR-1 584 965 et JP-062 711 435 de teindre les cheveux avec des compositions de teinture à base de colorants directs nitrés et/ou de colorants dispersés azoïques et d'eau oxygénée ammoniacale en appliquant sur les cheveux un mélange desdits colorants et dudit oxydant, réalisé juste avant l'emploi. Mais les colorations obtenues se sont révélées insuffisamment tenaces et disparaissent aux shampooings en laissant apparaître l'éclaircissement de la fibre capillaire. Une telle coloration devient inesthétique en évoluant au cours du temps.

On a également proposé dans les demandes de brevets JP-53 95693 et JP-55 022638 de teindre les cheveux avec des compositions à base de colorants directs cationiques de type oxazine et d'eau oxygénée ammoniacale, en appliquant sur les cheveux, dans une première étape, de l'eau oxygénée ammoniacale, puis dans une seconde étape, une composition à base du colorant direct oxazinique. Cette coloration n'est pas satisfaisante, en raison du fait qu'elle nécessite un procédé rendu trop lent par les temps de pause des deux étapes successives. Si par ailleurs on applique sur les cheveux un mélange extemporané du colorant direct oxazinique avec de l'eau oxygénée ammoniacale, on ne colore pas, ou du moins, on obtient une coloration de la fibre capillaire qui est presque inexistante.

Plus récemment, dans la demande de brevet FR 2 741 798 il a été décrit des compositions tinctoriales contenant des colorants directs azoïques ou azométhiniques comportant au moins un atome d'azote quaternisé, lesdites compositions devant être mélangées extemporanément à pH basique à une composition oxydante. Ces compositions permettent d'obtenir des colorations avec des reflets homogènes, tenaces et brillants. Cependant, elles ne permettent pas de teindre les fibres kératiniques avec autant de puissance qu'avec des compostions de coloration d'oxydation.

Par ailleurs, la demande de brevet FR 1 353 497, qui ne concerne toutefois pas la teinture des fibres kératiniques divulgue des composés tétraazapentaméthiniques substitués par un radical benzimidazole et par un groupement benzimidazolium particulièrement appropriés à la teinture des fibres à base de polymères ou copolymères du nitrile acrylique.

Il existe donc un réel besoin de disposer de colorants directs qui ne présentent pas les inconvénients de ceux de l'art antérieur.

De manière avantageuse et inattendue, la demanderesse a découvert qu'il était possible d'atteindre ce but en utilisant au moins un composé tétraazapentaméthinique monocationique de formule (I).

Ces colorants directs sont chromatiques et permettent de teindre les fibres kératiniques aussi puissamment que les colorants d'oxydation, ils sont aussi stables à la lumière que les colorants d'oxydation, ils sont également résistants aux intempéries, aux lavages et à la transpiration. Ces colorants directs permettent de teindre les fibres kératiniques selon une gamme très large de couleurs, en particulier très chromatiques, sans oublier les nuances dites «fondamentales» comme les noirs et les marrons.

La présente demande a donc pour objet l'utilisation, en tant que colorant direct dans des ou pour la fabrication de compositions tinctoriales pour fibres kératiniques, en particulier pour fibres kératiniques humaines et plus particulièrement des cheveux, d'au moins un composé tétraazapentaméthinique de formule (I) 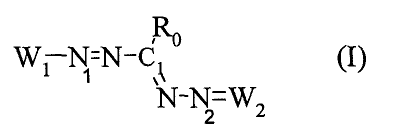 dans laquelle
- W₁ représente un radical hétéroaromatique cationique de formule (II) ou (III) : 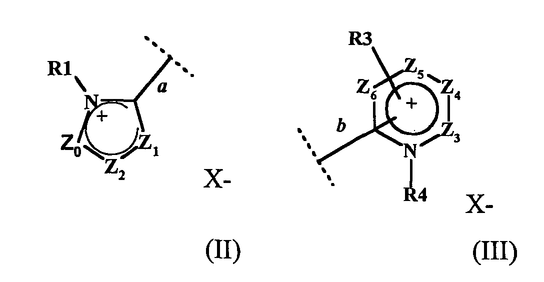
- W₂ représente un radical hétéroaromatique de formule (IV) ou (V): 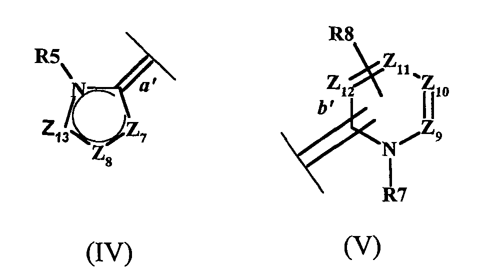
dans lesquelles :
- Z₀ représente un radical CR₂, un atome d'azote ou un radical NR₂₁,
- Z₁ représente un atome d'oxygène, de soufre ou un radical NR₉,
- Z₂ représente un atome d'azote ou un radical CR₁₀,
- Z₃ représente un atome d'azote ou un radical CR₁₁,
- Z₄ représente un atome d'azote ou un radical CR₁₂,
- Z₅ représente un atome d'azote ou un radical CR₁₃,
- Z₆ représente un atome d'azote ou un radical CR₁₄,
- Z₇ représente un atome d'oxygène, de soufre ou un radical NR₁₅,
- Z₈ représente un atome d'azote ou un radical CR₁₆,
- Z₉ représente un atome d'azote ou un radical CR₁₇,
- Z₁₀ représente un atome d'azote ou un radical CR₁₈,
- Z₁₁ représente un atome d'azote ou un radical CR₁₉,
- Z₁₂ représente un atome d'azote ou un radical CR₂₀,
- Z₁₃ représente un radical CR₆, un atome d'azote ou un radical NR₂₂,
- étant entendu que chacun des cycles des formules (II), (III), (IV) et (V) ne comportent pas plus de trois atomes d'azote et que deux des trois atomes d'azote peuvent être contigus,
- la liaison a du radical hétéroaromatique cationique à 5 chaînons de la formule (II) étant reliée à l'atome d'azote N₁ de la formule (I),
- la liaison b du radical hétéroaromatique cationique à 6 chaînons de la formule (III) étant reliée à l'atome d'azote N₁ de la formule (I).
- la double liaison a' du radical hétéroaromatique à 5 chaînons de la formule (IV) étant reliée à l'atome d'azote N₂ de la formule (I),
- la double liaison b' du radical hétéroaromatique à 6 chaînons de la formule (V) étant reliée à l'atome d'azote N₂ de la formule (I),
- la liaison b, reliant le radical hétéroaromatique cationique de la formule III à l'atome d'azote N₁ de la formule (I), étant située en position ortho ou para de l'atome d'azote portant le radical R₄ lorsque Z₅ représente un radical CR₁₃ ; la liaison b étant située en position ortho de l'atome d'azote portant le radical R₄ lorsque Z₅ représente un atome d'azote,
- la liaison b', reliant le radical hétéroaromatique de la formule V à l'atome d'azote N₂ de la formule (I), étant située en position ortho ou para de l'atome d'azote portant le radical R₇ lorsque Z₁₁ représente un radical CR₁₉ ; la liaison b' étant située en position ortho de l'atome d'azote portant le radical R₇ lorsque Z₁₁ représente un atome d'azote,
- R₂, R₆, R₁₀ et R₁₆ représentent, indépendamment l'un de l'autre, un atome d'hydrogène ; un radical alkyle en C₁-C₄ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique ; un radical phényle non substitué ou substitué par un ou deux radicaux choisis parmi les radicaux hydroxy, alcoxy en C₁-C₂, (poly)-hydroxyalcoxy en C₂-C₄, amino, (di)alkylamino en C₁-C₂ ; un radical carboxy ; un radical sulfonylamino,
- R₁, R₄, R₅, R₇, R₉, R₁₅, R₂₁, et R₂₂ représentent, indépendamment l'un de l'autre, un radical alkyle en C₁-C₈ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂ , carboxy ou sulfonique ;
- R₀, R₃, R₈, R₁₁, R₁₂, R₁₃, R₁₄, R₁₇,R₁₈, R₁₉,et R₂₀ représentent, indépendamment l'un de l'autre, un atome d'hydrogène, une chaîne hydrocarbonée en C₁-C₁₆ linéaire ou ramifiée, cette chaîne pouvant être saturée ou insaturée par une à trois insaturations, cette chaîne étant non substituée ou substituée par un à trois radicaux choisis parmi les radicaux hydroxy, alcoxy en C₁-C₂, (poly)-hydroxyalcoxy en C₂-C₄, amino, (di)alkylamino en C₁-C₂ , carboxy, sulfonique, sulfonylamino, (poly)-hydroxyalkylamino en C₂-C₄ ou un atome d'halogène tel que chlore, fluor ou brome ; un radical phényle non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂ , carboxy, sulfonique, sulfonylamino, (poly)-hydroxyalkylamino en C₂-C₄ ou un atome d'halogène tel que chlore, fluor ou brome ; un radical hétéroaryle choisi parmi les radicaux pyrazolyle, pyrrolyle, imidazolyle, thiazolyle, oxazolyle, triazolyle, pyridinyle, pyrimidinyle, triazinyle, pyrazinyle, pyridazinyle, en outre cette chaîne hydrocarbonée peut être interrompue par un ou deux atomes d'oxygène, d'azote, de soufre ou par un radical SO₂,
- étant entendu que R₀, R₃, R₈, R₁₁, R₁₂, R₁₃, R₁₄, R₁₇, R₁₈, R₁₉, et R₂₀ ne comportent pas de liaison peroxyde, ni de radicaux diazo ou nitroso,
- R₂ avec R₁₀, R₁₁ avec R₁₂, R₆ avec R₁₆, et R₁₇ avec R₁₈ pouvant former indépendamment les uns des autres un cycle aromatique carboné à 5 ou 6 chaînons non substitué ou substitué par un ou deux radicaux hydroxy, amino, (di)alkyl (C₁-C₂) amino, alcoxy C₁-C₂, (poly)hydroxyalkyl C₂-C₄ amino,

X est un anion organique ou minéral.

Par "chaîne hydrocarbonée ramifiée" au sens de la présente demande, on entend une chaîne hydrocarbonée ramifiée pouvant également former un à cinq cycles carbonés comportant de 3 à 7 chaînons, cette chaîne pouvant comprendre une à trois insaturations, c'est-à-dire une à trois liaisons double et/ou liaisons triple.

Par "(poly)hydroxyalcoxy en C₂-C₄, on entend un groupement alcoxy en C₂-C₄ substitué par 1 à 2 groupes hydroxy.

Par "(poly)hydroxyalkylamino en C₂-C₄" au sens de la présente invention, on entend un groupe alkylamino substitué par 1 à 2 groupes hydroxy.

L'expression selon laquelle la chaîne hydrocarbonée peut être interrompue par un ou plusieurs atomes d'oxygène, d'azote, de soufre ou par un radical SO₂, ou encore que cette chaîne est insaturée signifie que la chaîne carbonée peut être modifiée de la façon suivante :

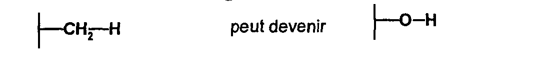

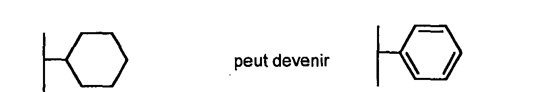

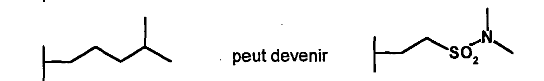

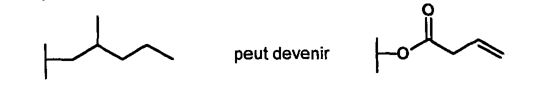

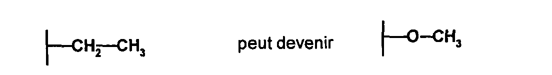

Au sens de la présente demande, un anion organique ou minéral est par exemple choisi parmi un halogénure tel que chlorure, bromure, fluorure, iodure ; un hydroxyde ; un sulfate ; un hydrogénosulfate ; un alkyl(C₁-C₆)sulfate tel que par exemple un méthylsulfate ou un éthylsulfate ; un acétate ; un tartrate ; un oxalate ; un alkyl(C₁-C₆)sulfonate tel que méthylsulfonate ; un arylsulfonate non substitué ou substitué par un radical alkyle en C₁-C₄ tel que par exemple un 4-toluylsulfonate.

De préférence, R₀ représente un atome d'hydrogène ; un radical alkyle en C₁-C₆ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, (poly)hydroxyalkylamino en C₂-C₄, carboxy ou sulfonique ; un radical phényle non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy, sulfonique ou un atome d'halogène tel que chlore, fluor ou brome, un radical hétéroaryle éventuellement cationique choisi parmi les radicaux pyrazolyle, pyrrolyle, imidazolyle, thiazolyle, oxazolyle, triazolyle, pyridinyle, pyrimidinyletriazinyle, pyrazinyle, pyridazinyle.

De manière encore préférée, R₀ représente préférentiellement un atome d'hydrogène ; un radical alkyle en C₁-C₃ linéaire ou ramifié, non substitué ou substitué par un à deux radicaux choisis parmi les radicaux hydroxy, alcoxy en C₁-C₂, amino, (di)alkylamino en C₁-C₂, carboxy ou sulfonique ; un radical phényle non substitué ou substitué par un à deux radicaux choisis parmi les radicaux hydroxy, amino, (di)alkylamino en C₁-C₂ ou (poly)-hydroxyalkylamino en C₂-C₄, un radical hétéroaryle éventuellement cationique choisi parmi les radicaux pyrazolyle, pyrrolyle, imidazolyle, pyridinyle.

Selon un mode de réalisation particulièrement préféré, R₀ représente un atome d'hydrogène ; un méthyle, éthyle, propyle, butyle, 2-hydroxyéthyle , 2-aminoéthyle ;1-carboxyméthyle, 2-carboxyéthyle, 2-sulfonyléthyle, 2-méthoxyéthyle ; un radical phényle non substitué ou substitué par un à deux radicaux choisis parmi les radicaux amino, (di)alkylamino en C₁-C₂, (poly)-hydroxyalkylamino en C₂-C₄, un radical hétéroaryle éventuellement cationique choisi parmi les radicaux pyrazolyle, pyrrolyle, imidazolyle, pyridinyle.

R₀ représente un atome d'hydrogène ; un radical méthyle, éthyle, 2-méthoxyéthyle ; un radical phényle non substitué ou substitué par un radical un amino, (di)méthylamino , (di)(2-hydroxyéthyl)amino, un radical hétéroaryle éventuellement cationique choisi parmi les radicaux imidazolyle, pyridinyle.

R₂, R₆, R₁₀ et R₁₆ représentent préférentiellement un atome d'hydrogène, un radical phényle, un radical alkyle en C₁-C₄ non substitué ou substitué par un à deux radicaux choisis parmi les radicaux hydroxy, amino, (di)alkylamino en C₁-C₂ , carboxy.

Selon un mode de réalisation particulièrement préféré, R₂, R₆, R₁₀ et R₁₆ représentent préférentiellement un atome d'hydrogène, un radical méthyle, phényle, 2-hydroxyméthyle, un carboxy, un radical phényl.

R₁, R₄, R₅, R₇, R₉, R₁₅, R₂₁ et R₂₂ représentent préférentiellement un radical alkyle en C₁-C₄ non substitué ou substitué par un à deux radicaux choisis parmi les radicaux hydroxy, alcoxy en C₁-C₂, amino, (di)alkylamino en C₁-C₂ , carboxy, sulfonique.

Selon un mode de réalisation particulièrement préféré, R₁, R₄, R₅, R₇, R₉, R₁₅, R₂₁ et R₂₂ représentent préférentiellement un radical méthyle, éthyle, 2-hydroxyéthyle, 1-carboxyméthyle, 2- carboxyéthyle, 2-sulfonyléthyle.

R₃, R₈, R₁₁, R₁₂, R₁₃, R₁₄, R₁₇, R₁₈, R₁₉, et R₂₀ représentent, préférentiellement et indépendamment l'un de l'autre, un atome d'hydrogène ; un radical alkyle en C₁-C₄ linéaire ou ramifié, non substitué ou substitué par un à deux radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique; un radical phényle non substitué ou substitué par un à deux radicaux choisis parmi les radicaux hydroxy, alcoxy en C₁-C₂, (poly)-hydroxyalcoxy en C₂-C₄, amino, (di)alkylamino en C₁-C₂, carboxy, sulfonique ou un atome d'halogène tel que chlore, fluor ou brome ; un radical sulfonylamino ; un radical (poly)-hydroxyalkylamino en C₂-C₄,

Plus préférentiellement, R₃, R₈, R₁₁, R₁₂, R₁₃, R₁₄, R₁₇, R₁₈, R₁₉, et R₂₀ représentent un atome d'hydrogène, un radical alkyle en C₁-C₄ non substitué ou substitué par un à deux radicaux choisis parmi les radicaux hydroxy, un radical alcoxy en C₁-C₂ ; un radical amino ; un radical (di)alkylamino en C₁-C₂ ; un radical carboxy ; un radical (poly)-hydroxyalkylamino en C₂-C₄.

Selon un mode de réalisation particulièrement préféré R₃, R₈, R₁₁, R₁₂, R₁₃, R₁₄, R₁₇, R₁₈, R₁₉, et R₂₀ représentent indépendamment l'un de l'autre un atome d'hydrogène, un radical méthyle, 2-hydroxyméthyle, un carboxy, un radical méthoxy, éthoxy, 2-hydroxyéthyloxy, un radical amino, méthylamino, diméthylamino, 2-hydroxyéthylamino.

Les composés de formule (I) utilisés de manière préférée sont ceux pour lesquels dans la formule (I) W₁ est un radical 2-benzimidazolium et W₂ est un radical 2-benzimidazole. De manière encore plus préférée, ce sont les composés suivants :
- Chlorure de 2-[5-(1,3-diméthyl-2-benzimidazolidène)-1-formazano]-1,3-diméthyl-benzimidazolium
- Chlorure de 2-[5-(1,3-diméthyl-2-benzimidazolidène)-3-méthyl-1-formazano]-1,3-diméthyl-benzimidazolium
- Chlorure de 2-[5-(1,3-diméthyl-2-benzimidazolidène)-3-éthyl-1-formazano]-1,3-diméthyl-benzimidazolium
- Chlorure de 2-[5-(1,3-diméthyl-2-benzimidazolidène)-3-isopropyl-1-formazano]-1,3-diméthyl-benzimidazolium
- Chlorure de 2-[5-(1,3-diméthyl-2-benzimidazolidene)-3-phényl-1-formazano]- 1 ,3-diméthyl-benzimidazolium
- Chlorure de 2-[5-(1,3-diméthyl-2-benzimidazolidène)-3-(4'-méthoxyphényl)-1-formazano]-1,3-diméthyl-benzimidazolium
- Chlorure de 2-[5-(1,3-diméthyl-2-benzimidazolidène)-3-(4'-hydroxyphényl)-1-formazano]-1,3-diméthyl-benzimidazolium
- Chlorure de 2-[5-(1,3-diméthyl-2-benzimidazolidène)-3-(4'-N,N-dimethylaminophényl)-1-formazano]-1,3-diméthyl-benzimidazolium
- Chlorure de 2-[5-(1,3-dihydroxyéthyl-2-benzimidazolidène)-1-formazano]-1,3-dihydroxyéthylbenzimidazolium
- Chlorure de 2-[5-(1,3-dihydroxyéthyl-2-benzimidazolidène)-3-méthyl-1-formazano]-1,3-dihydroxyéthylbenzimidazolium
- Chlorure de 2-[5-(1,3-dihydroxyéthyl-2-benzimidazolidène)-3-ethyl-1-formazano]-1,3-dihydroxyéthyl -benzimidazolium
- Chlorure de 2-[5-(1,3-dihydroxyéthyl -2-benzimidazolidène)-3-phenyl-1-formazano]-1,3-dihydroxyéthyl -benzimidazolium
- Chlorure de 2-[5-(1,3-dihydroxyéthyl -2-benzimidazolidène)-3-isopropyl-1-formazano]-1,3-dihydroxyéthyl -benzimidazolium
- Chlorure de 2-[5-(1,3-dihydroxyéthyl-2-benzimidazolidène)-3-(4'-methoxyphényl)-1-formazano]-1,3-dihydroxyéthyl -benzimidazolium
- Chlorure de 2-[5-(1,3-dihydroxyéthyl -2-benzimidazolidène)-3-(4'-hydroxyphényl)-1-formazano]-1,3-dihydroxyéthyl -benzimidazolium
- Chlorure de 2-[5-(1,3-dihydroxyéthyl -2-benzimidazolidène)-3-(4'-N,N-dimethylaminophenyl)-1-formazano]-1,3-dihydroxyéthyl-benzimidazolium
- Chlorure de 2-[5-(1,3-dicarboxyéthyl-2-benzimidazolidène)-1-formazano]-1,3-dicarboxyéthyl-benzimidazolium
- Chlorure de 2-[5-(1,3-dicarboxyéthyl-2-benzimidazolidène)-3-méthyl-1-formazano]-1,3-dicarboxyéthyl-benzimidazolium
- Chlorure de 2-[5-(1,3-dicarboxyéthyl-2-benzimidazolidène)-3-ethyl-1-formazano]-1,3-dicarboxyéthyl-benzimidazolium
- Chlorure de 2-[5-(1,3-dicarboxyéthyl-2-benzimidazolidène)-3-phenyl-1-formazano]-1,3-dicarboxyéthyl-benzimidazolium
- Chlorure de 2-[5-(1,3-dicarboxyéthyl-2-benzimidazolidène)-3-isopropyl-1-formazano]-1,3-dicarboxyéthyl-benzimidazolium
- Chlorure de 2-[5-(1,3-dicarboxyéthyl-2-benzimidazolidène)-3-(4'-methoxyphényl)-1-formazano]-1,3-dicarboxyéthyl-benzimidazolium
- Chlorure de 2-[5-(1,3-dicarboxyéthyl-2-benzimidazolidène)-3-(4'-hydroxyphényl)-1-formazano]-1,3-dicarboxyéthyl -benzimidazolium
- Chlorure de 2-[5-(1,3-dicarboxyéthyl -2-benzimidazolidène)-3-(4'-N,N-diméthylaminophényl)-1-formazano]-1,3-dicarboxyéthyl- benzimidazolium

Un deuxième objet de la présente demande consiste en une composition pour la teinture des fibres kératiniques, et en particulier des fibres kératiniques humaines et plus particulièrement les cheveux comprenant, dans un milieu approprié pour la teinture, au moins un composé de formule (I), en tant que colorant direct.

De préférence, les composés de formule (I) sont présents dans la composition selon la présente invention dans une concentration en poids allant de 0,001 à 5%, de préférence allant de 0,05 à 2% par rapport au poids total de la composition.

Selon une variante, la composition selon la présente invention comprend également au moins un colorant direct différent de ceux de formule (I) selon l'invention et choisis notamment dans le groupe formé par les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs méthiniques, les colorants directs triarylméthaniques, les colorants indoaminiques et les colorants directs naturels. Le ou les colorants directs additionnels représentent de préférence de 0,001 à 20% en poids environ du poids total de la composition et encore plus préférentiellement de 0,005 à 10% en poids environ.

La composition de l'invention peut en outre comprendre un agent oxydant. Cet agent oxydant peut être n'importe quel agent oxydant utilisé de façon classique pour la coloration ou la décoloration des fibres kératiniques. L'agent oxydant est choisi de préférence parmi le peroxyde d'hydrogène, le peroxyde d'urée, les bromates de métaux alcalins, les persels tels que les perborates et les persulfates, les peracides et les enzymes parmi lesquelles on peut citer les oxydo-réductases à 2 électrons telles que les uricases, les peroxydases et les oxydo-réductases à 4 électrons comme les laccases. L'utilisation du peroxyde d'hydrogène est particulièrement préférée en tant qu'agent oxydant.

La composition selon l'invention peut en outre comprendre une base d'oxydation. Cette base d'oxydation peut être choisie parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques.

La composition selon l'invention peut contenir en outre un ou plusieurs coupleurs conventionnellement utilisés pour la teinture de fibres kératiniques. Parmi ces coupleurs, on peut notamment citer les métaphénylènediamines, les métaaminophénols, les métadiphénols, les coupleurs naphtaléniques et les coupleurs hétérocycliques.

Dans la composition de la présente invention, le ou les coupleurs sont en général présents en quantité allant de 0,001 à 10% en poids environ du poids total de la composition tinctoriale et plus préférentiellement de 0,005 à 6%. La ou les bases d'oxydation sont présentes en une quantité allant de préférence de 0,001 à 10% en poids environ du poids total de la composition tinctoriale, et plus préférentiellement de 0,005 à 6%.

D'une manière générale, les sels d'addition avec un acide utilisables dans le cadre des compositions tinctoriales de l'invention pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄ tels que l'éthanol et l'isopropanol, les polyols et éthers de polyols comme le 2-butoxyéthanol, le propylèneglycol, le monométhyléther de propylèneglycol, le monoéthyléther et le monométhyléther de diéthylèneglycol, ainsi que les alcools aromatiques comme l'alcool benzylique ou le phénoxyéthanol, et leurs mélanges.

Les solvants organiques peuvent être présents dans des proportions de préférence comprises entre 1 et 40% en poids environ par rapport au poids total de la composition tinctoriale, et encore plus préférentiellement entre 5 et 30% en poids environ.

La composition tinctoriale conforme à l'invention peut également renfermer divers adjuvants utilisés classiquement dans les compositions pour la teinture des cheveux, tels que des agents tensioactifs anioniques, cationiques, non-ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les polymères associatifs anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants cités ci-dessus sont en général présents en quantité comprise pour chacun d'eux entre 0,01 et 20% en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium et de potassium et les composés de formule (III) suivante : 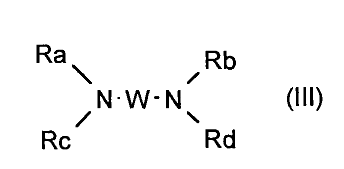 dans laquelle W est un reste propylène éventuellement substitué par un groupement hydroxyle ou un radical alkyle en C₁-C₄, Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels ou sous toute autre forme appropriée pour réaliser une teinture des fibres kératiniques, et notamment des cheveux humains.

Un troisième objet de la présente invention consiste en un procédé de teinture directe qui comprend l'application d'une composition tinctoriale comprenant au moins un colorant de formule (I) telle que définie précédemment sur les fibres kératiniques en particulier des fibres kératiniques humaines et plus particulièrement les cheveux pendant une durée suffisante pour développer la coloration désirée. Les fibres sont ensuite rincées puis séchées.

L'application sur les fibres kératiniques de la composition contenant le colorant de formule (I) peut être mise en oeuvre en présence d'agent oxydant, ce qui provoque la décoloration de la fibre (procédé de teinture directe éclaircissante). Cet agent oxydant peut être ajouté à la composition contenant le colorant de formule (I) au moment de l'emploi ou directement sur la fibre kératinique.

L'invention a aussi pour objet un procédé de teinture d'oxydation qui comprend l'application sur les fibres d'une composition tinctoriale qui comprend au moins un colorant de formule (I), au moins une base d'oxydation et optionnellement au moins un coupleur, en présence d'agent oxydant.

La base d'oxydation, le coupleur et l'agent oxydant sont tels que définis précédemment.

La couleur peut être révélée à pH acide, neutre ou alcalin et l'agent oxydant peut être ajouté à la composition selon l'invention juste au moment de l'emploi ou il peut être mis en oeuvre à partir d'une composition oxydante le contenant, il est alors appliqué sur les fibres simultanément ou séquentiellement à la composition tinctoriale.

Dans le cas de la teinture directe éclaircissante ou de la teinture d'oxydation, la composition tinctoriale est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pause de 3 à 50 minutes environ, de préférence 5 à 30 minutes environ, les fibres kératiniques sont rincées, lavées au shampooing, rincées à nouveau puis séchées.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

La présente invention a de plus pour objet un procédé de décoloration des colorants directs, qui font l'objet de l'invention et qui viennent d'être décrits. Avantageusement, ce procédé de décoloration peut être appliqué à des fibres kératiniques préalablement teintes conformément aux procédés de teinture qui viennent d'être décrits. L'un des avantages de ce procédé est que la couleur naturelle des fibres avant la teinture avec la composition selon l'invention est conservée, seule la couleur apportée par le colorant direct est éliminée.

Ce procédé présente aussi l'avantage d'être rapide et facile de mise en oeuvre. Ainsi, le procédé consiste à appliquer sur le composé de formule (I) et (I') une composition de décoloration comprenant au moins un agent réducteur choisi parmi les réducteurs comprenant du soufre. Dans le cas où le procédé est appliqué sur des fibres kératiniques, il consiste à mettre en oeuvre les étapes suivantes :
a) on applique sur les fibres préalablement traitées par une composition tinctoriale selon l'invention, une composition de décoloration comprenant, dans un milieu approprié, au moins un agent réducteur choisi parmi les réducteurs comprenant du soufre ;
b) on laisse pauser pendant une durée suffisante pour avoir la décoloration ;
c) on rince éventuellement les fibres ;
d) on lave les fibres, on les rince puis on les sèche.

A titre d'exemple, les réducteurs comprenant du soufre sont choisis parmi les composés présentant au moins une fonction thiol, sulfure ou sulfite.

Parmi les thiols utilisables en tant que composé réducteur, on peut citer l'acide thioglycolique, l'acide thiolactique, leurs sels de métal alcalin ou alcalino-terreux (comme le sodium, le potassium, le calcium) et leurs esters ; β-mercaptoéthanol, la cystéine, la cystéamine et leurs dérivés ; l'homocystéine et l'un de ses sels ; les mercaptoaldéhydes ; la pénicillamine ; le glutathion. Ces composés pouvant être utilisés seuls ou en mélanges.

En ce qui concerne les sulfites utilisables en tant que réducteurs, parmi lesquels figurent aussi les bisulfites, les hydrosulfites, conviennent les sels de métaux alcalins, alcalino-terreux ou d'ammonium, ainsi que leurs mélanges. Plus particulièrement, on peut citer le sulfite de sodium, le métasulfite de soudium, et l'hydrosulfite de sodium.

En ce qui concerne les sulfures parmi lesquels figurent aussi les disulfures, on peut citer notamment la cystine.

La teneur en agent réducteur dans la composition est avantageusement comprise entre 0,01 et 10 % en poids de la composition de décoloration du colorant, de préférence entre 0,1 et 5 % en poids de ladite composition de décoloration.

Le milieu de la composition comprend de manière classique de l'eau ou un solvant organique. Ce qui a été indiqué auparavant relativement au milieu de la composition tinctoriale reste valable et l'on pourra s'y reporter.

La composition peut de même comprendre des adjuvants classiques tels que des agents tensioactifs anioniques, cationiques, non-ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les polymères associatifs anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants cités ci-dessus sont en général présents en quantité comprise pour chacun d'eux entre 0,01 et 20% en poids par rapport au poids de la composition de décoloration. Bien entendu, l'homme de l'art veillera à choisir ce ou ces éventuels adjuvants de manière telle que les propriétés avantageuses attachées intrinsèquement à l'utilisation de la composition de décoloration ne soient pas, ou substantiellement pas, altérées par la ou les adjonctions envisagées.

Le pH de la composition de décoloration est généralement compris entre 6 et 10, avantageusement entre 7 et 9. Il peut être ajusté à la valeur désirée au moyen d'agents acidifiants ou alcalinisants habituellement utilisés dans le domaine, dont des listes ont été données auparavant, ou bien encore à l'aide de systèmes tampons classiques.

La composition de décoloration peut être appliquée indifféremment sur cheveux secs ou mouillés.

A titre indicatif, la température à laquelle est mis en oeuvre le procédé de décoloration est comprise entre 15 et 50°C, de préférence voisine de la température ambiante.

La durée de pause de la composition est en général comprise entre 1 et 30 minutes, plus particulièrement entre 2 et 20 minutes. Il est à noter que cette gamme n'est donnée qu'à titre indicatif. En effet, celle-ci peut être déterminée sans difficulté par l'homme de l'art, la décoloration des cheveux pouvant être constatée visuellement, car le phénomène est visible. Généralement, plus la teneur en agent réducteur est importante et/ou plus la température est élevée, moins la durée de pause est longue pour décolorer le colorant ou les cheveux préalablement colorés avec les colorants selon l'invention.

La présente invention concerne encore les composés tétraazapentaméthiniques de formule (I') 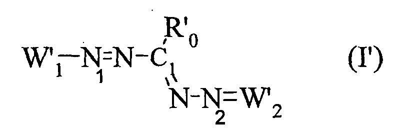 dans laquelle
- W'₁ représente un radical hétéroaromatique cationique de formule (II') ou (III') : 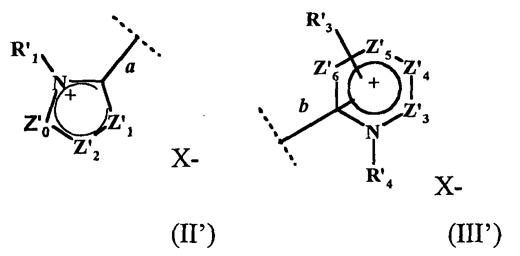
- W'₂ représente un radical hétéroaromatique de formule (IV') ou (V'): 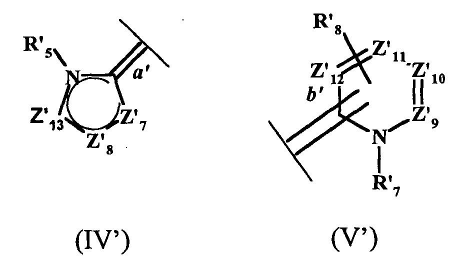
dans lesquelles :
- Z'₀ représente un radical CR'_{2,} un atome d'azote ou un radical NR'_{21,}
- Z'₁ représente un atome d'oxygène ou un radical NR'₉,
- Z'₂ représente un atome d'azote ou un radical CR'₁₀,
- Z'₃ représente un atome d'azote ou un radical CR'₁₁,
- Z'₄ représente un atome d'azote ou un radical CR'₁₂,
- Z'₅ représente un atome d'azote ou un radical CR'₁₃,
- Z'₆ représente un atome d'azote ou un radical CR'₁₄,
- Z'₇ représente un atome d'oxygène ou un radical NR'₁₅,
- Z'₈ représente un atome d'azote ou un radical CR'₁₆,
- Z'₉ représente un atome d'azote ou un radical CR'₁₇,
- Z'₁₀ représente un atome d'azote ou un radical CR'₁₈,
- Z'₁₁ représente un atome d'azote ou un radical CR'₁₉,
- Z'₁₂ représente un atome d'azote ou un radical CR'₂₀,
- Z'₁₃ représente un radical CR'₆, un atome d'azote ou un radical NR'₂₂,
- étant entendu que chacun des cycles des formules (II'), (III'), (IV') et (V') ne comportent pas plus de trois atomes d'azote, et que deux des trois atomes d'azote peuvent être contigus,
- la liaison a du radical hétéroaromatique cationique à 5 chaînons de la formule (II') étant reliée à l'atome d'azote N₁ de la formule (I'),
- la liaison b du radical hétéroaromatique cationique à 6 chaînons de la formule (III') étant reliée à l'atome d'azote N₁ de la formule (I').
- la double liaison a' du radical hétéroaromatique à 5 chaînons de la formule (IV') étant reliée à l'atome d'azote N₂ de la formule (I'),
- la double liaison b' du radical hétéroaromatique à 6 chaînons de la formule (V') étant reliée à l'atome d'azote N₂ de la formule (I'),
- la liaison b, reliant le radical hétéroaromatique cationique de la formule III' à l'atome d'azote N₁ de la formule (I'), étant située en position ortho ou para de l'atome d'azote portant le radical R'₄ lorsque Z'₅ représente un radical CR'₁₃ ; la liaison b étant située en position ortho de l'atome d'azote portant le radical R'₄ lorsque Z'₅ représente un atome d'azote,
- la liaison b', reliant le radical hétéroaromatique de la formule V' à l'atome d'azote N₂ de la formule (I'), étant située en position ortho ou para de l'atome d'azote portant le radical R'₇ lorsque Z'₁₁ représente un radical CR'₁₉ ; la liaison b' étant située en position ortho de l'atome d'azote portant le radical R'₇ lorsque Z'₁₁ représente un atome d'azote,
- R'₂, R'₆, R'₁₀ et R'₁₆ représentent, indépendamment l'un de l'autre, un atome d'hydrogène ; un radical alkyle en C₁-C₄ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique ; un radical phényle non substitué ou substitué par un ou deux radicaux choisis parmi les radicaux hydroxy, alcoxy en C₁-C₂, (poly)-hydroxyalcoxy en C₂-C₄, amino, (di)alkylamino en C₁-C₂ ; un radical carboxy ; un radical sulfonylamino,
   R'₁, R'₄, R'₅, R'₇, , R'₉, R'₁₅, R'₂₁, et R'₂₂ représentent, indépendamment l'un de l'autre, un radical alkyle en C₁-C₈ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique ;
- R'₀, R'₃, R'₈, R'₁₁, R'₁₂, R'₁₃, R'₁₄, R'₁₇, R'₁₈, R'₁₉, et R'₂₀ représentent, indépendamment l'un de l'autre, un atome d'hydrogène, une chaîne hydrocarbonée en C₁-C₁₆ linéaire ou ramifiée, cette chaîne pouvant être saturée ou insaturée par une à trois insaturations, cette chaîne étant non substituée ou substituée par un à trois radicaux choisis parmi les radicaux hydroxy, alcoxy en C₁-C₂, (poly)-hydroxyalcoxy en C₂-C₄, amino, (di)alkylamino en C₁-C₂, carboxy, sulfonique, sulfonylamino, (poly)-hydroxyalkylamino en C₂-C₄ ou un atome d'halogène tel que chlore, fluor ou brome ; un radical phényle non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy, sulfonique, sulfonylamino, (poly)-hydroxyalkylamino en C₂-C₄ ou un atome d'halogène tel que chlore, fluor ou brome ; un radical hétéroaryle choisi parmi les radicaux pyrazolyle, pyrrolyle, imidazolyle, thiazolyle, oxazolyle, triazolyle, pyridinyle, pyrimidinyletriazinyle, pyrazinyle, pyridazinyle, en outre cette chaîne hydrocarbonée peut être interrompue par un ou deux atomes d'oxygène, d'azote, de soufre ou par un radical SO_{2,}
- R'₂ avec R'₁₀, R'₁₁ avec R'₁₂, R'₆ avec R'₁₆, et R'₁₇ avec R'₁₈ pouvant former, indépendamment les uns des autres un cycle aromatique carboné à 5 ou 6 chaînons non substitué ou substitué par un ou deux radicaux hydroxy, amino, (di)alkyl(C₁-C₂)amino, alcoxyen C₁-C₂, (poly)hydroxyalkyl(C₂-C₄)amino, sous réserve que W'₁ et W'₂ ne représentent pas simultanément le même système bicyclique,
- étant entendu que R'₀, R'₃, R'₈, R'₁₁, R'₁₂, R'₁₃, R'₁₄, R'₁₇, R' ₁₈, R' ₁₉, et R'₂₀ ne comportent pas de liaison peroxyde, ni de radicaux diazo ou nitroso,
- X est un anion organique ou minéral.

De préférence R'₀ représente préférentiellement un atome d'hydrogène ; un radical alkyle en C₁-C₆ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, (poly)hydroxyalkylamino en C₂-C₄, carboxy ou sulfonique ; un radical phényle , non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy, sulfonique ou un atome d'halogène tel que chlore, fluor ou brome ; un radical hétéroaryle éventuellement cationique choisi parmi les radicaux pyrazolyle, pyrrolyle, imidazolyle, thiazolyle, oxazolyle, triazolyle, pyridinyle, pyrimidinyletriazinyle, pyrazinyle, pyridazinyle.

De manière encore plus préférée, R'₀ représente préférentiellement un atome d'hydrogène ; un radical alkyle en C₁-C₃ linéaire ou ramifié, non substitué ou substitué par un à deux radicaux choisis parmi les radicaux hydroxy, alcoxy en C₁-C₂, amino, (di)alkylamino en C₁-C₂ , carboxy ou sulfonique ; un radical phényle , non substitué ou substitué par un à deux radicaux choisis parmi les radicaux hydroxy, amino, (di)alkylamino en C₁-C₂ ou (poly)-hydroxyalkylamino en C₂-C₄, un radical hétéroaryle éventuellement cationique choisi parmi les radicaux pyrazolyle, pyrrolyle, imidazolyle, pyridinyle.

Selon un mode de réalisation particulièrement préféré, R'₀ représente un atome d'hydrogène ; un méthyle, éthyle, propyle, butyle, 2-hydroxyéthyle , 2-aminoéthyle ;1-carboxyméthyle, 2-carboxyéthyle, 2-sulfonyléthyle, 2-méthoxyéthyle ; un radical phényle , non substitué ou substitué par un à deux radicaux choisis parmi les radicaux amino, (di)alkylamino en C₁-C₂ , (poly)-hydroxyalkylamino en C₂-C₄, un radical hétéroaryle éventuellement cationique choisi parmi les radicaux pyrazolyle, pyrrolyle, imidazolyle, pyridinyle.

R'₀ représente un atome d'hydrogène ; un radical méthyle, éthyle, 2-méthoxyéthyle ; un radical phényle non substitué ou substitué par un radical un amino, (di)méthylamino , (di)(2-hydroxyéthyl)amino, un radical hétéroaryle éventuellement cationique choisi parmi les radicaux pyrazolyle, pyrrolyle, imidazolyle, pyridinyle.

R'₂, R'₆, R'₁₀ et R'₁₆ représentent préférentiellement un atome d'hydrogène, un radical alkyle en C₁-C₄ non substitué ou substitué par un à deux radicaux choisis parmi les radicaux hydroxy, amino, (di)alkylamino en C₁-C₂ , carboxy, un radical phényl.

Selon un mode de réalisation particulièrement préféré, R'₂, R'₆, R'₁₀ et R'₁₆ représentent préférentiellement un atome d'hydrogène, un radical méthyle, phényle, 2-hydroxyméthyle, un carboxy.

R'₁, R'₄, R'₅, R'₇, R'₉, R'₁₅, R'₂₁, et R'₂₂ représentent préférentiellement un radical alkyle en C₁-C₄, non substitué ou substitué par un à deux radicaux choisis parmi les radicaux hydroxy, alcoxy en C₁-C₂, amino, (di)alkylamino en C₁-C₂, carboxy, sulfonique.

Selon un mode de réalisation particulièrement préféré, R'₁, R'₄, R'₅, R'₇, R'₉, R'₁₅, R'₂₁ et R'₂₂ représentent préférentiellement un radical méthyle, éthyle, 2-hydroxyéthyle, 1-carboxyméthyle, 2-carboxyéthyle, 2-sulfonyléthyle.

R'₃, R'₈, R'₁₁, R'₁₂, R'₁₃, R'₁₄, R'₁₇, R'₁₈, R'₁₉, et R'₂₀ représentent, préférentiellement et indépendamment l'un de l'autre, un atome d'hydrogène ; un radical alkyle en C₁-C₄ linéaire ou ramifié, non substitué ou substitué par un à deux radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique; un radical phényle , non substitué ou substitué par un à deux radicaux choisis parmi les radicaux hydroxy, alcoxy en C₁-C₂, (poly)-hydroxyalcoxy en C₂-C₄, amino, (di)alkylamino en C₁-C₂, carboxy, sulfonique ou un atome d'halogène tel que chlore, fluor ou brome ; un radical sulfonylamino ; un radical (poly)-hydroxyalkylamino en C₂-C₄.

Plus préférentiellement, R'₃, R'₈, R'₁₁, R'₁₂, R'₁₃, R'₁₄, R'₁₇, R'₁₈, R'₁₉, et R'₂₀ représentent un atome d'hydrogène, un radical alkyle en C₁-C₄, non substitué ou substitué par un à deux radicaux choisis parmi les radicaux hydroxy, un radical alcoxy en C₁-C₂ ; un radical amino ; un radical (di)alkylamino en C₁-C₂ ; un radical carboxy ; un radical (poly)-hydroxyalkylamino en C₂-C₄.

Selon un mode de réalisation particulièrement préféré R'₃, R'₈, R'₁₁, R'₁₂, R'₁₃, R'₁₄, R'₁₇, R'₁₈, R'₁₉, et R'₂₀ représentent indépendamment l'un de l'autre un atome d'hydrogène, un radical méthyle, 2-hydroxyméthyle, un carboxy, un radical méthoxy, éthoxy, 2-hydroxyéthyloxy, un radical amino, méthylamino, diméthylamino, 2-hydroxyéthylamino.
A. De préférence, les composés de formule I' sont choisis dans la famille définie par les composés pour lesquels W'₁ est un radical 2-pyridinium et W'₂ est un radical 2-pyridine. De manière encore plus préférée, ce sont les composés suivants :
   - Chlorure de 2-[5-(N-methyl-2-pyridinylidène)-1-formazano]-N-méthylpyridinium
   - Chlorure de 2-[5-(N-methyl-2-pyridinylidène)-3-méthyl-1-formazano]-N-méthylpyridinium
   - Chlorure de 2-[5-(N-methyl-2-pyridinylidène)-3-éthyl-1-formazano]-N-méthylpyridinium
   - Chlorure de 2-[5-(N-méthyl-2-pyridinylidene)-3-isopropyl-1-formazano]-N-méthylpyridinium
   - Chlorure de 2-[5-(N-methyl-2-pyridinylidène)-3-phényl-1-formazano]-N-méthylpyridinium
   - Chlorure de 2-[5-(N-méthyl-2-pyridinylidène)-3-(4'-methoxyphényl)-1-formazano]-N-méthylpyridinium
   - Chlorure de 2-[5-(N-méthyl-2-pyridinylidène)-3-(4'-hydroxyphényl)-1-formazano]-N-méthylpyridinium
   - Chlorure de 2-[5-(N-méthyl-2-pyridinylidène)-3-(4'-N,N-dimethylaminophényl)-1-formazano] -N-méthylpyridinium
   - Chlorure de 2-[5-(4-pyrrolidino-N-méthyl-2-pyridinylidène)-1-formazano]-4-pyrrolidino-N-méthylpyridinium
   - Chlorure de 2-[5-(4-pyrrolidino-N-méthyl-2-pyridinylidène)-3-méthyl-1-formazano]- 4-pyrrolidino-N-méthylpyridinium
   - Chlorure de 2-[5-(4-pyrrolidino-N-méthyl-2-pyridinylidène)-3-éthyl-1-formazano]- 4-pyrrolidino-N-méthylpyridinium
   - Chlorure de 2-[5-(4-pyrrolidino-N-méthyl-2-pyridinylidène)-3-isopropyl-1-formazano]- 4-pyrrolidino-N-méthylpyridinium
   - Chlorure de 2-[5-(4-pyrrolidino-N-méthyl-2-pyridinylidène)-3-phenyl-1-formazano]- 4-pyrrolidino-N-méthylpyridinium
   - Chlorure de 2-[5-(4-pyrrolidino-N-methyl-2-pyridinylidène)-3-(4'-methoxyphenyl)-1-formazano]- 4-pyrrolidino-N-méthylpyridinium
   - Chlorure de 2-[5-(4-pyrrolidino-N-méthyl-2-pyridinylidène)-3-(4'-hydroxyphényl)-1-formazano]- 4-pyrrolidino-N-méthylpyridinium
   - Chlorure de 2-[5-(4-pyrrolidino-N-méthyl-2-pyridinylidène)-3-(4'-N,N-diméthylaminophényl)- 1 -formazano]- 4-pyrrolidino-N-méthylpyridinium
   - Chlorure de 2-[5-(N-hydroxyéthyl-2-pyridinylidène)-1-formazano]-N-hydroxyethylpyridinium
   - Chlorure de 2-[5-(N-hydroxyethyl-2-pyridinylidène)-3-méthyl-1-formazano]-N-hydroxyethylpyridinium
   - Chlorure de 2-[5-(N-hydroxyethyl-2-pyridinylidène)-3-éthyl-1-formazano]-N- hydroxyethyl-pyridinium
   - Chlorure de 2-[5-(N- hydroxyéthyl -2-pyridinylidène)-3-phényl-1-formazano]-N- hydroxyethyl-pyridinium.
   - Bromure de 2-[5-(N- hydroxyéthyl -2-pyridinylidène)-3-(2-pyridinyl)-1-formazano]-N- hydroxyéthyl -pyridinium.
   - Bromure de 2-[5-(N- hydroxyéthyl -2-pyridinylidène)-3-(méthosulfate de 2-N-méthylpyridinylium)-1-formazano]-N-hydroxyéthyl -pyridinium.
   - Chlorure de 2-[5-(N- hydroxyéthyl -2-pyridinylidène)-3-isopropyl-1-formazano]-N- hydroxyéthyl -pyridinium
   - Chlorure de 2-[5-(N- hydroxyéthyl-2-pyridinylidène)-3-(4'-méthoxyphényl)-1-formazano]-N- hydroxyéthyl -pyridinium
   - Chlorure de 2-[5-(N- hydroxyéthyl -2-pyridinylidène)-3-(4'-hydroxyphényl)-1-formazano]-N- hydroxyéthyl -pyridinium
   - Chlorure de 2-[5-(N- hydroxyéthyl -2-pyridinylidène)-3-(4'-N,N-diméthylaminophényl)-1-formazano]-N- hydroxyéthyl -pyridinium
   - Chlorure de 2-[5-(N-carboxyéthyl-2-pyridinylidène)-1-formazano]-N- carboxyéthyl -pyridinium
   - Chlorure de 2-[5-(N- carboxyéthyl -2-pyridinylidène)-3-méthyl-1-formazano]-N- carboxyéthyl -pyridinium
   - Chlorure de 2-[5-(N- carboxyéthyl -2-pyridinylidène)-3-éthyl-1-formazano]-N- carboxyéthyl -pyridinium
   - Chlorure de 2-[5-(N- carboxyéthyl -2-pyridinylidène)-3-phényl-1-formazano]-N- carboxyéthyl -pyridinium
   - Chlorure de 2-[5-(N- carboxyéthyl -2-pyridinylidène)-3-isopropyl-1-formazano]-N- carboxyéthyl -pyridinium
   - Chlorure de 2-[5-(N- carboxyéthyl -2-pyridinylidène)-3-(4'-méthoxyphényl)-1-formazano]-N- carboxyéthyl -pyridinium
   - Chlorure de 2-[5-(N- carboxyéthyl -2-pyridinylidène)-3-(4'-hydroxyphényl)-1-formazano]-N- carboxyéthyl -pyridinium
   - Chlorure de 2-[5-(N- carboxyéthyl -2-pyridinylidène)-3-(4'-N,N-diméthylaminophényl)-1-formazano]-N- carboxyéthyl -pyridinium
B. De préférence, les composés de formule I' sont choisis dans la famille définie par les composés pour lesquels W'₁ est un radical 4-pyridinium et W'₂ est un radical 4-pyridine. De manière encore plus préférée, ce sont les composés suivants :
   - Chlorure de 4-[5-(N-méthyl-4-pyridinylidène)-1-formazano]-N-méthylpyridinium
   - Chlorure de 4-[5-(N-méthyl-4-pyridinylidène)-3-méthyl-1-formazano]-N-méthylpyridinium
   - Chlorure de 4-[5-(N-méthyl-4-pyridinylidène)-3-éthyl-1-formazano]-N-méthylpyridinium
   - Chlorure de 4-[5-(N-méthyl-4-pyridinylidène)-3-isopropyl-1-formazano]-N-méthylpyridinium
   - Chlorure de 4-[5-(N-méthyl-4-pyridinylidène)-3-phényl-1-formazano]-N-méthylpyridinium
   - Chlorure de 4-[5-(N-méthyl-4-pyridinylidène)-3-(4'-méthoxyphényl)- 1 -formazano] -N-méthylpyridinium
   - Chlorure de 4-[5-(N-méthyl-4-pyridinylidène)-3-(4'-hydroxyphényl)-1-formazano]-N-méthylpyridinium
   - Chlorure de 4-[5-(N-méthyl-4-pyridinylidène)-3-(4'-N,N-diméthylaminophényl)- 1 -formazano] -N-méthylpyridinium
   - Chlorure de 4-[5-(N-hydroxyéthyl-4-pyridinylidène)-1-formazano]-N-hydroxyéthylpyridinium
   - Chlorure de 4-[5-(N-hydroxyéthyl-4-pyridinylidène)-3-méthyl-1-formazano]-N-hydroxyéthylpyridinium
   - Chlorure de 4-[5-(N-hydroxyéthyl-4-pyridinylidène)-3-éthyl-1-formazano]-N- hydroxyéthyl-pyridinium
   - Chlorure de 4-[5-(N- hydroxyéthyl -4-pyridinylidène)-3-phényl-1-formazano]-N- hydroxyéthyl-pyridinium
   - Chlorure de 4-[5-(N- hydroxyéthyl -4-pyridinylidène)-3-isopropyl-1-formazano]-N- hydroxyéthyl -pyridinium
   - Chlorure de 4-[5-(N- hydroxyéthyl-4-pyridinylidène)-3-(4'-méthoxyphényl)-1-formazano]-N- hydroxyéthyl -pyridinium
   - Chlorure de 4-[5-(N- hydroxyéthyl -4-pyridinylidène)-3-(4'-hydroxyphényl)-1-formazano]-N- hydroxyéthyl -pyridinium
   - Chlorure de 4-[5-(N- hydroxyéthyl -4-pyridinylidène)-3-(4'-N,N-diméthylaminophényl)-1-formazano]-N- hydroxyéthyl -pyridinium
   - Chlorure de 4-[5-(N-carboxyéthyl-4-pyridinylidène)-1-formazano]-N- carboxyéthyl -pyridinium
   - Chlorure de 4-[5-(N- carboxyéthyl -4-pyridinylidène)-3-méthyl-1-formazano]-N- carboxyéthyl -pyridinium
   - Chlorure de 4-[5-(N- carboxyéthyl -4-pyridinylidène)-3-éthyl-1-formazano]-N- carboxyéthyl -pyridinium
   - Chlorure de 4-[5-(N- carboxyéthyl -4-pyridinylidène)-3-phényl-1-formazano]-N- carboxyéthyl -pyridinium
   - Chlorure de 4-[5-(N- carboxyéthyl -4-pyridinylidène)-3-isopropyl-1-formazano]-N- carboxyéthyl -pyridinium
   - Chlorure de 4-[5-(N- carboxyéthyl -4-pyridinylidène)-3-(4'-méthoxyphényl)-1-formazano]-N- carboxyéthyl -pyridinium
   - Chlorure de 4-[5-(N- carboxyéthyl -4-pyridinylidène)-3-(4'-hydroxyphényl)-1-formazano]-N- carboxyéthyl -pyridinium
   - Chlorure de 4-[5-(N- carboxyéthyl -4-pyridinylidène)-3-(4'-N,N-diméthylaminophényl)-1-formazano]-N- carboxyéthyl -pyridinium
C. De préférence, les composés de formule I' sont choisis dans la famille définie par les composés pour lesquels W'₁ est un radical 2-imidazolium et W'₂ est un radical 2-imidazole. De manière encore plus préférée, ce sont les composés suivants :
   - Chlorure de 2-[5-(1,3-diméthyl-2-imidazolidène)-1-formazano]- 1,3-diméthyl-imidazolium
   - Chlorure de 2-[5-(1,3-diméthyl-2-imidazolidène)-3-méthyl-1-formazano]-1,3-diméthyl-imidazolium
   - Chlorure de 2-[5-(1,3-diméthyl-2-imidazolidène)-3-éthyl-1-formazano]-1,3-diméthyl-imidazolium
   - Chlorure de 2-[5-(1,3-diméthyl-2-imidazolidène)-3-isopropyl-1-formazano]-1,3-diméthyl-imidazolium
   - Chlorure de 2-[5-(1,3-diméthyl-2-imidazolidène)-3-phényl-1-formazano]-1,3-diméthyl-imidazolium
   - Chlorure de 2-[5-(1,3-diméthyl-2-imidazolidène)-3-(4'-methoxyphenyl)- 1 -formazano]-1,3-diméthyl-imidazolium
   - Chlorure de 2-[5-(1,3-diméthyl-2-imidazolidène)-3-(4'-hydroxyphényl)-1-formazano]-1,3-diméthyl-imidazolium
   - Chlorure de 2-[5-(1,3-diméthyl-2-imidazolidène)-3-(4'-N,N-diméthylaminophényl)-1-formazano]-1,3-diméthyl-imidazolium
   - Chlorure de 2-[5-(1,3-dihydroxyéthyl-2-imidazolidène)-1-formazano]-1,3-dihydroxyéthylimidazolium
   - Chlorure de 2-[5-(1,3-dihydroxyéthyl-2-imidazolidène)-3-méthyl-1-formazano]-1,3-dihydroxyéthylimidazolium
   - Chlorure de 2-[5-(1,3-dihydroxyéthyl-2-imidazolidène)-3-éthyl-1-formazano]-1,3-dihydroxyéthyl -imidazolium
   - Chlorure de 2-[5-(1,3-dihydroxyéthyl -2-imidazolidène)-3-phenyl-1-formazano]-1,3-dihydroxyéthyl -imidazolium
   - Chlorure de 2-[5-(1,3-dihydroxyéthyl -2-imidazolidène)-3-isopropyl-1-formazano]-1,3-dihydroxyéthyl -imidazolium
   - Chlorure de 2-[5-(1,3-dihydroxyéthyl-2-imidazolidène)-3-(4'-méthoxyphényl)-1-formazano]-1,3-dihydroxyéthyl -imidazolium
   - Chlorure de 2-[5-(1,3-dihydroxyéthyl -2-imidazolidène)-3-(4'-hydroxyphényl)-1-formazano]-1,3-dihydroxyéthyl -imidazolium
   - Chlorure de 2-[5-(1,3-dihydroxyéthyl -2-imidazolidène)-3-(4'-N,N-diméthylaminophényl)-1-formazano]-1,3-dihydroxyéthyl-imidazolium
   - Chlorure de 2-[5-(1,3-dicarboxyéthyl-2-imidazolidène)-1-formazano]-1,3-dicarboxyéthyl -imidazolium
   - Chlorure de 2-[5-(1,3-dicarboxyéthyl -2-imidazolidène)-3-méthyl-1-formazano]-1,3-dicarboxyéthyl -imidazolium
   - Chlorure de 2-[5-(1,3-dicarboxyéthyl -2-imidazolidène)-3-éthyl-1-formazano]-1,3-dicarboxyéthyl -imidazolium
   - Chlorure de 2-[5-(1,3-dicarboxyéthyl -2-imidazolidène)-3-phényl-1-formazano]-1,3-dicarboxyéthyl -imidazolium
   - Chlorure de 2-[5-(1,3-dicarboxyéthyl -2-imidazolidène)-3-isopropyl-1-formazano]-1,3-dicarboxyéthyl -imidazolium
   - Chlorure de 2-[5-(1,3-dicarboxyéthyl -2-imidazolidène)-3-(4'-méthoxyphényl)-1-formazano]-1,3-dicarboxyéthyl -imidazolium
   - Chlorure de 2-[5-(1,3-dicarboxyéthyl -2-imidazolidène)-3-(4'-hydroxyphényl)-1-formazano]-1,3-dicarboxyéthyl -imidazolium
   - Chlorure de 2-[5-(1,3-dicarboxyéthyl -2-imidazolidène)-3-(4'-N,N-diméthylaminophényl)-1-formazano]-1,3-dicarboxyéthyl-imidazolium
D. De préférence, les composés de formule I' sont choisis dans la famille définie par les composés pour lesquels W'₁ est un radical 5-pyrazolium et W'₂ est un radical 5-pyrazole. De manière encore plus préférée, ce sont les composés suivants :
   - Chlorure de 5-[5-(1,2-diméthyl-5-pyrazolidène)-1-formazano]-1,2-diméthyl-pyrazolinium
   - Chlorure de 5-[5-(1,2-diméthyl-5-pyrazolidène)-3-méthyl-1-formazano]-1,2-diméthyl-pyrazolinium
   - Chlorure de 5-[5-(1,2-diméthyl-5-pyrazolidène)-3-éthyl-1-formazano]-1,2-diméthyl-pyrazolinium
   - Chlorure de 5-[5-(1,2-diméthyl-5-pyrazolidène)-3-isopropyl-1-formazano]-1,2-diméthyl-pyrazolinium
   - Chlorure de 5-[5-(1,2-diméthyl-5-pyrazolidene)-3-phényl-1-formazano]-1,2-diméthyl-pyrazolinium
   - Chlorure de 5-[5-(1,2-diméthyl-5-pyrazolidène)-3-(4'-méthoxyphényl)-1-formazano]-1,2-diméthyl-pyrazolinium
   - Chlorure de 5-[5-(1,2-diméthyl-5-pyrazolidène)-3-(4'-hydroxyphényl)- 1-formazano]-1,2-diméthyl-pyrazolinium
   - Chlorure de 5-[5-(1,2-diméthyl-5-pyrazolidène)-3-(4'-N,N-diméthylaminophényl)-1-formazano]-1,2-diméthyl-pyrazolinium
   - Chlorure de 5-[5-(1,2-dihydroxyéthyl-5-pyrazolidène)-1-formazano]-1,2-dihydroxyéthylpyrazolinium
   - Chlorure de 5-[5-(1,2-dihydroxyéthyl-5-pyrazolidène)-3-méthyl-1-formazano]-1,2-dihydroxyéthylpyrazolinium
   - Chlorure de 5-[5-(1,2-dihydroxyéthyl-5-pyrazolidène)-3-éthyl-1-formazano]-1,2-dihydroxyéthyl -pyrazolinium
   - Chlorure de 5-[5-(1,2-dihydroxyéthyl -5-pyrazolidène)-3-phényl-1-formazano]-1,2-dihydroxyéthyl -pyrazolinium
   - Chlorure de 5-[5-(1,2-dihydroxyéthyl -5-pyrazolidène)-3-isopropyl-1-formazano]-1,2-dihydroxyéthyl -pyrazolinium
   - Chlorure de 5-[5-(1,2-dihydroxyéthyl-5-pyrazolidène)-3-(4'-méthoxyphényl)-1-formazano]-1,2-dihydroxyéthyl -pyrazolinium
   - Chlorure de 5-[5-(1,2-dihydroxyéthyl -5-pyrazolidène)-3-(4'-hydroxyphényl)-1-formazano]-1,2-dihydroxyéthyl -pyrazolinium
   - Chlorure de 5-[5-(1,2-dihydroxyéthyl -5-pyrazolidène)-3-(4'-N,N-diméthylaminophényl)-1-formazano]-1,2-dihydroxyéthyl-pyrazolinium
   - Chlorure de 5-[5-(1,2-dicarboxyéthyl-5-pyrazolidène)-1-formazano]-1,2-dicarboxyéthyl -pyrazolinium
   - Chlorure de 5-[5-(1,2-dicarboxyéthyl -5-pyrazolidène)-3-méthyl-1-formazano]-1,2-dicarboxyéthyl -pyrazolinium
   - Chlorure de 5-[5-(1,2-dicarboxyéthyl -5-pyrazolidène)-3-éthyl- 1 -formazano]-1,2-dicarboxyéthyl -pyrazolinium
   - Chlorure de 5-[5-(1,2-dicarboxyéthyl -5-pyrazolidène)-3-phényl-1-formazano]-1,2-dicarboxyéthyl -pyrazolinium
   - Chlorure de 5-[5-(1,2-dicarboxyéthyl -5-pyrazolidène)-3-isopropyl-1-formazano]-1,2-dicarboxyéthyl -pyrazolinium
   - Chlorure de 5-[5-(1,2-dicarboxyéthyl -5-pyrazolidène)-3-(4'-méthoxyphényl)-1-formazano]-1,2-dicarboxyéthyl -pyrazolinium
   - Chlorure de 5-[5-(1,2-dicarboxyéthyl -5-pyrazolidène)-3-(4'-hydroxyphényl)-1-formazano]-1,2-dicarboxyéthyl -pyrazolinium.
   - Chlorure de 5-[5-(1,2-dicarboxyéthyl -5-pyrazolidène)-3-(4'-N,N-diméthylaminophényl)-1-formazano]-1,2-dicarboxyéthyl-pyrazolinium
E. De préférence, les composés de formule I' sont choisis dans la famille définie par les composés pour lesquels W'₁ est un radical 2-benzimidazolium et W'₂ est un radical 2-pyridine. De manière encore plus préférée, ce sont les composés suivants :
   - Chlorure de 2-[5-(N-méthyl-2-pyridinylidène)-1-formazano]-1,3-diméthyl-benzimidazolium
   - Chlorure de 2-[5-(N-méthyl-2-pyridinylidène)-3-méthyl-1-formazano]-1,3-diméthyl-benzimidazolium
   - Chlorure de 2-[5-(N-méthyl-2-pyridinylidène)-3-éthyl-1-formazano]-1,3-diméthyl-benzimidazolium
   - Chlorure de 2-[5-(N-méthyl-2-pyridinylidène)-3-isopropyl-1-formazano]- 1,3-diméthyl-benzimidazolium
   - Chlorure de 2-[5-(N-méthyl-2-pyridinylidène)-3-phényl-1-formazano]-1,3-diméthyl-benzimidazolium
   - Chlorure de 2-[5-(N-méthyl-2-pyridinylidène)-3-(4'-méthoxyphényl)-1-formazano]-1,3-diméthyl-benzimidazolium
   - Chlorure de 2-[5-(N-méthyl-2-pyridinylidène)-3-(4'-hydroxyphényl)-1-formazano]-1,3-diméthyl-benzimidazolium
   - Chlorure de 2-[5-(N-méthyl-2-pyridinylidène)-3-(4'-N,N-diméthylaminophényl)-1-formazano]-1,3-diméthyl-benzimidazolium.
   - Chlorure de 2-[5-(N-hydroxyéthyl-2-pyridinylidène)-1-formazano]- 1,3 -dihydroxyéthylbenzimidazolium
   - Chlorure de 2-[5-(N-hydroxyéthyl-2-pyridinylidène)-3-méthyl-1-formazano]-1,3-dihydroxyéthylbenzimidazolium
   - Chlorure de 2-[5-(N-hydroxyethyl-2-pyridinylidène)-3-éthyl-1-formazano]-1,3-dihydroxyéthyl -benzimidazolium
   - Chlorure de 2-[5-(N-hydroxyéthyl-2-pyridinylidène)-3-phényl- 1 -formazano]- 1,3-dihydroxyéthyl -benzimidazolium
   - Chlorure de 2-[5-(N-hydroxyethyl-2-pyridinylidène)-3-isopropyl-1-formazano]-1,3-dihydroxyéthyl -benzimidazolium
   - Chlorure de 2-[5-(N-hydroxyéthyl-2-pyridinylidène)-3-(4'-méthoxyphényl)- 1 -formazano]-1,3-dihydroxyéthyl -benzimidazolium
   - Chlorure de 2-[5-(N-hydroxyéthyl-2-pyridinylidène)-3-(4'-hydroxyphényl)- 1 -formazano] 1 ,3-dihydroxyéthyl -benzimidazolium
   - Chlorure de 2-[5-(N-hydroxyéthyl-2-pyridinylidène)-3-(4'-N,N-diméthylaminophényl)-1-formazano]-1,3-dihydroxyéthyl-benzimidazolium
   - Chlorure de 2-[5-(N-carboxyéthyl-2-pyridinylidène)-1-formazano]- 1,3-dicarboxyéthyl -benzimidazolium
   - Chlorure de 2-[5-(N-carboxyéthyl-2-pyridinylidène)-3-méthyl-1-formazano]-1,3-dicarboxyéthyl -benzimidazolium
   - Chlorure de 2-[5-(N-carboxyéthyl-2-pyridinylidène)-3-éthyl-1 -formazano]-1,3-dicarboxyéthyl -benzimidazolium
   - Chlorure de 2-[5-(N-carboxyéthyl-2-pyridinylidène)-3-phényl-1-formazano]-1,3-dicarboxyéthyl -benzimidazolium
   - Chlorure de 2-[5-(N-carboxyéthyl-2-pyridinylidène)-3-isopropyl-1-formazano]- 1 ,3-dicarboxyéthyl -benzimidazolium
   - Chlorure de 2-[5-(N-carboxyéthyl-2-pyridinylidène)-3-4'-méthoxyphényl)- 1 -formazano]-1,3-dicarboxyéthyl -benzimidazolium
   - Chlorure de 2-[5-(N-carboxyéthyl-2-pyridinylidène)-3-(4'-hydroxyphényl)-1-formazano]-1,3-dicarboxyéthyl -benzimidazolium
   - Chlorure de 2-[5-(N-carboxyéthyl-2-pyridinylidène)-3-(4'-N,N-diméthylaminophényl)-1-formazano]-1,3-dicarboxyéthyl
   - benzimidazolium.
F. De préférence, les composés de formule I' sont choisis dans la famille définie par les composés pour lesquels W'₁ est un radical 2-benzimidazolium et W'₂ est un radical 4-pyridine. De manière encore plus préférée, ce sont les composés suivants :
   - Chlorure de 2-[5-(N-methyl-4-pyridinylidène)-1-formazano]-1,3-diméthyl-benzimidazolium
   - Chlorure de 2-[5-(N-méthyl-4-pyridinylidène)-3-méthyl-1-formazano]-1,3-diméthyl-benzimidazolium
   - Chlorure de 2-[5-(N-méthyl-4-pyridinylidène)-3-éthyl-1-formazano]-1,3-diméthyl-benzimidazolium
   - Chlorure de 2-[5-(N-methyl-4-pyridinylidène)-3-isopropyl-1-formazano]-1,3-diméthyl-benzimidazolium
   - Chlorure de 2-[5-(N-méthyl-4-pyridinylidène)-3-phényl-1-formazano]-1,3-diméthyl-benzimidazolium
   - Chlorure de 2-[5-(N-méthyl-4-pyridinylidène)-3-(4'-méthoxyphényl)-1-formazano]-1,3-diméthyl-benzimidazolium
   - Chlorure de 2-[5-(N-méthyl-4-pyridinylidène)-3-(4'-hydroxyphényl)-1-formazano]-1,3-diméthyl-benzimidazolium
   - Chlorure de 2-[5-(N-méthyl-4-pyridinylidène)-3-(4'-N,N-diméthylaminophényl)-1-formazano]-1,3-diméthyl-benzimidazolium
   - Chlorure de 2-[5-(N-hydroxyéthyl-4-pyridinylidène)-1-formazano]-1,3-dihydroxyéthylbenzimidazolium
   - Chlorure de 2-[5-(N-hydroxyethyl-4-pyridinylidène)-3-méthyl-1-formazano]-1,3-dihydroxyéthylbenzimidazolium
   - Chlorure de 2-[5-(N-hydroxyéthyl-4-pyridinylidène)-3-éthyl-1-formazano]-1,3-dihydroxyéthyl-benzimidazolium
   - Chlorure de 2-[5-(N-hydroxyéthyl-4-pyridinylidène)-3-phényl-1-formazano]-1,3-dihydroxyéthyl -benzimidazolium
   - Chlorure de 2-[5-(N-hydroxyéthyl-4-pyridinylidène)-3-isopropyl- 1 -formazano]-1,3-dihydroxyéthyl -benzimidazolium
   - Chlorure de 2-[5-(N-hydroxyéthyl-4-pyridinylidène)-3-(4'-méthoxyphényl)-1-formazano]-1,3-dihydroxyéthyl -benzimidazolium
   - Chlorure de 2-[5-(N-hydroxyéthyl-4-pyridinylidène)-3-(4'-hydroxyphényl)-1-formazano]-1,3-dihydroxyéthyl -benzimidazolium
   - Chlorure de 2-[5-(N-hydroxyéthyl-4-pyridinylidène)-3-(4'-N,N-diméthylaminophényl)-1-formazano]-1,3-dihydroxyéthyl-benzimidazolium
   - Chlorure de 2-[5-(N-carboxyéthyl-4-pyridinylidène)-1-formazano]-1,3-dicarboxyéthyl -benzimidazolium
   - Chlorure de 2-[5-(N-carboxyéthyl-4-pyridinylidène)-3-méthyl-1-formazano]-1,3-dicarboxyéthyl -benzimidazolium
   - Chlorure de 2-[5-(N-carboxyéthyl-4-pyridinylidène)-3-éthyl-1-formazano]-1,3-dicarboxyéthyl-benzimidazolium
   - Chlorure de 2-[5-(N-carboxyéthyl-4-pyridinylidène)-3-phényl-1-formazano]-1,3-dicarboxyéthyl -benzimidazolium
   - Chlorure de 2-[5-(N-carboxyéthyl-4-pyridinylidène)-3-isopropyl-1-formazano]-1,3-dicarboxyéthyl -benzimidazolium
   - Chlorure de 2-[5-(N-carboxyéthyl-4-pyridinylidène)-3-(4'-méthoxyphényl)-1-formazano]-1,3-dicarboxyéthyl -benzimidazolium
   - Chlorure de 2-[5-(N-carboxyéthyl-4-pyridinylidène)-3-(4'-hydroxyphényl)-1-formazano]-1,3-dicarboxyéthyl -benzimidazolium.
   - Chlorure de 2-[5-(N-carboxyéthyl-4-pyridinylidène)-3-(4'-N,N-diméthylaminophényl)-1-formazano]-1,3-dicarboxyéthyl-benzimidazolium
G. De préférence, les composés de formule I' sont choisis dans la famille définie par les composés pour lesquels W'₁ est un radical 2-imidazolium et W'₂ est un radical 2-pyridine. De manière encore plus préférée, ce sont les composés suivants :
   Chlorure de 2-[5-(N-méthyl-2-pyridinylidène)-1-formazano]-1,3-diméthyl-imidazolium
   - Chlorure de 2-[5-(N-méthyl-2-pyridinylidène)-3-méthyl-1-formazano]-1,3-diméthyl-imidazolium
   - Chlorure de 2-[5-(N-méthyl-2-pyridinylidène)-3-éthyl- 1-formazano]-1,3-diméthyl-imidazolium
   - Chlorure de 2-[5-(N-méthyl-2-pyridinylidène)-3-isopropyl-1-formazano]-1,3-diméthyl-imidazolium
   - Chlorure de 2-[5-(N-méthyl-2-pyridinylidène)-3-phényl-1-formazano]-1,3-diméthyl-imidazolium
   - Chlorure de 2-[5-(N-méthyl-2-pyridinylidène)-3-(4'-méthoxyphényl)-1-formazano]-1,3-diméthyl-imidazolium
   - Chlorure de 2-[5-(N-méthyl-2-pyridinylidène)-3-(4'-hydroxyphényl)-1-formazano]-1,3-diméthyl-imidazolium
   - Chlorure de 2-[5-(N-méthyl-2-pyridinylidène)-3-(4'-N,N-diméthylaminophényl)-1-formazano]-1,3-diméthyl-imidazolium.
   - Chlorure de 2-[5-(N-hydroxyéthyl-2-pyridinylidène)-1-formazano]-1,3-dihydroxyéthylimidazolium
   - Chlorure de 2-[5-(N-hydroxyéthyl-2-pyridinylidène)-3-méthyl-1-formazano]-1,3-dihydroxyéthylimidazolium
   - Chlorure de 2-[5-(N-hydroxyéthyl-2-pyridinylidène)-3-éthyl-1-formazano]-1,3-dihydroxyéthyl-imidazolium
   - Chlorure de 2-[5-(N-hydroxyéthyl-2-pyridinylidène)-3-phényl-1-formazano]-1,3-dihydroxyéthyl -imidazolium
   - Chlorure de 2-[5-(N-hydroxyéthyl-2-pyridinylidène)-3-isopropyl- 1 -formazano]-1,3-dihydroxyéthyl -imidazolium
   - Chlorure de 2-[5-(N-hydroxyéthyl-2-pyridinylidène)-3-(4'-méthoxyphényl)- 1 -formazano]-1,3-dihydroxyéthyl -imidazolium
   - Chlorure de 2-[5-(N-hydroxyéthyl-2-pyridinylidène)-3-(4'-hydroxyphényl)- 1 -formazano]-1,3-dihydroxyéthyl -imidazolium
   - Chlorure de 2-[5-(N-hydroxyéthyl-2-pyridinylidène)-3-(4'-N,N-diméthylaminophényl)-1-formazano]-1,3-dihydroxyéthyl-imidazolium
   - Chlorure de 2-[5-(N-carboxyéthyl-2-pyridinylidène)-1-formazano]-1,3-dicarboxyéthyl -imidazolium
   - Chlorure de 2-[5-(N-carboxyéthyl-2-pyridinylidène)-3-méthyl-1 -formazano]-1,3-dicarboxyéthyl -imidazolium
   - Chlorure de 2-[5-(N-carboxyéthyl-2-pyridinylidène)-3-éthyl-1-formazano]-1,3-dicarboxyéthyl -imidazolium
   - Chlorure de 2-[5-(N-carboxyéthyl-2-pyridinylidène)-3-phényl-1 -formazano]-1,3-dicarboxyéthyl -imidazolium
   - Chlorure de 2-[5-(N-carboxyéthyl-2-pyridinylidène)-3-isopropyl-1-formazano]-1,3-dicarboxyéthyl -imidazolium
   - Chlorure de 2-[5-(N-carboxyéthyl-2-pyridinylidène)-3-(4'-méthoxyphényl)-1 -formazano]-1,3-dicarboxyéthyl -imidazolium
   - Chlorure de 2-[5-(N-carboxyéthyl-2-pyridinylidène)-3-(4'-hydroxyphényl)-1-formazano]-1,3-dicarboxyéthyl -imidazolium
   - Chlorure de 2-[5-(N-carboxyéthyl-2-pyridinylidène)-3-(4'-N,N-diméthylaminophényl)-1-formazano]-1,3-dicarboxyéthyl-imidazolium
H. De préférence, les composés de formule I' sont choisis dans la famille définie par les composés pour lesquels W'₁ est un radical 2-imidazolium et W'₂ est un radical 4-pyridine. De manière encore plus préférée, ce sont les composés suivants :
   - Chlorure de 2-[5-(N-méthyl-4-pyridinylidène)-1-formazano]-1,3-diméthyl-imidazolium
   - Chlorure de 2-[5-(N-méthyl-4-pyridinylidène)-3-méthyl-1-formazano]-1,3-diméthyl-imidazolium
   - Chlorure de 2-[5-(N-méthyl-4-pyridinylidène)-3-éthyl-1-formazano]-1,3-diméthyl-imidazolium
   - Chlorure de 2-[5-(N-méthyl-4-pyridinylidène)-3-isopropyl-1-formazano]-1,3-diméthyl-imidazolium
   - Chlorure de 2-[5-(N-méthyl-4-pyridinylidène)-3-phényl-1-formazano]-1,3-diméthyl-imidazolium
   - Chlorure de 2-[5-(N-méthyl-4-pyridinylidène)-3-(4'-méthoxyphényl)-1-formazano]-1,3-diméthyl-imidazolium
   - Chlorure de 2-[5-(N-méthyl-4-pyridinylidène)-3-(4'-hydroxyphényl)-1-formazano]-1,3-diméthyl-imidazolium
   - Chlorure de 2-[5-(N-méthyl-4-pyridinylidène)-3-(4'-N,N-diméthylaminophényl)-1-formazano]-1,3-diméthyl-imidazolium
   - Chlorure de 2-[5-(N-hydroxyéthyl-4-pyridinylidène)-1-formazano]- 1,3-dihydroxyéthylimidazolium
   - Chlorure de 2-[5-(N-hydroxyéthyl-4-pyridinylidène)-3-méthyl-1-formazano]-1,3-dihydroxyéthylimidazolium
   - Chlorure de 2-[5-(N-hydroxyeéhyl-4-pyridinylidène)-3-éthyl-1 -formazano]-1,3-dihydroxyéthyl -imidazolium
   - Chlorure de 2-[5-(N-hydroxyéthyl-4-pyridinylidène)-3-phényl-1-formazano]-1,3-dihydroxyéthyl -imidazolium
   - Chlorure de 2-[5-(N-hydroxyéthyl-4-pyridinylidène)-3-isopropyl-1-formazano]-1,3-dihydroxyéthyl -imidazolium
   - Chlorure de 2-[5-(N-hydroxyéthyl-4-pyridinylidène)-3-(4'-méthoxyphényl)-1-formazano]-1,3-dihydroxyéthyl -imidazolium
   - Chlorure de 2-[5-(N-hydroxyéthyl-4-pyridinylidène)-3-(4'-hydroxyphényl)-1 -formazano]-1,3-dihydroxyéthyl -imidazolium
   - Chlorure de 2-[5-(N-hydroxyéthyl-4-pyridinylidène)-3-(4'-N,N-diméthylaminophényl)-1-formazano]-1,3-dihydroxyéthyl-imidazolium
   - Chlorure de 2-[5-(N-carboxyéthyl-4-pyridinylidène)-1-formazano]-1,3-dicarboxyéthyl -imidazolium
   - Chlorure de 2-[5-(N-carboxyéthyl-4-pyridinylidène)-3-méthyl-1-formazano]-1,3-dicarboxyéthyl -imidazolium
   - Chlorure de 2-[5-(N-carboxyéthyl-4-pyridinylidène)-3-éthyl-1 -formazano]-1, 3-dicarboxyéthyl -imidazolium
   - Chlorure de 2-[5-(N-carboxyéthyl-4-pyridinylidène)-3-phényl-1-formazano]-1,3-dicarboxyéthyl -imidazolium
   - Chlorure de 2-[5-(N-carboxyéthyl-4-pyridinylidène)-3-isopropyl-1-formazano]-1,3-dicarboxyéthyl -imidazolium
   - Chlorure de 2-[5-(N-carboxyéthyl-4-pyridinylidène)-3-(4'-méthoxyphényl)-1-formazano]-1,3-dicarboxyéthyl -imidazolium
   - Chlorure de 2-[5-(N-carboxyéthyl-4-pyridinylidène)-3-(4'-hydroxyphényl)-1-formazano]-1,3-dicarboxyéthyl -imidazolium
   - Chlorure de 2-[5-(N-carboxyéthyl-4-pyridinylidène)-3-(4'-N,N-diméthylaminophényl)-1-formazano]-1,3-dicarboxyéthyl-imidazolium.

Les composés et familles de composés nouveaux présentés dans les listes ci-dessus peuvent être utilisés en tant que colorants directs dans les compositions tinctoriales selon l'invention.

La présente invention concerne encore un procédé de préparation du composé de formule (I') dans lequel on fait réagir au moins deux équivalents d'au moins une hydrazone choisie parmi les hydrazones de formule A et les hydrazones de formule B : 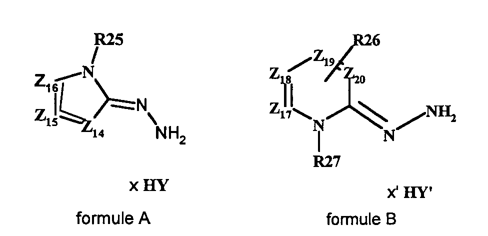 dans lesquelles Z₁₄, Z₁₅, Z₁₆, R₂₅ ont respectivement les mêmes significations que Z'₇, Z'₈, Z'₁₃, R'₅ de la formule IV',
Y étant un anion organique ou minéral,
x étant un entier allant de 1 à 3,
Z₁₇, Z₁₈, Z₁₉, Z₂₀, R₂₆, R₂₇ ont respectivement les mêmes significations que Z'₉, Z'₁₀, Z'₁₁, Z'₁₂, R'₈, R'₇ de la formule V',
Y' étant un anion organique ou minéral,
x' étant un entier allant de 1 à 3,
avec un équivalent d'un aldéhyde de formule R₂₃CHO, R₂₃ ayant la même signification que R'₀ de la formule I'. On peut ainsi faire réagir deux équivalents de la même hydrazone ou de deux hydrazones différentes.

Selon une variante préférée, l'aldéhyde de formule R₂₃CHO est généré dans le milieu réactionnel à partir d'un précurseur d'aldéhyde de formule R'₂₃CH₂OH en présence d'un système oxydant, R'₂₃ a alors la même signification que R'₀ de la formule I'.

Ce système oxydant peut être un oxydant chimique ou un oxydant biocatalytique tel qu'une enzyme.

De façon particulière, le procédé de préparation du composé de formule (I') selon la présente invention, ainsi que le procédé de préparation du composé de formule (I), peut être mis en oeuvre en présence d'un précurseur d'aldéhyde de formule R'₂₃CH₂OH et d'au moins une enzyme capable de générer un aldéhyde à partir du précurseur d'aldéhyde de formule R'₂₃CH₂OH.

Le procédé de synthèse des composés de formule (I) par voie enzymatique défini ci-dessus s'effectue en faisant réagir au moins deux équivalents d'au moins une hydrazone choisie parmi les hydrazones de formule A et les hydrazones de formule B dans lesquelles Z₁₄, Z₁₅, Z₁₆, R₂₅ ont respectivement les mêmes significations que Z₇, Z₈, Z₁₃, R₅ de la formule IV,
Y étant un anion organique ou minéral,
x étant un entier allant de 1 à 3,
Z₁₇, Z₁₈, Z₁₉, Z₂₀, R₂₆, R₂₇ ont respectivement les mêmes significations que Z₉, Z₁₀, Z₁₁, Z₁₂, R₈, R₇ de la formule V,
Y' étant un anion organique ou minéral,
x' étant un entier allant de 1 à 3,
avec un équivalent d'un aldéhyde de formule R₂₃CHO, R₂₃ ayant la même signification que R₀ de la formule I.

La présente invention a encore pour objet un tel procédé.

Selon les réactifs utilisés, la réaction est conduite avec ou sans système oxydant, avec ou sans cofacteur pour l'enzyme, avec ou sans système de régénération du cofacteur.

Au sens de la présente demande, on entend par "sans système oxydant", qu'aucun système oxydant autre que l'oxygène atmosphérique n'est utilisé.

De préférence, la réaction est effectuée en milieu aérobie à pH compris entre 3 et 11 et à température comprise entre 6°C et 80°C.

De manière plus générale, le précurseur d'aldéhyde pouvant être utilisé pour préparer le composé de formule (I') ou le composé de formule (I), peut être choisi parmi les alcools primaires, la sarcosine, le 4-hydroxymandelate, la N6-méthyl-lysine, la diméthylglycine, le méthylglutamate, les 2-oxo-acides par exemple le 2-oxo-acide pyruvate, le benzoylformate, le phénylpyruvate, la thréonine. De préférence, il sera choisi parmi les alcools primaires.

Les enzymes capables de générer un aldéhyde à partir de ce précurseur d'aldéhyde, peuvent notamment être choisis parmi les alcool déshydrogenases EC 1.1.1.1, les alcool déshydrogénases EC 1.1.1.2, les alcool déshydrogénases EC 1.1.1.71, les alcool aromatique déshydrogénases EC 1.1.1.90 encore appelées aryl alcool déshydrogénases, les alcool aromatique déshydrogénases EC 1.1.1.97, les alcool 3-hydroxybenzylique déshydrogénases EC 1.1.1.97, les alcool coniferylique déshydrogénases EC 1.1.1.194, les alcool cinnamylique déshydrogénases EC 1.1.1.195, les méthanol déshydrogénases EC 1.1.1.244, les alcool aromatique oxydases EC 1.1.3.7 encore appelées aryl alcool oxydases, les alcool oxydases EC 1.1.3.13, les 4-hydroxymandelate oxydases EC 1.1.3.19, les alcool à longue chaîne hydrocarbonée oxydases EC 1.1.3.20, les méthanol oxydases EC 1.1.3.31, les alcool déshydrogénases EC 1.1.99.20, les sarcosinase oxydases EC 1.5.3.1, les N6-méthyl-lysine oxydases EC 1.5.3.4, les diméthylglycine oxidases EC 1.5.3.10, les sarcosine déshydrogénases EC 1.5.99.1, les diméthylglycine déshydrogénases EC 1.5.99.2, les méthylglutamate déshydrogénases EC 1.5.99.5, les 2-oxo-acides décarboxylases EC 4.1.1.1, les benzoylformate décarboxylases EC 4.1.1.7, les phénylpyruvate décarboxylases EC 4.1.1.43, les thréonine aldolase EC 4.1.2.5.

On peut également citer les enzymes suivantes capables de générer un aldéhyde dont le substrat préféré est précisé entre paranthèses : la N-méthyl L amino acide oxydase EC 1.5.3.2 (N-méthyl-L-amino acide), la triméthylamine déshydrogénase EC 1.5.99.7 (triméthylamine), la diméthylamine déshydrogénase EC 1.5.99.10 (diméthylamine), la nitroéthane oxydase EC 1.7.3.1 (nitroéthane), l'indole 2,3-dioxygénase EC 1.13.11.17 (indole), la taurine dioxygénase EC 1.14.11.17 (taurine), l'acétoïne ribose 5 phosphate transaldolase EC 2.2.1.4 (3-hydroxybutan-2-one), la diamine aminotransférase EC 2.6.1.29 (alpha oméga diamine + 2-oxoglutérate), l'alkénylglycérophosphocholine hydrolase EC 3.3.2.2 (alkénylglycérophosphocholine), l'alkénylglycérophosphoéthanolamine hydrolase EC 3.3.2.5 (alkénylglycérophosphocholine), l'alkylalidase EC 3.8.1.1 (halométhane), la phosphonoacétaldéhyde hydrolase EC 3.11.1.1 (phosphonoacétaldéhyde), l'indolepyruvate décarboxylase EC 4.1.1.74 (3-indol3-yl pyruvate), la mandelonitrile lyase EC 4.1.2.10 (mandelonitrile), l'hydroxymandelonitrile lyase EC 4.1.2.11 (hydroxymandelonitrile), la kétopantoaldolase EC 4.1.2.12 (2-hydroxy-2-isopropylbutanedioate), la diméthylaniline-N-oxyde aldolase EC 4.1.2.24 (diméthylaniline-N-oxyde), la phénylsérine aldolase EC 4.1.2.26 (phénylsérine), la sphinganine-1-phosphate aldolase EC 4.1.2.27 (sphinganine-1-phosphate), 17-alpha hydroxyprogestérone aldolase EC 4.1.2.30 (17-alpha hydroxyprogestérone), la triméthylamine -oxyde aldolase EC 4.1.2.23 (triméthylamine -oxyde), la fucostérol -époxyde lyase EC 4.1.2.23 (fucostérol -époxyde), (3E)-4-(2-carboxyphényl)-2-oxobut-3-énoate aldolase EC 4.1.2.34 ((3E)-4-(2-carboxyphényl)-2-oxobut-3-énoate), la lactate aldolase EC 4.1.2.36 (lactate), la benzoïne aldolase EC 4.1.2.38 (benzoïne), l'octoamine déshydratase EC 4.2.1.87 (1-(4-hydroxyphényl)2-aminoéthanol), la synéphrine déshydratase EC 4.2.1.88 (1-(4-hydroxyphényl)-2-(méthylamino)éthanol), l'éthanolamine -phosphate phospho-lyase EC 4.2.3.2 (éthanolamine -phosphate), l'éthanolamine ammonia-lyase EC 4.3.1.7 (éthanolamine), le dichlorométhane déshalogénase EC 4.5.1.3 (dichlorométhane), la styrène -oxyde isomérase EC 5.3.99.7 (styrène oxyde)

L'enzyme capable de générer un aldéhyde à partir du précurseur d'aldéhyde utilisée dans la composition tinctoriale selon l'invention peut être issue d'un extrait de végétaux, d'animaux, de microorganismes (bactérie, champignon, levure, microalgue) ou de virus, de cellules différenciées ou dédifférenciées, obtenues *in vivo* ou *in vitro,* modifiées ou non modifiées génétiquement, ou synthétiques (obtenues par synthèse chimique ou biotechnologique).

A titre d'exemples d'enzymes utiles on peut citer en particulier les genres *Plectranthus, Pinus, Gastropode, Manduca, Pichia, Candida, Pleurotus, Pseudomonas,* et de façon encore plus particulière les espèces suivantes : *Plectranthus colleoides, Pinus strobus* qui est une espèce d'origine végétale, *Gastropode mollusc, Manduca sexta* qui sont d'origine animale, *Pichia pastoris* et *Candida boidinii* qui sont des levures, *Pleurotus pulmonarius* qui est un champignon, et *Pseudomonas pseudoalcaligenes* qui est une bactérie.

Le choix de l'enzyme est fonction de la nature du précurseur d'aldéhyde. Par exemple, lorsque le précurseur d'aldéhyde est un alcool, alors l'enzyme est choisie parmi les enzymes capables de générer un aldéhyde à partir de cet alcool. Lorsque le précurseur d'aldéhyde est le méthylglutamate, alors l'enzyme est une méthylglutamate déshydrogénase.

Selon une variante préférée, le précurseur d'aldéhyde est un alcool primaire et l'enzyme est une enzyme capable de générer l'aldéhyde à partir d'un alcool. Par exemple, lorsque l'alcool primaire est un alcool aliphatique en C₁ à C₆, alors l'enzyme capable de générer l'aldéhyde est choisie parmi les alcool oxydases, les alcool déshydrogénases, les méthanol déshydrogénases, les méthanol oxydases. Lorsque l'alcool primaire est l'alcool benzylique, le 4-terbutyl benzylique alcool, le 3-hydroxy-4-méthoxybenzyl alcool, le vératryl alcool, le 4-méthoxybenzyl alcool, l'alcool cinnamique, le 2,4 hexadiéne-1-ol, on peut utiliser comme précurseur d'aldéhyde les aryl alcool oxydases ou les alcool aromatique déshydrogénases.

Pour les enzymes deshydrogénases, il est indispensable d'inclure le ou les cofacteurs nécessaire à leur activité, plus précisément du NAD⁺ ou NADP⁺ ou d'autres molécules succeptibles d'agir comme accepteur d'électrons. L'ajout d'un système de régénération des cofacteurs peut être utilisé pour des raisons réactionnelles ou économiques. Ce système de régénération peut etre enzymatique, chimique ou électrochimique.

Les oxydants les plus divers sont utilisables pour la mise en oeuvre de ce procédé : l'eau oxygénée, les peracides organiques tels que l'acide peracétique, les persels tels que permanganate, perborate, les persulfates, les chromates ou les bichromates, les hypochlorites, les hypobromites, les ferricyanures, les peroxydes tels que les bioxydes de manganèse ou de plomb. Préférentiellement l'eau oxygénée sera utilisée.

La concentration en substrat de l'enzyme (précurseur d'aldéhyde) peut être comprise entre 0,001 M et 6 M de préférence entre 0,1 M et 4M.

La réaction peut être réalisée entre pH 3 et pH 11 de préférence entre pH 5 et pH 9.5.

La température pour la réaction peut être comprise entre 10°C et 80°C de préférence entre 20°C et 65°C.

La concentration du milieu réactionnel en hydrazone est comprise entre 0,01M et 3 M de préférence entre 0,1M et 1M.

La teneur en cofacteur pour les dites enzymes peut être comprise entre 0,01mM et 1 M de préférence 0,1mM et 10mM.

Lors de la mise en oeuvre de la synthèse des composés de formules (I') et (I), les réactifs : la ou les hydrazones, la ou les enzymes, le substrat pour la dite enzyme, et/ou le cofacteur pour la dite enzyme, et ou l'oxydant et/ou le système de régénération du cofacteur sont mélangés, le pH et la température sont ajustés.

La présente invention concerne encore un procédé de préparation du composé de formule (I') dans lequel on fait réagir un composé de formule (F1) ou (F2) 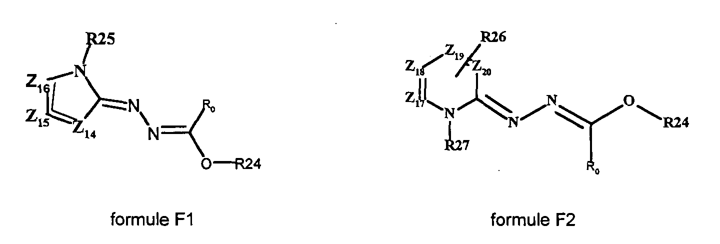 avec Z₁₄, Z₁₅, Z₁₆, R₀, R₂₄, R₂₅ pour la formule F1 et Z₁₇, Z₁₈, Z₁₉, Z₂₀, R₀, R₂₄, R₂₆ pour la formule F2 ont les significations décrites précédemment, en présence d'un équivalent d'une hydrazone de formule (A) ou (B).

Selon une variante préférée, les composés de formule (F1) ou (F2) peuvent être obtenus par réaction d'un composé de formule R"₂₃C(OR₂₄)₃ et d'une hydrazone de formule (A) ou (B) en présence ou non d'un solvant protique dont le point d'ébullition varie entre 66°C et 180°C. R"₂₃ a la même signification que R'₀ de la formule (I') et R₂₄ représente un radical choisi parmi les groupes méthyle et éthyle.

De préférence, la réaction est effectuée en présence de triéthylorthoformiate et triméthylorthoacétate à une température comprise entre 0°C et 150°C pendant une période comprise entre 30 minutes et 12 heures.

De préférence, le solvant protique est choisi parmi H₂O, éthanol, et méthanol.

### EXEMPLES DE SYNTHESE

### A) Procédé de synthèse mettant en oeuvre au moins une hydrazone de formule (A) et/ou (B) et un précurseur d'aldéhyde de formule R'₂₃CH₂OH

Les composés cités dans la formule (I') peuvent être synthétisés en mettant en réaction au moins une hydrazone telle que définie dans les formules (A) et (B) avec une ou plusieurs enzymes de type : alcool oxydase, alcool deshydrogénase, oxydoréductase, transférase, et/ou lyase, et le ou les substrats de formule R'₂₃CH₂OH pour la ou lesdites enzymes avec ou sans oxydant, avec ou sans cofacteur pour l'enzyme, avec ou sans système de régénération du dit cofacteur. La réaction est réalisée entre le pH 3 et pH 11 en milieu aérobie à une température entre 10°C et 80°C.

### Exemple 1

| | |
|---|---|
| Ethanol (100%) | 2M |
| Alcool oxidase (*Pichia pastoris*) | 1000U |
| N-hydroxyethyl pyridone 2-hydrazone | 0,3M |
| Tampon 30mM KH₂PO₄/K₂HPO₄ pH 7 | qsp 100ml |

### Exemple 2

| | |
|---|---|
| Methanol (100%) | 2,5M |
| Alcool oxidase *(Candida boidinii)* | 2000U |
| 4-methoxy-N-methyl pyridone 2-hydrazone | 0,3M |
| Tampon 30mM KH₂PO₄/K₂HPO₄ pH 7 | qsp 100ml |

### Exemple 3

| | |
|---|---|
| Ethanol (100%) | 2M |
| Extrait *plectranthus colloides* | 0,4g |
| 4-methoxy-N-methyl pyridone 2-hydrazone | 0,3M |
| Tampon 30mM KH₂PO₄/K₂HPO₄ pH 7 | qsp 100ml |

### Exemple 4

| | |
|---|---|
| alcool benzylique (100%) | 1M |
| Extrait Pleurotus pulmonarius | 0,4g |
| N-hydroxyethyl pyridone 2-hydrazone | 0.3M |
| Tampon 30mM KH₂PO₄/K₂HPO₄ pH 9 | qsp 100ml |

### Exemple 5

| | |
|---|---|
| Méthanol (100%) | 2,5M |
| Alcool dehydrogenase *(Saccharomyces cervisiae)* | 1000U |
| NAD+ | 10mM |
| N-methyl- pyridone-2-hydrazone | 0,3M |
| Eau oxygénée 3% | 0,5ml |
| Tampon 30mM KH₂PO₄/K₂HPO₄ pH 9 | qsp 100ml |

### Exemple 6

| | |
|---|---|
| Ethanol (100%) | 2,5M |
| Alcool oxidase *(Pichia pastoris)* | 2000U |
| 1,3 dimethyl imidazolidone 2-hydrazone | 0,3M |
| Tampon 30mM KH₂PO₄/K₂HPO₄ pH 8.5 | qsp 100ml |

### Exemple 7

| | |
|---|---|
| Méthanol (100%) | 2,5M |
| Alcool oxidase *(Pichia pastoris)* | 2000U |
| 1,3 dimethyl imidazolidone 2-hydrazone | 0,3M |
| Tampon 30mM KH₂PO₄/K₂HPO₄ pH 8.5 | qsp 100ml |

### Exemple 8

| | |
|---|---|
| Méthanol (100%) | 2,5M |
| Alcool oxydase *(Pichia pastoris)* | 2000U |
| N-méthyl-pyridone-2-hydrazone | 0,3M |
| N-hydroxyéthylpyridone 2-hydrazone | 0,3M |
| Tampon 30mM KH₂PO₄/K₂HPO₄ pH 8.5 | qsp 100ml |

### Exemple 9

| | |
|---|---|
| Ethanol | 1M |
| Alcool oxydase*(Pichia pastoris)* | 50 U |
| (2-methyl-6-methoxy-4H-pyridazin-3-ylidene)-hydrazine | 0.1 M |
| Tampon 200 mM KH₂PO₄/K₂HPO₄ pH 8.5 | qsq 10 ml |

### Exemple 10

| | |
|---|---|
| Ethanol | 1M |
| Alcool oxydase*(Pichia pastoris)* | 50 U |
| (2-methyl-6-pyrrolidon-1-yl-4H-pyridazin-3-ylidene)-hydrazine | 0.1 M |
| Tampon 200 mM KH₂PO₄/K₂HPO₄ pH 8.5 | qsq 10 ml |

### Exemple 11

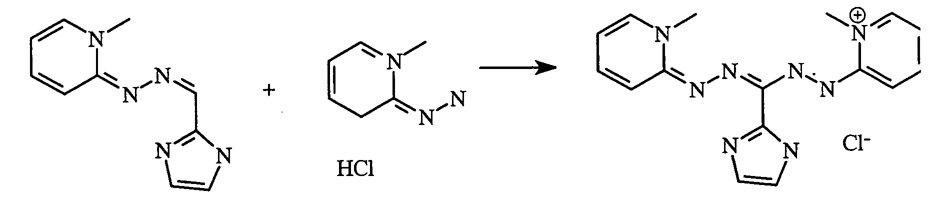

La réaction suivante a été menée :

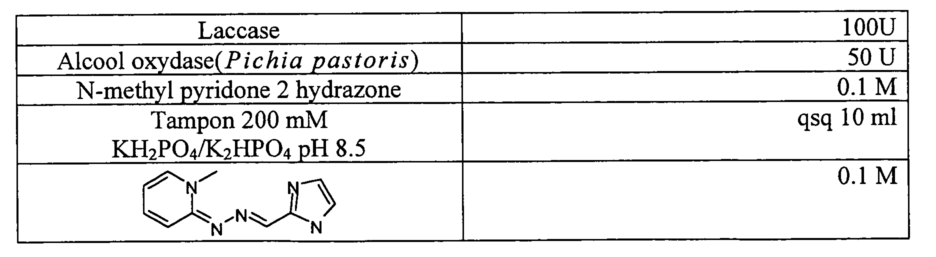

Une coloration apparaît entre 1 minute et 24 heures à température ambiante.

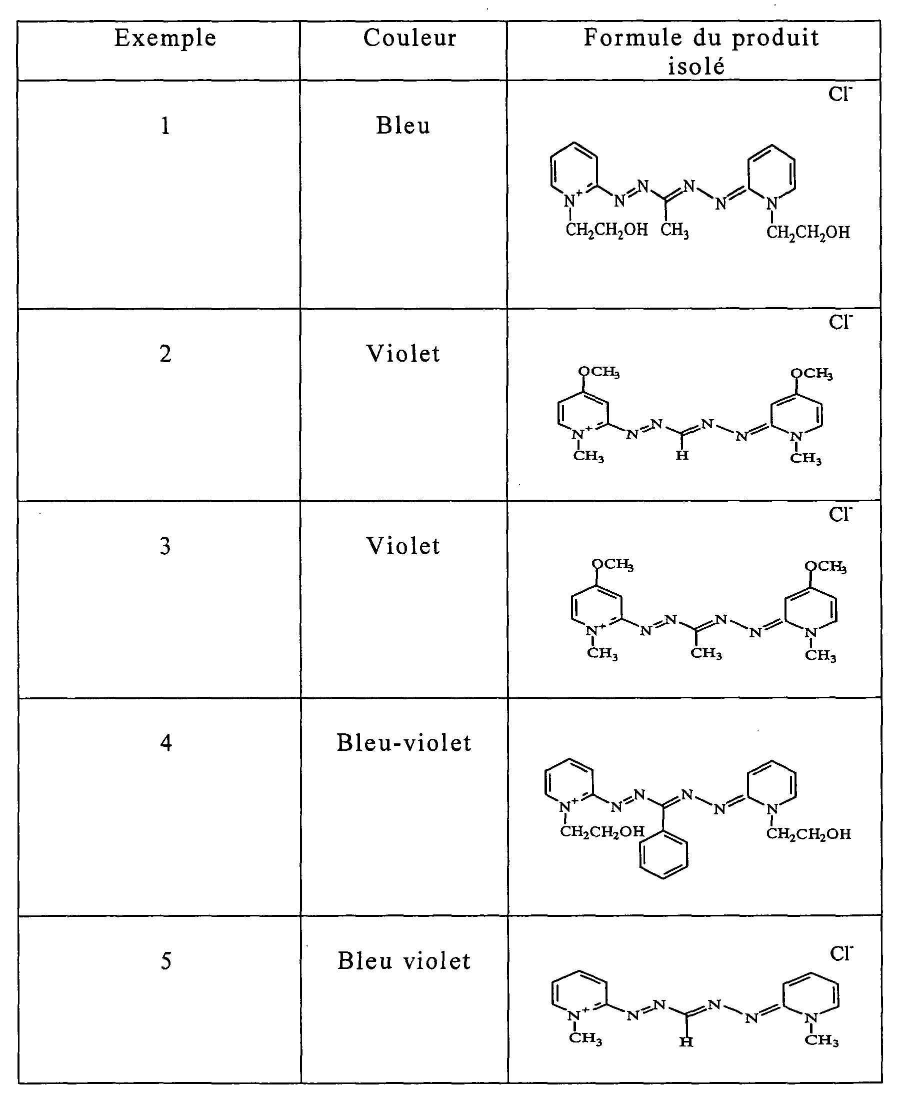

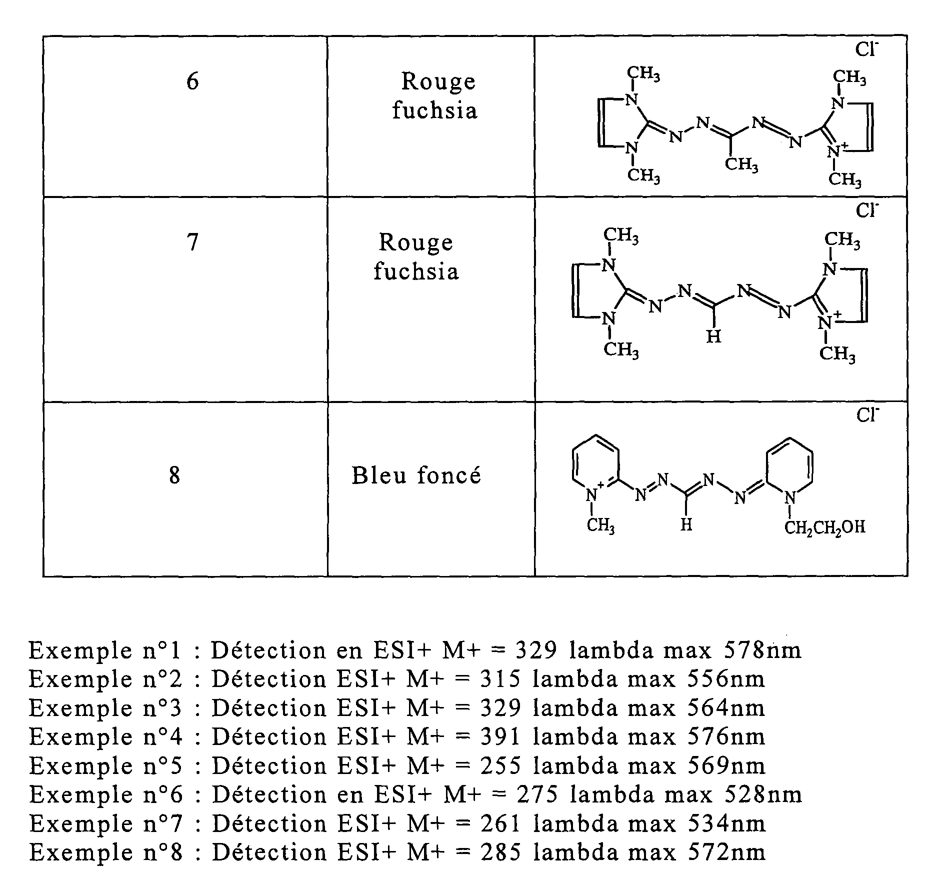

Une coloration apparaît entre 24 heures et 72 heures à température ambiante.

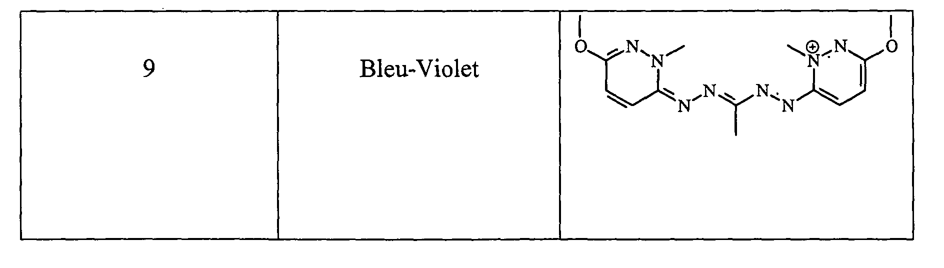

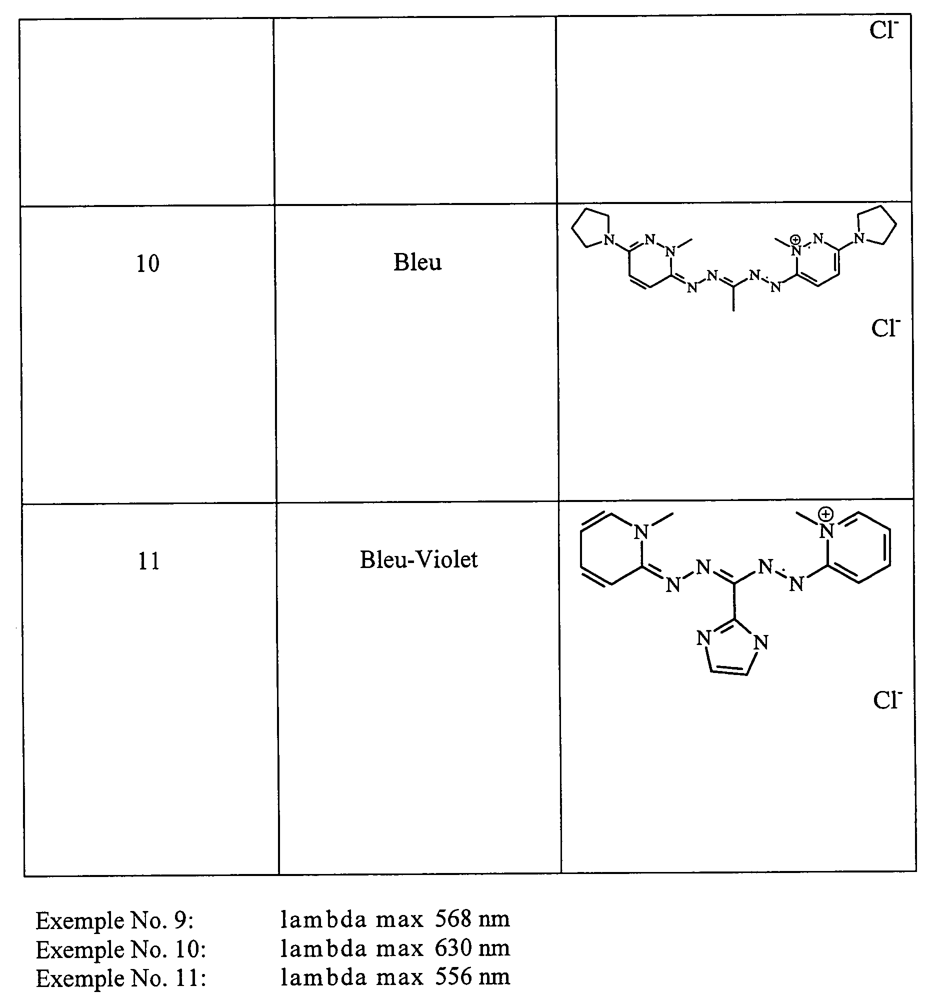

Les produits isolés après réaction correspondent aux composés de formule générale (I').

### B) Procédé de synthèse mettant en oeuvre au moins une hydrazone de formule (A) et/ou (B) et un aldéhyde de formule R₂₃CHO

Ce procédé de synthèse se déroule en deux étapes :
1- Une hydrazone de formule (A) ou (B) est au préalable mise en réaction en présence d'un aldéhyde de formule R₂₃CHO d'au moins un solvant polaire et d'un sel organique. Un composé de formule (A') ou (B') représenté ci-dessous est formé et isolé :

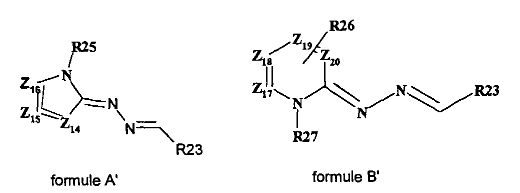

Les significations des radicaux Z₁₄, Z₁₅, Z₁₆, Z₁₇, Z₁₈, Z₁₉, Z₂₀, R₂₃, R₂₅, R₂₆, R₂₇ des composés de formule (A') ou (B') sont les mêmes que celles des composés de formule (A) et (B).

### EXEMPLES DE SYNTHÈSE DES FORMULES A' ET B' :

### Synthèses réalisées :

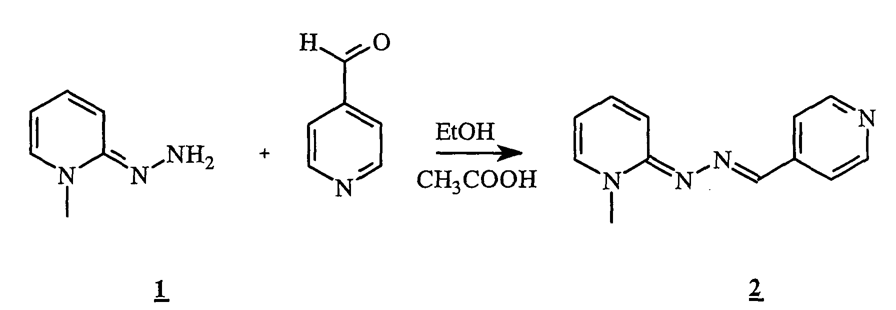

Dans un ballon on a mis sous agitation à 45°C pendant 30 minutes l'hydrazone 1 (1 g, 4,6 mmoles) en présence de 4-pyridinecarboxaldéhyde (0,7 ml, 7 mmoles) de 0,5 ml d'acide acétique et 6 ml d'éthanol. Après réaction, le milieu réactionnel a été concentré à sec. On a repris le résidu en ajoutant 50 ml d'acétate d'éthyle. Le précipité obtenu a été filtré puis séché au dessicateur. On a obtenu 1 g d'une poudre jaune.

### Analyse :

MS(ES⁺) : 213 (MH⁺)

RNM (¹H, 400 Mhz : 4,21 ppm (s, CH₃) ; 7,36 PPM (m, 1 H); 8,26 ppm (m, 2 H) ; 8,37 ppm (m, 1 H) ; 8,47 ppm (m, 2 H) ; 8,80 ppm (s, 1 H) ; 8,91 ppm (m, 2 H).

Suivant le même protocole opératoire, ont été synthétisés les produits suivants :

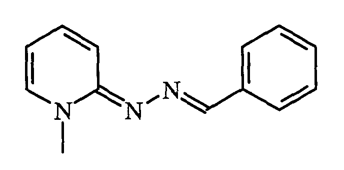

### Analyse :

Poudre légèrement colorée
Rdt : 98%
MS(ES⁺) : 212 (MH⁺)

RNM (¹H, 400 Mhz : 4,1 ppm (s, CH₃) ; 7,16 ppm (m, 1 H) ; 7,49 ppm (m, 2 Har) ; 7,81 ppm (m, 2 Har) ; 7,97 ppm (m, 1 H) ; 8,12 ppm (m, 1 Har) ; 8,35 ppm (m, 1 H) ; 8,97 ppm (s, 1 H).

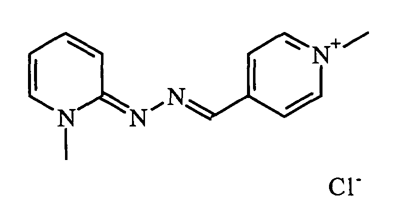

### Analyse :

Poudre jaune
Rdt : quantitatif
MS(ES⁺) : 227 (M⁺)

RNM (¹H, 400 Mhz : 4,2 ppm (s, CH₃) ; 4,4 ppm (m, 1 H) ; 7,37 ppm (m, 2 H) ; 8,26 ppm (m, 2 H) ; 8,44 ppm (m, 2 H) ; 8,75 ppm (s, 1 H) ; 8,93 ppm (m, 2 H).

2- La réaction est réalisée entre le pH 3 et pH 11 de préférence entre pH 5 et pH 9,5 en milieu aérobie à une température comprise entre 10°C et 80°C de préférence entre 20°C et 65°C. Les oxydoréductases utilisées seront choisies parmi les enzymes suivantes du groupe EC 1.10.3 à titre d'exemple catechol oxydase EC 1.10.3.1, Laccase EC 1.10.3.2, L ascorbate oxydase EC 1.10.3.3 et aminophenol oxydase EC 1.10.3.4.

Le composé de formule (A') ou (B') est le substrat pour la ou les dites enzymes. Un médiateur peut être introduit dans le mélange réactionnel afin d'améliorer la vitesse de réaction. Ledit médiateur sert de réactif intermédiaire entre l'enzyme et le composé de formule (A') ou (B') ou le composé de formule (A) ou (B).

A titre d'exemples de médiateurs utilisables, on pourra citer les 1-hydroxybenzotriazole, ABTS, TEMPO. La concentration du milieu réactionnel en composé de formule (A) ou (B) est comprise entre 0,01 M et 3 M de préférence entre 0,1 M et 1M. La concentration du milieu réactionnel en composé de formule (A') ou (B') est comprise entre 0,01M et 3 M de préférence entre 0,1M et 1M.

les réactifs : le composé de formule (A) ou (B) et le composé de formule (A') ou (B'), la ou les enzymes, et ou le médiateur pour la dite enzyme, et ou l'oxydant sont mélangés, le pH et la température sont ajustés.

### Exemple 12

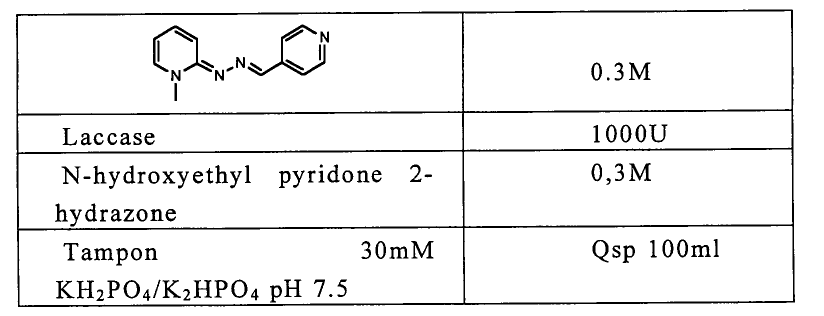

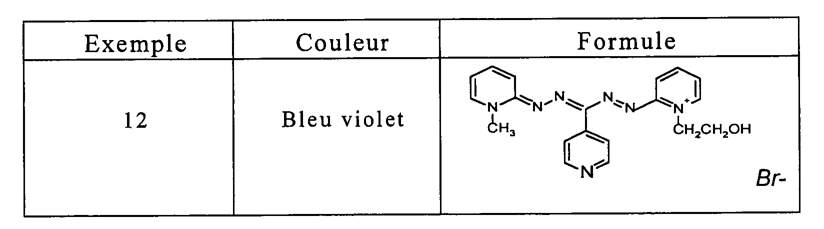

### C) Procédé de synthèse mettant en oeuvre au moins une hydrazone de formule (A) et/ou (B) et un composé de formule R"₂₃C(OR₂₄)₃

L'hydrazone de formule (A) ou (B) utilisée pour la synthèse est mise en réaction en présence d'un équivalent d'un composé de formule R"₂₃C(OR₂₄)₃ et éventuellement d'un solvant protique dont le point d'ébullition varie entre 66°C et 180°C à une température comprise entre 30°C et 150°C sous agitation. Préférentiellement, l'hydrazone de formule (A) ou (B) est mise en réaction en présence de triméthylorthoformiate ou de triéthylorthoacétate et éventuellement d'un solvant de préférence choisi parmi le méthanol et l'éthanol, à une température comprise entre 100°C et 150°C, pendant une période comprise entre 30 et 60 minutes.

Suivant la nature même de l'hydrazone utilisée, un composé de formules (F1), (F2) ou (G1),-(G2) suivantes peut être obtenu :

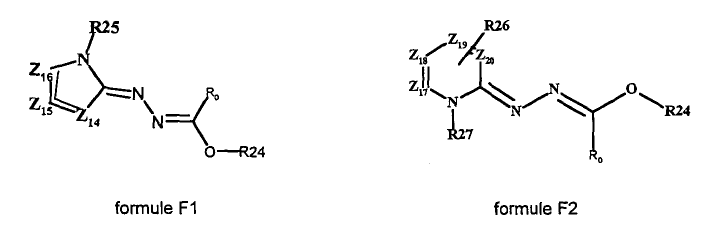

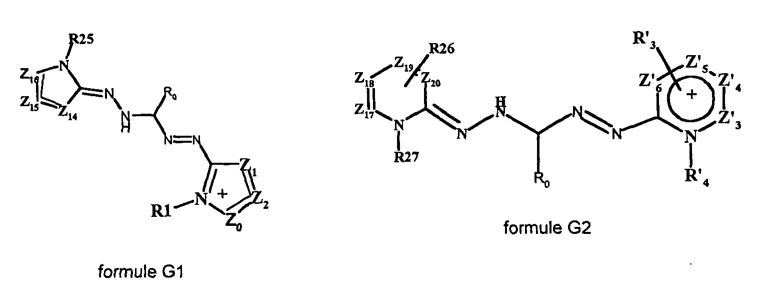

Lorsque les composés de formule (F1) ou (F2) sont obtenus, ceux-ci peuvent par la suite être transformés en composé tétraazapentaméthinique de formule (I) de la façon suivante :

Le composé de formule (F1) ou (F2) et un composé de formule (A) ou (B) sont mis en réaction en présence d'un agent oxydant dans un solvant polaire, à une température comprise entre 0°C et 120°C. Le pH de la solution est compris entre 3 et 11.5. L'agent oxydant est choisi parmi O₂, Na₂O, K₃Fe(CN)₆, MnO₂, H₂O₂, Ag₂O, AgO, NiO₂, NaBO₃, Na₂S₂O₈, CH₃CO₃H, C₆H₅CO₃H, (NH₄)₂Ce(NO₃)₆, PbO₂, Pb(OCOCH₃)₄, SeO₂, CrO₃.2C₅H₄N. Les oxydants préférés seront choisis parmi K₃Fe(CN)₆, MnO₂, H₂O₂, Pb(OCOCH₃)₄. Et de façon préférentielle, les agents oxydants préférentiels seront choisis parmi O₂, K₃Fe(CN)₆, MnO₂, H₂O₂. La réaction est préférentiellement réalisée à une température comprise entre 0°C et 80°C dans un solvant de préférence polaire à un pH compris entre 5 et 9.5

### EXEMPLES DE SYNTHÈSE DES FORMULES G1 ET G2 :

Dans un ballon on a mis sous agitation à 130°C pendant 30 minutes l'hydrazone 1 (0,23 g, 1 mmole) en présence de triéthylorthoformiate (0,15 ml, 1 mmole). Après réaction, le milieu réactionnel a été concentré à sec. On a repris le résidu en ajoutant 10 ml d'éther diisopropylique. Le précipité obtenu a été filtré puis séché au dessicateur. On a obtenu 0,15 g d'une poudre blanche. Rendement : 41%.

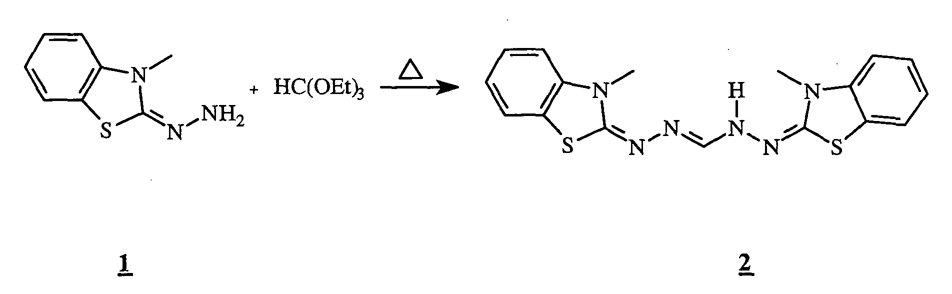

### Analyse :

Poudre blanche

RMN (¹H, 400 Mhz) : 3,63 ppm (s, 2 CH₃) ; 7,2 ppm (m, 2 H); 7,30 ppm (m, 2 H) ; 7,43 ppm (m, 2 H) ; 7,59 ppm (m, 2 H) ; 8,17 ppm (s, 1 H).

### Exemple 13

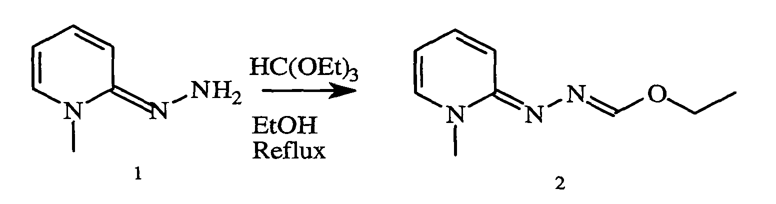

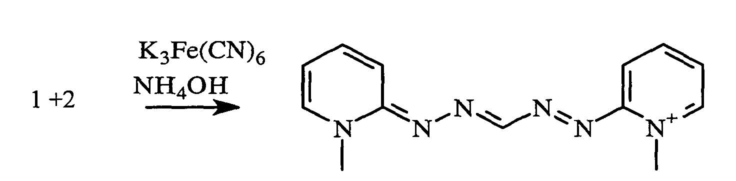

### 1^{ère} étape :

Dans un ballon, on a mis sous agitation à 130°C pendant 12 heures l'hydrazone 1 (4,28 g, 0,01998 mole) en présence de triéthylorthoformiate (50 ml, 0,30 mole). Le milieu réactionnel a été refroidi et le produit a été précipité dans de l'éther diisopropylique. Le précipité a été filtré sous vide puis conservé sous argon. Rendement quantitatif.

### Analyse :

MS(ES⁺) : 180 (MH⁺)

RMN (¹H, 400 MHz : 1,38 ppm (t, CH₃) ; 3,93 ppm (m, CH₃) ; 1 4,30 ppm (t, CH₂) ; 6,97 ppm (m, Har) ; 7,64 ppm(m, Har) ; 8,15-7,97 ppm (m, 2 Har) ; 8,36 ppm (s, CH).

### 2^{ème} étape :

Dans un ballon, on a mis sous agitation le composé 2 (0,1 g, 0,558 mmole), le composé 1 (0,119 g, 0,558 mole) et la pyridine (16,74 ml). Le milieu était hétérogène. Puis on a ajouté au milieu réactionnel successivement l'ammoniaque (0,84 ml, ajout rapide) et une solution aqueuse d'hexacyanoferrate (III) de potassium (K₃Fe(CN)₆/H₂O : 0,81 g / 5,94 ml, ajout goutte à goutte rapide). Une coloration violette puis bleue foncé a été obtenue après 1,5 heure d'agitation. Le produit a été précipité en présence d'éther diisopropylique et d'acétate d'éthyle puis filtré. Il a été par la suite lavé successivement par de l'acétate d'éthyle.

### Analyse :

MS(ES⁺) : 255 (M⁺)
λmax. : 589 nm
ε = 79800

### Exemple 14

### Etape 1

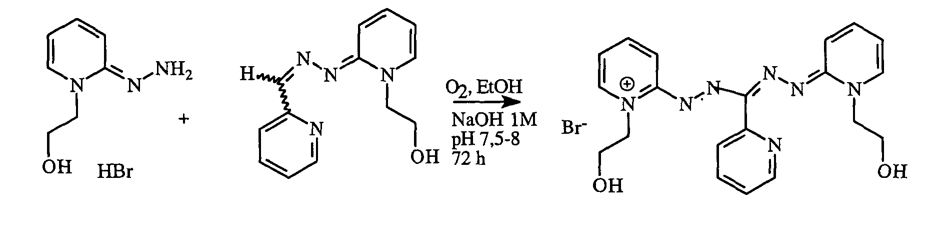

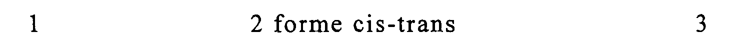

Dans un bécher, 2.42 g de l'hydrazone (1) (0.01 mole) et 3.15 g du composé (2) (0.01 mole) dans 60 ml d'éthanol sont mis sous agitation. Une solution de soude 1M est ajoutée jusqu'à pH 7,5. La réaction est laissée sous agitation 72 heures à l'air. La réaction est suivie par CCM. Une fois celle-ci achevée, 100 ml d'acétone sont ajoutés au milieu réactionnel qui est agité pendant 30minutes. Un précipité foncé apparaît. Celui-ci est filtré puis séché sous vide. Une poudre bleu foncée est obtenue.

### Etape 2

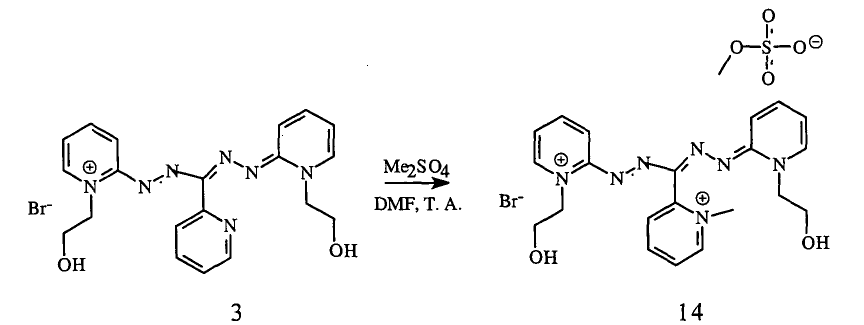

Dans un ballon, 0.5 g du composé 3 obtenu à l'étape précédente est dissous dans 3 ml de DMF. 1 ml de diméthylsulfate est alors ajouté. La réaction est laissée sous agitation pendant 15 heures. Le milieu réactionnel a changé de couleur passant du bleu au fuchsia. Le milieu réactionnel est versé dans de l'éther diisopropylique. Le résiduobtenu est repris à l'éthanol. Par ajout successif d'acétate d'éthyle et d'acétone, un précipité est observé puis filtré et enfin séché au dessicateur. Une poudre fuchsia foncée est obtenue.

La synthèse exposée ci-dessus a permis de préparer les composés suivants :

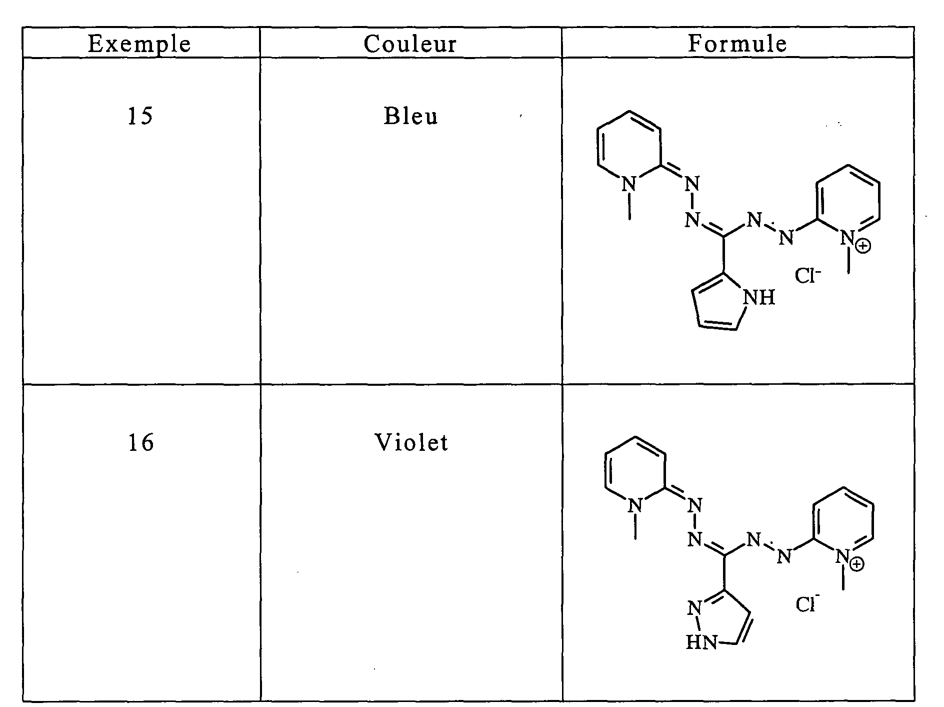

### Exemple d'application

On a préparé la composition de teinture suivante
(teneurs exprimés en grammes de matière active)

| | |
|---|---|
| Colorant de l'exemple 1 selon l'invention | 0,2 |
| Hydroxyéthylcellulose | 0,72 |
| Tensio-actif non ionique : Alkyl(C8/C10 50/50) polyglucoside en solution aqueuse à 60% | 3,00 |
| Alcool benzylique | 4,00 |
| Polyéthylène glycol (8OE | 6,00 |
| Conservateurs | 0,06 |
| Tampon borate q.s. pH 9 | 50 |
| Eau déminéralisée | 100 |

La composition a été appliquée d'une part sur des mèches de cheveux gris naturels à 90% de blancs, d'autre part sur des mèches de cheveux gris permanentés à 90% de blancs, pendant 30 minutes à température ambiante (20°C).

A l'issue du temps de pause, les mèches ont été rincées, shampooinées puis rincées et séchées. Elles ont été teintes dans une nuance bleu-violet très chromatique.

### Exemple de décoloration

### a) Coloration

On applique sur une mèche de cheveux gris naturels à 90% de blancs (0,5g), une solution aqueuse de colorant de l'exemple 1 selon l'invention (à 0,5 % poids), à pH 7 (tampon phosphate de potassium 0.1M) et on laisse pauser pendant 30 minutes à température ambiante (20°C).

La mèche est ensuite rincée, shampooinée puis séchée. Elle a été teinte dans une nuance bleu-violet très chromatique.

### b) Décoloration

On applique 5 ml d'une solution aqueuse d'hydrosulfite de sodium (ou dithionite de sodium) à 5 % en poids tamponnée à pH 7 avec du phosphate de potassium (0,1M), sur 0,5 g de mèche de cheveux obtenue à l'issue de l'étape a), et on laisse pauser 5 minutes à température ambiante (20°C).

La mèche est ensuite rincée, shampooinée puis séchée.

La mèche obtenue est décolorée.

On obtient une mèche de couleur grise, soit la couleur initiale des cheveux avant le traitement de coloration (étape a).

## Revendications

1. Composé tétraazapentaméthinique de formule I' 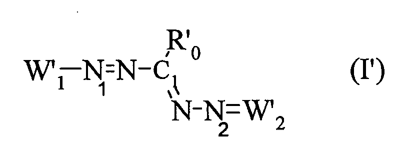 dans laquelle
- W'₁ représente un radical hétéroaromatique cationique de formule (II') ou (III') : 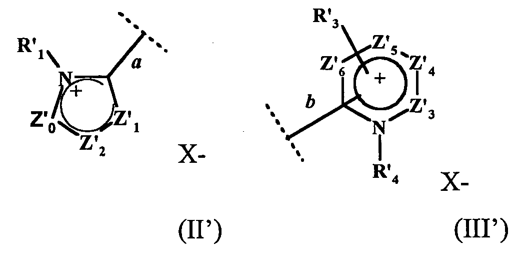
- W'₂ représente un radical hétéroaromatique de formule (IV') ou (V'): 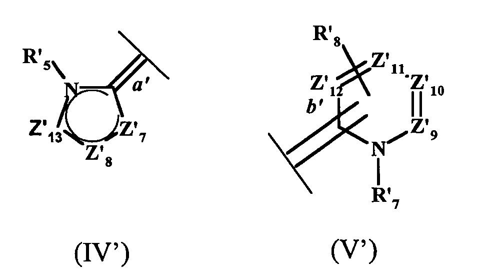
dans lesquelles :
- Z'₀ représente un radical CR'₂, un atome d'azote ou un radical NR'₂₁,
- Z'₁ représente un atome d'oxygène ou un radical NR'₉,
- Z'₂ représente un atome d'azote ou un radical CR'₁₀,
- Z'₃ représente un atome d'azote ou un radical CR'₁₁,
- Z'₄ représente un atome d'azote ou un radical CR'₁₂,
- Z'₅ représente un atome d'azote ou un radical CR'₁₃,
- Z'₆ représente un atome d'azote ou un radical CR' ₁₄,
- Z'₇ représente un atome d'oxygène ou un radical NR'₁₅,
- Z'₈ représente un atome d'azote ou un radical CR'₁₆,
- Z'₉ représente un atome d'azote ou un radical CR'₁₇,
- Z'₁₀ représente un atome d'azote ou un radical CR'₁₈,
- Z'₁₁ représente un atome d'azote ou un radical CR'₁₉,
- Z'₁₂ représente un atome d'azote ou un radical CR'₂₀,
- Z'₁₃ représente un radical CR'₆, un atome d'azote ou un radical NR'₂₂,
- étant entendu que chacun des cycles des formules (II'), (III'), (IV') et (V') ne comportent pas plus de trois atomes d'azote, et que deux des trois atomes d'azote peuvent être contigus,
- la liaison a du radical hétéroaromatique cationique à 5 chaînons de la formule (II') étant reliée à l'atome d'azote N₁ de la formule (I'),
- la liaison b du radical hétéroaromatique cationique à 6 chaînons de la formule (III') étant reliée à l'atome d'azote N₁ de la formule (I').
- la double liaison a' du radical hétéroaromatique à 5 chaînons de la formule (IV') étant reliée à l'atome d'azote N₂ de la formule (I'),
- la double liaison b' du radical hétéroaromatique à 6 chaînons de la formule (V') étant reliée à l'atome d'azote N₂ de la formule (I'),
- la liaison b, reliant le radical hétéroaromatique cationique de la formule III' à l'atome d'azote N₁ de la formule (I'), étant située en position ortho ou para de l'atome d'azote portant le radical R'₄ lorsque Z'₅ représente un radical CR'₁₃ ; la liaison b étant située en position ortho de l'atome d'azote portant le radical R'₄ lorsque Z'₅ représente un atome d'azote,
- la liaison b', reliant le radical hétéroaromatique de la formule V' à l'atome d'azote N₂ de la formule (I'), étant située en position ortho ou para de l'atome d'azote portant le radical R'₇ lorsque Z'₁₁ représente un radical CR'₁₉ ; la liaison b' étant située en position ortho de l'atome d'azote portant le radical R'₇ lorsque Z'₁₁ représente un atome d'azote,
- R'₂, R'₆, R'₁₀ et R'₁₆ représentent, indépendamment l'un de l'autre, un atome d'hydrogène ; un radical alkyle en C₁-C₄ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique ; un radical phényle non substitué ou substitué par un ou deux radicaux choisis parmi les radicaux hydroxy, alcoxy en C₁-C₂, (poly)-hydroxyalcoxy en C₂-C₄, amino, (di)alkylamino en C₁-C₂ ; un radical carboxy ; un radical sulfonylamino,
R'₁, R'₄, R'₅, R'₇, , R'₉, R'₁₅, R'₂₁, et R'₂₂ représentent, indépendamment l'un de l'autre, un radical alkyle en C₁-C₈ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique ;
- R'₀, R'₃, R'₈, R'₁₁, R'₁₂, R'₁₃, R'₁₄, R'₁₇, R'₁₈, R'₁₉, et R'₂₀ représentent, indépendamment l'un de l'autre, un atome d'hydrogène, une chaîne hydrocarbonée en C₁-C₁₆ linéaire ou ramifiée, cette chaîne pouvant être saturée ou insaturée par une à trois insaturations, cette chaîne étant non substituée ou substituée par un à trois radicaux choisis parmi les radicaux hydroxy, alcoxy en C₁-C₂, (poly)-hydroxyalcoxy en C₂-C₄, amino, (di)alkylamino en C₁-C₂ , carboxy, sulfonique, sulfonylamino, (poly)-hydroxyalkylamino en C₂-C₄ ou un atome d'halogène tel que chlore, fluor ou brome ; un radical phényle non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy, sulfonique, sulfonylamino, (poly)-hydroxyalkylamino en C₂-C₄ ou un atome d'halogène tel que chlore, fluor ou brome ; un radical hétéroaryle choisi parmi les radicaux pyrazolyle, pyrrolyle, imidazolyle, thiazolyle, oxazolyle, triazolyle, pyridinyle, pyrimidinyletriazinyle, pyrazinyle, pyridazinyle, en outre cette chaîne hydrocarbonée peut être interrompue par un ou deux atomes d'oxygène, d'azote, de soufre ou par un radical SO₂,
- R'₂ avec R'₁₀, R'₁₁ avec R' ₁₂, R'₆ avec R' ₁₆, et R'₁₇ avec R' ₁₈ pouvant former, indépendamment les uns des autres un cycle aromatique carboné à 5 ou 6 chaînons non substitué ou substitué par un ou deux radicaux hydroxy, amino, (di)alkyl(C₁-C₂)amino, alcoxyen C₁-C₂, (poly)hydroxyalkyl(C₂-C₄)amino, sous réserve que W'₁ et W'₂ ne représentent pas simultanément le même système bicyclique,
- étant entendu que R'₀, R'₃, R'₈, R'₁₁, R'₁₂, R'₁₃, R'₁₄, R'₁₇, R'₁₈, R'₁₉, et R'₂₀ ne comportent pas de liaison peroxyde, ni de radicaux diazo ou nitroso,
- X est un anion organique ou minéral.

2. Composé de formule I' selon la revendication 1 dans laquelle R'₀ représente un atome d'hydrogène ; un radical alkyle en C₁-C₆ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, (poly)hydroxyalkylamino en C₂-C₄ , carboxy ou sulfonique ; un radical phényle , non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂, carboxy, sulfonique ou un atome d'halogène tel que chlore, fluor ou brome ; un radical hétéroaryle éventuellement cationique choisi parmi les radicaux pyrazolyle, pyrrolyle, imidazolyle, thiazolyle, oxazolyle, triazolyle, pyridinyle, pyrimidinyle, triazinyle, pyrazinyle, pyridazinyle.

3. Composé de formule I' selon l'une des revendications 1 ou 2 dans laquelle R'₀ représente un atome d'hydrogène ; un radical méthyle, éthyle, 2-méthoxyéthyle ; un radical phényle non substitué ou substitué par un radical un amino, (di)méthylamino , (di)(2-hydroxyéthyl)amino, un radical hétéroaryle éventuellement cationique choisi parmi les radicaux pyrazolyle, pyrrolyle, imidazolyle, pyridinyle.

4. Composé de formule I' selon l'une des revendications 1 à 3 dans laquelle R'₂, R'₆, R'₁₀ et R'₁₆ représentent un atome d'hydrogène, un radical alkyle en C₁-C₄ non substitué ou substitué par un à deux radicaux choisis parmi les radicaux hydroxy, amino, (di)alkylamino en C₁-C₂ , carboxy, un radical phényl.

5. Composé de formule I' selon l'une des revendications 1 à 4 dans laquelle R'₂, R'₆, R'₁₀ et R'₁₆ représentent préférentiellement un atome d'hydrogène, un radical méthyle, phényle, 2-hydroxyméthyle, un carboxy.

6. Composé de formule I' selon l'une des revendications 1 à 5 dans laquelle R'₁, R'₄, R'₅, R'₇, R'₉, R'₁₅, R'₂₁ et R'₂₂ représentent préférentiellement un radical alkyle en C₁-C₄, non substitué ou substitué par un à deux radicaux choisis parmi les radicaux hydroxy, alcoxy en C₁-C₂, amino, (di)alkylamino en C₁-C₂, carboxy, sulfonique.

7. Composé de formule I' selon l'une des revendications 1 à 6 dans laquelle, R'₁, R'₄, R'₅, R'₇, R'_{9,} R'₁₅, R'₂₁ et R'₂₂ représentent un radical méthyle, éthyle, 2-hydroxyéthyle, 1-carboxyméthyle, 2-carboxyéthyle, 2-sulfonyléthyle.

8. Composé de formule I' selon l'une des revendications 1 à 7 dans laquelle R'_{3,} R'_{8,} R'_{11,} R'_{12,} R'_{13,} R'_{14,} R'_{17,} R'_{18,} R'_{19,} et R'₂₀ représentent indépendamment l'un de l'autre, un atome d'hydrogène ; un radical alkyle en C₁-C₄ linéaire ou ramifié, non substitué ou substitué par un à deux radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique; un radical phényle , non substitué ou substitué par un à deux radicaux choisis parmi les radicaux hydroxy, alcoxy en C₁-C₂, (poly)-hydroxyalcoxy en C₂-C₄, amino, (di)alkylamino en C₁-C₂, carboxy, sulfonique ou un atome d'halogène tel que chlore, fluor ou brome ; un radical sulfonylamino ; un radical (poly)-hydroxyalkylamino en C₂-C₄.

9. Composé de formule I' selon l'une des revendications 1 à 8 dans laquelle R'₃, R'₈, R'₁₁, R' ₁₂, R'₁₃, R'₁₄, R'₁₇, R'₁₈, R'₁₉, et R'₂₀ représentent indépendamment l'un de l'autre un atome d'hydrogène, un radical méthyle, 2-hydroxyméthyle, un carboxy, un radical méthoxy, éthoxy, 2-hydroxyéthyloxy, un radical amino, méthylamino, diméthylamino, 2-hydroxyéthylamino.

10. Composé de formule I' selon l'une des revendications 1 à 9 dans laquelle W'₁ est un radical 2-pyridinium et W'₂ est un radical 2-pyridine, de préférence choisi parmi :
- Chlorure de 2-[5-(N-méthyl-2-pyridinylidène)-1-formazano]-N-méthylpyridinium.
- Chlorure de 2-[5-(N-méthyl-2-pyridinylidène)-3-méthyl-1-formazano]-N-méthylpyridinium.
- Chlorure de 2-[5-(N-méthyl-2-pyridinylidène)-3-éthyl-1-formazano]-N-méthylpyridinium.
- Chlorure de 2-[5-(N-méthyl-2-pyridinylidène)-3-isopropyl-1-formazano]-N-méthylpyridinium.
- Chlorure de 2-[5-(N-méthyl-2-pyridinylidène)-3-phényl-1-formazano]-N-méthylpyridinium.
- Chlorure de 2-[5-(N-méthyl-2-pyridinylidène)-3-(4'-méthoxyphényl)-1-formazano] -N-méthylpyridinium.
- Chlorure de 2-[5-(N-méthyl-2-pyridinylidène)-3-(4'-hydroxyphényl)-1-formazano]-N-méthylpyridinium.
- Chlorure de 2-[5-(N-méthyl-2-pyridinylidène)-3-(4'-N,N-diméthylaminophényl)-1-formazano] -N-méthylpyridinium.
- Chlorure de 2-[5-(4-pyrrolidino-N-méthyl-2-pyridinylidène)-1-formazano]-4-pyrrolidino-N-méthylpyridinium.
- Chlorure de 2-[5-(4-pyrrolidino-N-méthyl-2-pyridinylidène)-3-méthyl-1-formazano]- 4-pyrrolidino-N-méthylpyridinium.
- Chlorure de 2-[5-(4-pyrrolidino-N-méthyl-2-pyridinylidène)-3-éthyl-1-formazano]- 4-pyrrolidino-N-méthylpyridinium.
- Chlorure de 2-[5-(4-pyrrolidino-N-méthyl-2-pyridinylidène)-3-isopropyl-1-formazano]- 4-pyrrolidino-N-méthylpyridinium.
- Chlorure de 2-[5-(4-pyrrolidino-N-méthyl-2-pyridinylidène)-3-phényl-1-formazano]- 4-pyrrolidino-N-méthylpyridinium.
- Chlorure de 2-[5-(4-pyrrolidino-N-méthyl-2-pyridinylidène)-3-(4' -méthoxyphenyl)-1-formazano]-4-pyrrolidino-N-méthylpyridinium.
- Chlorure de 2-[5-(4-pyrrolidino-N-méthyl-2-pyridinylidène)-3-(4'-hydroxyphényl)-1-formazano]- 4-pyrrolidino-N-méthylpyridinium.
- Chlorure de 2-[5-(4-pyrrolidino-N-méthyl-2-pyridinylidène)-3-(4'-N,N-diméthylaminophényl)-1-formazano]- 4-pyrrolidino-N-méthylpyridinium.
- Chlorure de 2-[5-(N-hydroxyéthyl-2-pyridinylidène)-1-formazano]-N-hydroxyéthylpyridinium.
- Chlorure de 2-[5-(N-hydroxyéthyl-2-pyridinylidène)-3-méthyl-1-formazano]-N-hydroxyéthylpyridinium.
- Chlorure de 2-[5-(N-hydroxyéthyl-2-pyridinylidène)-3-ethyl-1-formazano]-N- hydroxyethyl-pyridinium.
- Chlorure de 2-[5-(N- hydroxyethyl -2-pyridinylidene)-3-phényl-1-formazano]-N- hydroxyéthyl-pyridinium.
- Chlorure de 2-[5-(N- hydroxyéthyl -2-pyridinylidène)-3-isopropyl-1-formazano]-N- hydroxyéthyl -pyridinium.
- Chlorure de 2-[5-(N-hydroxyéthyl-2-pyridinylidène)-3-(4'-methoxyphényl)-1-formazano]-N- hydroxyéthyl -pyridinium.
- Chlorure de 2-[5-(N- hydroxyéthyl -2-pyridinylidène)-3-(4'-hydroxyphényl)-1-formazano]-N- hydroxyéthyl -pyridinium.
- Chlorure de 2-[5-(N- hydroxyéthyl -2-pyridinylidène)-3-(4'-N,N-diméthylaminophényl)-1-formazano]-N- hydroxyéthyl-pyridinium.
- Chlorure de 2-[5-(N-carboxyéthyl-2-pyridinylidène)-1-formazano]-N- carboxyéthyl -pyridinium.
- Bromure de 2-[5-(N- hydroxyéthyl -2-pyridinylidène)-3-(2-pyridinyl)-1-formazano]-N- hydroxyéthyl -pyridinium.
- Bromure de 2-[5-(N- hydroxyéthyl -2-pyridinylidène)-3-(méthosulfate de 2-N-méthylpyridinylium)-1-formazano]-N-hydroxyéthyl -pyridinium.
- Chlorure de 2-[5-(N- carboxyéthyl -2-pyridinylidène)-3-méthyl-1-formazano]-N- carboxyéthyl -pyridinium.
- Chlorure de 2-[5-(N- carboxyéthyl -2-pyridinylidène)-3-éthyl-1-formazano]-N- carboxyéthyl -pyridinium.
- Chlorure de 2-[5-(N- carboxyéthyl -2-pyridinylidène)-3-phényl-1-formazano]-N- carboxyéthyl -pyridinium.
- Chlorure de 2-[5-(N- carboxyéthyl -2-pyridinylidène)-3-isopropyl-1-formazano]-N- carboxyéthyl -pyridinium.
- Chlorure de 2-[5-(N- carboxyéthyl -2-pyridinylidène)-3-(4'-méthoxyphényl)-1-formazano]-N- carboxyéthyl -pyridinium.
- Chlorure de 2-[5-(N- carboxyéthyl -2-pyridinylidène)-3-(4'-hydroxyphényl)-1-formazano]-N- carboxyéthyl -pyridinium.
- Chlorure de 2-[5-(N- carboxyéthyl -2-pyridinylidène)-3-(4'-N,N-diméthylaminophényl)-1-formazano]-N-carboxyéthyl-pyridinium.

11. Composé de formule I' selon l'une des revendications 1 à 9 dans laquelle W'₁ est un radical 4-pyridinium et W'₂ est un radical 4-pyridine de préférence choisi parmi :
- Chlorure de 4-[5-(N-méthyl-4-pyridinylidène)-1-formazano]-N-méthylpyridinium.
- Chlorure de 4-[5-(N-méthyl-4-pyridinylidène)-3-méthyl-1-formazano] -N-méthylpyridinium.
- Chlorure de 4-[5-(N-méthyl-4-pyridinylidène)-3-éthyl-1-formazano]-N-méthylpyridinium.
- Chlorure de 4-[5-(N-méthyl-4-pyridinylidène)-3-isopropyl-1-formazano]-N-méthylpyridinium.
- Chlorure de 4-[5-(N-méthyl-4-pyridinylidène)-3-phényl-1-formazano]-N-méthylpyridinium.
- Chlorure de 4-[5-(N-méthyl-4-pyridinylidène)-3-(4'-méthoxyphényl)-1-formazano]-N-méthylpyridinium.
- Chlorure de 4-[5-(N-méthyl-4-pyridinylidène)-3-(4'-hydroxyphényl)-1-formazano]-N-méthylpyridinium.
- Chlorure de 4-[5-(N-méthyl-4-pyridinylidène)-3-(4'-N,N-diméthylaminophényl)-1-formazano]-N-méthylpyridinium.
- Chlorure de 4-[5-(N-hydroxyéthyl-4-pyridinylidène)-1-formazano] -N-hydroxyéthylpyridinium.
- Chlorure de 4-[5-(N-hydroxyéthyl-4-pyridinylidène)-3-méthyl-1-formazano]-N-hydroxyéthylpyridinium.
- Chlorure de 4-[5-(N-hydroxyéthyl-4-pyridinylidène)-3-éthyl-1-formazano]-N- hydroxyéthyl-pyridinium.
- Chlorure de 4-[5-(N- hydroxyéthyl -4-pyridinylidène)-3-phényl-1-formazano]-N- hydroxyéthyl-pyridinium.
- Chlorure de 4-[5-(N- hydroxyéthyl -4-pyridinylidène)-3-isopropyl-1-formazano]-N- hydroxyéthyl -pyridinium.
- Chlorure de 4-[5-(N- hydroxyéthyl-4-pyridinylidène)-3-(4'-méthoxyphényl)-1-formazano]-N- hydroxyéthyl -pyridinium.
- Chlorure de 4-[5-(N- hydroxyéthyl -4-pyridinylidène)-3-(4'-hydroxyphényl)-1-formazano]-N- hydroxyéthyl -pyridinium.
- Chlorure de 4-[5-(N- hydroxyéthyl -4-pyridinylidène)-3-(4'-N,N-diméthylaminophényl)-1-formazano]-N- hydroxyéthyl-pyridinium.
- Chlorure de 4-[5-(N-carboxyéthyl-4-pyridinylidène)-1-formazano]-N- carboxyéthyl -pyridinium.
- Chlorure de 4-[5-(N- carboxyéthyl -4-pyridinylidène)-3-méthyl-1-formazano]-N- carboxyéthyl -pyridinium.
- Chlorure de 4-[5-(N- carboxyéthyl -4-pyridinylidene)-3-ethyl-1-formazano]-N- carboxyéthyl-pyridinium.
- Chlorure de 4-[5-(N- carboxyéthyl-4-pyridinylidène)-3-phényl-1-formazano]-N-carboxyéthyl-pyridinium.
- Chlorure de 4-[5-(N-carboxyéthyl-4-pyridinylidène)-3-isopropyl-1-formazano]-N-carboxyéthyl-pyridinium.
- Chlorure de 4-[5-(N- carboxyéthyl-4-pyridinylidène)-3-(4'-méthoxyphényl)-1-formazano]-N-carboxyéthyl-pyridinium.
- Chlorure de 4-[5-(N-carboxyéthyl-4-pyridinylidène)-3-(4'-hydroxyphényl)-1-formazano]-N-carboxyéthyl-pyridinium.
- Chlorure de 4-[5-(N-carboxyéthyl-4-pyridinylidène)-3-(4'-N,N-diméthylaminophényl)-1-formazano]-N-carboxyéthyl-pyridinium.

12. Composé de formule I' selon l'une des revendications 1 à 9 dans laquelle W'₁ est un radical 2-imidazolium et W'₂ est un radical 2-imidazole de préférence choisi parmi:
- Chlorure de 2-[5-(1,3-diméthyl-2-imidazolidène)-1-formazano]- 1,3-diméthyl-imidazolium.
- Chlorure de 2-[5-(1,3-diméthyl-2-imidazolidène)-3-méthyl-1-formazano]- 1,3-diméthyl-imidazolium.
- Chlorure de 2-[5-(1,3-diméthyl-2-imidazolidène)-3-éthyl-1-formazano]- 1,3-diméthyl-imidazolium.
- Chlorure de 2-[5-(1,3-diméthyl-2-imidazolidene)-3-isopropyl-1-formazano]- 1,3-diméthyl-imidazolium.
- Chlorure de 2-[5-(1,3-diméthyl-2-imidazolidène)-3-phényl-1-formazano]-1,3-diméthyl-imidazolium.
- Chlorure de 2-[5-(1,3-diméthyl-2-imidazolidène)-3-(4'-méthoxyphényl)- 1 -formazano] - 1 ,3-diméthyl-imidazolium.
- Chlorure de 2-[5-(1,3-diméthyl-2-imidazolidène)-3-(4'-hydroxyphényl)-1-formazano]-1,3-diméthyl-imidazolium.
- Chlorure de 2-[5-(1,3-diméthyl-2-imidazolidène)-3-(4'-N,N-diméthylaminophényl)-1-formazano]-1,3-diméthyl-imidazolium.
- Chlorure de 2-[5-(1,3-dihydroxyéthyl-2-imidazolidène)-1-formazano]-1,3-dihydroxyéthylimidazolium.
- Chlorure de 2-[5-(1,3-dihydroxyéthyl-2-imidazolidène)-3-méthyl-1-formazano]-1,3-dihydroxyéthylimidazolium.
- Chlorure de 2-[5-(1,3-dihydroxyéthyl-2-imidazolidène)-3-éthyl-1-formazano]-1,3-dihydroxyéthyl -imidazolium.
- Chlorure de 2-[5-(1,3-dihydroxyéthyl -2-imidazolidène)-3-phényl-1 -formazano]-1,3-dihydroxyéthyl -imidazolium.
- Chlorure de 2-[5-(1,3-dihydroxyéthyl -2-imidazolidène)-3-isopropyl-1-formazano]-1,3-dihydroxyéthyl -imidazolium.
- Chlorure de 2-[5-(1,3-dihydroxyéthyl-2-imidazolidène)-3-(4'-méthoxyphényl)-1-formazano]-1,3-dihydroxyéthyl -imidazolium.
- Chlorure de 2-[5-(1,3-dihydroxyéthyl -2-imidazolidène)-3-(4'-hydroxyphényl)-1-formazano]-1,3-dihydroxyéthyl -imidazolium.
- Chlorure de 2-[5-(1,3-dihydroxyéthyl -2-imidazolidène)-3-(4'-N,N-diméthylaminophényl)-1-formazano]-1,3-dihydroxyéthyl-imidazolium.
- Chlorure de 2-[5-(1,3-dicarboxyéthyl-2-imidazolidène)-1-formazano]-1,3-dicarboxyéthyl -imidazolium.
- Chlorure de 2-[5-(1,3-dicarboxyéthyl -2-imidazolidène)-3-méthyl- 1 -formazano]-1,3-dicarboxyéthyl -imidazolium.
- Chlorure de 2-[5-(1,3-dicarboxyéthyl -2-imidazolidène)-3-éthyl-1-formazano]-1,3-dicarboxyéthyl -imidazolium.
- Chlorure de 2-[5-(1,3-dicarboxyéthyl -2-imidazolidène)-3-phényl- 1 -formazano]-1,3-dicarboxyéthyl -imidazolium.
- Chlorure de 2-[5-(1,3-dicarboxyéthyl -2-imidazolidène)-3-isopropyl-1-formazano]-1,3-dicarboxyéthyl -imidazolium.
- Chlorure de 2-[5-(1,3-dicarboxyéthyl -2-imidazolidène)-3-(4'-méthoxyphényl)-1-formazano]- 1,3-dicarboxyéthyl -imidazolium.
- Chlorure de 2-[5-(1,3-dicarboxyéthyl -2-imidazolidène)-3-(4'-hydroxyphényl)-1-formazano]-1,3-dicarboxyéthyl -imidazolium.
- Chlorure de 2-[5-(1,3-dicarboxyéthyl -2-imidazolidène)-3-(4'-N,N-diméthylaminophényl)-1-formazano]-1,3-dicarboxyéthyl-imidazolium.

13. Composé de formule I' selon l'une des revendications 1 à 9 dans laquelle W'₁ est un radical 5-pyrazolium et W'₂ est un radical 5-pyrazole de préférence choisi parmi:
- Chlorure de 5-[5-(1,2-diméthyl-5-pyrazolidène)-1-formazano]-1,2-diméthyl-pyrazolinium.
- Chlorure de 5-[5-(1,2-diméthyl-5-pyrazolidène)-3-méthyl-1-formazano]-1,2-diméthyl-pyrazolinium.
- Chlorure de 5-[5-(1 ,2-diméthyl-5-pyrazolidène)-3-éthyl- 1-formazano]-1,2-diméthyl-pyrazolinium.
- Chlorure de 5-[5-(1,2-diméthyl-5-pyrazolidène)-3-isopropyl-1-formazano]-1,2-diméthyl-pyrazolinium.
- Chlorure de 5-[5-(1,2-diméthyl-5-pyrazolidène)-3-phényl-1-formazano]-1,2-diméthyl-pyrazolinium.
- Chlorure de 5-[5-(1,2-diméthyl-5-pyrazolidène)-3-(4'-méthoxyphényl)-1-formazano]- 1 ,2-diméthyl-pyrazolinium.
- Chlorure de 5-[5-(1,2-diméthyl-5-pyrazolidène)-3-(4'-hydroxyphényl)-1-formazano]-1,2-diméthyl-pyrazolinium.
- Chlorure de 5-[5-(1,2-diméthyl-5-pyrazolidène)-3-(4'-N,N-dimethylaminophényl)-1-formazano]-1,2-diméthyl-pyrazolinium.
- Chlorure de 5-[5-(1,2-dihydroxyéthyl-5-pyrazolidène)-1-formazano]-1,2-dihydroxyéthylpyrazolinium.
- Chlorure de 5-[5-(1,2-dihydroxyéthyl-5-pyrazolidène)-3-méthyl-1-formazano]-1,2-dihydroxyéthylpyrazolinium.
- Chlorure de 5-[5-(1,2-dihydroxyéthyl-5-pyrazolidène)-3-éthyl-1-formazano]-1,2-dihydroxyéthyl -pyrazolinium.
- Chlorure de 5-[5-(1,2-dihydroxyéthyl -5-pyrazolidène)-3-phényl-1-formazano]-1,2-dihydroxyéthyl -pyrazolinium.
- Chlorure de 5-[5-(1,2-dihydroxyéthyl -5-pyrazolidène)-3-isopropyl-1-formazano]-1,2-dihydroxyéthyl -pyrazolinium.
- Chlorure de 5-[5-(1,2-dihydroxyéthyl-5-pyrazolidène)-3-(4'-méthoxyphényl)-1-formazano]-1,2-dihydroxyéthyl -pyrazolinium.
- Chlorure de 5-[5-(1,2-dihydroxyéthyl -5-pyrazolidène)-3-(4'-hydroxyphényl)-1-formazano]-1,2-dihydroxyéthyl -pyrazolinium.
- Chlorure de 5-[5-(1,2-dihydroxyéthyl -5-pyrazolidène)-3-(4'-N,N-diméthylaminophényl)-1-formazano]-1,2-dihydroxyéthyl-pyrazolinium.
- Chlorure de 5-[5-(1,2-dicarboxyéthyl-5-pyrazolidène)-1-formazano]-1,2-dicarboxyéthyl -pyrazolinium.
- Chlorure de 5-[5-(1,2-dicarboxyéthyl -5-pyrazolidène)-3-méthyl-1 -formazano]-1,2-dicarboxyéthyl -pyrazolinium.
- Chlorure de 5-[5-(1,2-dicarboxyéthyl -5-pyrazolidène)-3-éthyl- 1 -formazano]-1,2-dicarboxyéthyl -pyrazolinium.
- Chlorure de 5-[5-(1,2-dicarboxyéthyl -5-pyrazolidène)-3-phényl-1-formazano]-1 ,2-dicarboxyéthyl -pyrazolinium.
- Chlorure de 5-[5-(1,2-dicarboxyéthyl -5-pyrazolidène)-3-isopropyl-1-formazano]-1,2-dicarboxyéthyl -pyrazolinium.
- Chlorure de 5-[5-(1,2-dicarboxyéthyl-5-pyrazolidène)-3-(4'-méthoxyphényl)- 1 -formazano]-1,2-dicarboxyéthyl -pyrazolinium.
- Chlorure de 5-[5-(1,2-dicarboxyéthyl -5-pyrazolidène)-3-(4'-hydroxyphényl)- 1 -formazano]- 1 ,2-dicarboxyéthyl -pyrazolinium.
- Chlorure de 5-[5-(1,2-dicarboxyéthyl -5-pyrazolidène)-3-(4'-N,N-diméthylaminophényl)- 1 -formazano]- 1 ,2-dicarboxyéthyl
- pyrazolinium.

14. Composé de formule I' selon l'une des revendications 1 à 9 dans laquelle W'₁ est un radical 2-benzimidazolium et W'₂ est un radical 2-pyridine de préférence choisi parmi :
- Chlorure de 2-[5-(N-methyl-2-pyridinylidène)-1-formazano]-1,3-diméthyl-benzimidazolium.
- Chlorure de 2-[5-(N-methyl-2-pyridinylidène)-3-méthyl-1-formazano]-1,3-diméthyl-benzimidazolium.
- Chlorure de 2-[5-(N-méthyl-2-pyridinylidène)-3-éthyl-1-formazano]-1,3-diméthyl-benzimidazolium.
- Chlorure de 2-[5-(N-méthyl-2-pyridinylidène)-3-isopropyl-1-formazano]-1,3-diméthyl-benzimidazolium.
- Chlorure de 2-[5-(N-méthyl-2-pyridinylidène)-3-phényl-1-formazano]-1,3-diméthyl-benzimidazolium.
- Chlorure de 2-[5-(N-méthyl-2-pyridinylidène)-3-(4'-méthoxyphényl)-1-formazano]-1,3-diméthyl-benzimidazolium.
- Chlorure de 2-[5-(N-methyl-2-pyridinylidène)-3-(4'-hydroxyphényl)-1-formazano]-1,3-diméthyl-benzimidazolium.
- Chlorure de 2-[5-(N-méthyl-2-pyridinylidène)-3-(4'-N,N-diméthylaminophényl)-1 -formazano]-1,3-diméthyl-benzimidazolium.
- Chlorure de 2-[5-(N-hydroxyéthyl-2-pyridinylidène)-1-formazano]-1,3-dihydroxyéthylbenzimidazolium.
- Chlorure de 2-[5-(N-hydroxyéthyl-2-pyridinylidène)-3-méthyl-1 -formazano]-1,3-dihydroxyéthylbenzimidazolium.
- Chlorure de 2-[5-(N-hydroxyéthyl-2-pyridinylidène)-3-ethyl-1 -formazano]-1,3-dihydroxyéthyl -benzimidazolium.
- Chlorure de 2-[5-(N-hydroxyéthyl-2-pyridinylidène)-3-phényl-1-formazano]-1,3-dihydroxyéthyl -benzimidazolium.
- Chlorure de 2-[5-(N-hydroxyéthyl-2-pyridinylidène)-3-isopropyl-1-formazano]-1,3-dihydroxyéthyl -benzimidazolium.
- Chlorure de 2-[5-(N-hydroxyéthyl-2-pyridinylidène)-3-(4'-méthoxyphényl)-1-formazano]-1,3-dihydroxyéthyl -benzimidazolium.
- Chlorure de 2-[5-(N-hydroxyéthyl-2-pyridinylidène)-3-(4'-hydroxyphényl)-1-formazano]-1,3-dihydroxyéthyl -benzimidazolium.
- Chlorure de 2-[5-(N-hydroxyéthyl-2-pyridinylidène)-3-(4'-N,N-diméthylaminophényl)-1-formazano]-1,3-dihydroxyéthyl-benzimidazolium.
- Chlorure de 2-[5-(N-carboxyéthyl-2-pyridinylidène)-1-formazano]-1,3-dicarboxyéthyl -benzimidazolium.
- Chlorure de 2-[5-(N-carboxyéthyl-2-pyridinylidène)-3-méthyl-1-formazano]-1,3-dicarboxyéthyl -benzimidazolium.
- Chlorure de 2-[5-(N-carboxyéthyl-2-pyridinylidène)-3-ethyl-1-formazano]-1,3-dicarboxyéthyl -benzimidazolium.
- Chlorure de 2-[5-(N-carboxyéthyl-2-pyridinylidène)-3-phenyl-1-formazano]-1,3-dicarboxyéthyl -benzimidazolium.
- Chlorure de 2-[5-(N-carboxyéthyl-2-pyridinylidène)-3-isopropyl-1-formazano]-1,3-dicarboxyéthyl -benzimidazolium.
- Chlorure de 2-[5-(N-carboxyéthyl-2-pyridinylidène)-3-(4'-méthoxyphényl)-1-formazano]-1,3-dicarboxyéthyl -benzimidazolium.
- Chlorure de 2-[5-(N-carboxyéthyl-2-pyridinylidène)-3-(4'-hydroxyphényl)- 1 -formazano]-1,3-dicarboxyéthyl -benzimidazolium.
- Chlorure de 2-[5-(N-carboxyéthyl-2-pyridinylidène)-3-(4'-N,N-diméthylaminophényl)-1-formazano]-1,3-dicarboxyéthyl-benzimidazolium.

15. Composé de formule I' selon l'une des revendications 1 à 9 dans laquelle W'₁ est un radical 2-benzimidazolium et W'₂ est un radical 4-pyridine de préférence choisi parmi:
- Chlorure de 2-[5-(N-méthyl-4-pyridinylidène)-1-formazano]-1,3-diméthyl-benzimidazolium.
- Chlorure de 2-[5-(N-méthyl-4-pyridinylidène)-3-méthyl-1-formazano]-1,3-diméthyl-benzimidazolium.
- Chlorure de 2-[5-(N-méthyl-4-pyridinylidène)-3-éthyl-1-formazano]-1,3-diméthyl-benzimidazolium.
- Chlorure de 2-[5-(N-méthyl-4-pyridinylidène)-3-isopropyl-1-formazano]-1,3-diméthyl-benzimidazolium.
- Chlorure de 2-[5-(N-méthyl-4-pyridinylidène)-3-phényl-1-formazano]-1,3-diméthyl-benzimidazolium.
- Chlorure de 2-[5-(N-méthyl-4-pyridinylidène)-3-(4'-méthoxyphényl)-1-formazano]-1,3-diméthyl-benzimidazolium.
- Chlorure de 2-[5-(N-méthyl-4-pyridinylidène)-3-(4'-hydroxyphényl)-1-formazano]-1,3-diméthyl-benzimidazolium.
- Chlorure de 2-[5-(N-méthyl-4-pyridinylidène)-3-(4'-N,N-diméthylaminophényl)- 1 -formazano]-1,3-diméthyl-benzimidazolium.
- Chlorure de 2-[5-(N-hydroxyéthyl-4-pyridinylidène)-1-formazano]-1,3-dihydroxyéthylbenzimidazolium.
- Chlorure de 2-[5-(N-hydroxyéthyl-4-pyridinylidène)-3-méthyl-1-formazano]-1,3-dihydroxyéthylbenzimidazolium.
- Chlorure de 2-[5-(N-hydroxyéthyl-4-pyridinylidène)-3-éthyl-1-formazano]-1,3-dihydroxyéthyl-benzimidazolium.
- Chlorure de 2-[5-(N-hydroxyéthyl-4-pyridinylidène)-3-phényl-1-formazano]-1,3-dihydroxyéthyl -benzimidazolium.
- Chlorure de 2-[5-(N-hydroxyéthyl-4-pyridinylidène)-3-isopropyl-1-formazano]-1,3-dihydroxyéthyl -benzimidazolium.
- Chlorure de 2-[5-(N-hydroxyéthyl-4-pyridinylidène)-3-(4'-méthoxyphényl)-1-formazano]-1,3-dihydroxyéthyl -benzimidazolium.
- Chlorure de 2-[5-(N-hydroxyéthyl-4-pyridinylidène)-3-(4'-hydroxyphényl)- 1 -formazano]-1,3-dihydroxyéthyl -benzimidazolium.
- Chlorure de 2-[5-(N-hydroxyéthyl-4-pyridinylidène)-3-(4'-N,N-diméthylaminophényl)-1-formazano]-1,3-dihydroxyéthyl-benzimidazolium.
- Chlorure de 2-[5-(N-carboxyéthyl-4-pyridinylidène)-1-formazano]-1,3-dicarboxyéthyl -benzimidazolium.
- Chlorure de 2-[5-(N-carboxyéthyl-4-pyridinylidène)-3-méthyl-1 -formazano]-1,3-dicarboxyéthyl -benzimidazolium.
- Chlorure de 2-[5-(N-carboxyéthyl-4-pyridinylidène)-3-éthyl-1-formazano]-1,3-dicarboxyéthyl -benzimidazolium.
- Chlorure de 2-[5-(N-carboxyéthyl-4-pyridinylidène)-3-phényl- 1 -formazano]- 1,3-dicarboxyéthyl -benzimidazolium.
- Chlorure de 2-[5-(N-carboxyéthyl-4-pyridinylidène)-3-isopropyl- 1 -formazano]-1,3-dicarboxyéthyl -benzimidazolium.
- Chlorure de 2-[5-(N-carboxyéthyl-4-pyridinylidène)-3-(4'-méthoxyphényl)-1-formazano]-1,3-dicarboxyéthyl -benzimidazolium.
- Chlorure de 2-[5-(N-carboxyéthyl-4-pyridinylidène)-3-(4'-hydroxyphényl)-1-formazano]-1,3-dicarboxyéthyl -benzimidazolium.
- Chlorure de 2-[5-(N-carboxyéthyl-4-pyridinylidène)-3-(4'-N,N-diméthylaminophényl)-1-formazano]-1,3-dicarboxyéthyl-benzimidazolium.

16. Composé de formule I' selon l'une des revendications 1 9 dans laquelle W'₁ est un radical 2-imidazolium et W'₂ est un radical 2-pyridine de préférence choisi parmi:
- Chlorure de 2-[5-(N-méthyl-2-pyridinylidène)-1-formazano]-1,3-diméthyl-imidazolium.
- Chlorure de 2-[5-(N-méthyl-2-pyridinylidène)-3-méthyl-1-formazano]-1,3-diméthyl-imidazolium.
- Chlorure de 2-[5-(N-méthyl-2-pyridinylidène)-3-éthyl-1-formazano]- 1,3-diméthyl-imidazolium.
- Chlorure de 2-[5-(N-méthyl-2-pyridinylidène)-3-isopropyl-1-formazano]- 1,3-diméthyl-imidazolium.
- Chlorure de 2-[5-(N-méthyl-2-pyridinylidène)-3-phényl-1-formazano]-1,3-diméthyl-imidazolium.
- Chlorure de 2-[5-(N-méthyl-2-pyridinylidène)-3-(4'-méthoxyphényl)-1 -formazano]-1,3-diméthyl-imidazolium.
- Chlorure de 2-[5-(N-méthyl-2-pyridinylidène)-3-(4'-hydroxyphényl)-1-formazano]-1,3-diméthyl-imidazolium.
- Chlorure de 2-[5-(N-méthyl-2-pyridinylidène)-3-(4'-N,N-diméthylaminophényl)-1-formazano]-1,3-diméthyl-imidazolium.
- Chlorure de 2-[5-(N-hydroxyéthyl-2-pyridinylidène)-1-formazano]- 1 ,3-dihydroxyéthylimidazolium.
- Chlorure de 2-[5-(N-hydroxyéthyl-2-pyridinylidène)-3-méthyl-1 -formazano]-1,3-dihydroxyéthylimidazolium.
- Chlorure de 2-[5-(N-hydroxyéthyl-2-pyridinylidène)-3-éthyl-1 -formazano]- 1,3-dihydroxyéthyl -imidazolium.
- Chlorure de 2-[5-(N-hydroxyéthyl-2-pyridinylidène)-3-phényl-1-formazano]-1,3-dihydroxyéthyl -imidazolium.
- Chlorure de 2-[5-(N-hydroxyéthyl-2-pyridinylidène)-3-isopropyl-1-formazano]-1,3-dihydroxyéthyl -imidazolium.
- Chlorure de 2-[5-(N-hydroxyéthyl-2-pyridinylidène)-3-(4'-méthoxyphényl)-1-formazano]-1,3-dihydroxyéthyl -imidazolium.
- Chlorure de 2-[5-(N-hydroxyéthyl-2-pyridinylidène)-3-(4'-hydroxyphényl)-1-formazano]-1,3-dihydroxyéthyl -imidazolium.
- Chlorure de 2-[5-(N-hydroxyéthyl-2-pyridinylidène)-3-(4'-N,N-diméthylaminophényl)-1-formazano]-1,3-dihydroxyéthyl-imidazolium.
- Chlorure de 2-[5-(N-carboxyéthyl-2-pyridinylidène)-1-formazano]-1,3-dicarboxyéthyl -imidazolium.
- Chlorure de 2-[5-(N-carboxyéthyl-2-pyridinylidène)-3-méthyl-1-formazano]-1,3-dicarboxyéthyl -imidazolium.
- Chlorure de 2-[5-(N-carboxyéthyl-2-pyridinylidène)-3-éthyl-1-formazano]-1,3-dicarboxyéthyl -imidazolium.
- Chlorure de 2-[5-(N-carboxyéthyl-2-pyridinylidène)-3-phényl-1-formazano]-1,3-dicarboxyéthyl -imidazolium.
- Chlorure de 2-[5-(N-carboxyéthyl-2-pyridinylidène)-3-isopropyl- 1 -formazano]-1,3-dicarboxyéthyl -imidazolium.
- Chlorure de 2-[5-(N-carboxyéthyl-2-pyridinylidène)-3-(4'-méthoxyphényl)-1-formazano]-1,3-dicarboxyéthyl -imidazolium.
- Chlorure de 2-[5-(N-carboxyéthyl-2-pyridinylidène)-3-(4'-hydroxyphényl)-1-formazano]-1,3-dicarboxyéthyl -imidazolium.
- Chlorure de 2-[5-(N-carboxyéthyl-2-pyridinylidène)-3-(4'-N,N-diméthylaminophényl)-1-formazano]-1,3-dicarboxyéthyl-imidazolium.

17. Composé de formule I' selon l'une des revendications 1 à 9 dans laquelle W'₁ est un radical 2-imidazolium et W'₂ est un radical 4-pyridine de préférence choisi parmi :
- Chlorure de 2-[5-(N-méthyl-4-pyridinylidène)-1-formazano]-1,3-diméthyl-imidazolium.
- Chlorure de 2-[5-(N-méthyl-4-pyridinylidène)-3-méthyl-1-formazano]- 1 ,3-diméthyl-imidazolium.
- Chlorure de 2-[5-(N-méthyl-4-pyridinylidène)-3-éthyl-1-formazano]- 1 ,3-diméthyl-imidazolium.
- Chlorure de 2-[5-(N-méthyl-4-pyridinylidène)-3-isopropyl-1-formazano]-1,3-diméthyl-imidazolium.
- Chlorure de 2-[5-(N-méthyl-4-pyridinylidène)-3-phényl-1-formazano]-1,3-diméthyl-imidazolium.
- Chlorure de 2-[5-(N-méthyl-4-pyridinylidène)-3-(4'-méthoxyphényl)-1-formazano]-1,3-diméthyl-imidazolium.
- Chlorure de 2-[5-(N-méthyl-4-pyridinylidène)-3-(4'-hydroxyphényl)-1-formazano]-1,3-diméthyl-imidazolium.
- Chlorure de 2-[5-(N-méthyl-4-pyridinylidène)-3-(4'-N,N-diméthylaminophényl)- 1 -formazano]-1,3-diméthyl-imidazolium.
- Chlorure de 2-[5-(N-hydroxyéthyl-4-pyridinylidène)-1-formazano]-1,3-dihydroxyéthylimidazolium.
- Chlorure de 2-[5-(N-hydroxyéthyl-4-pyridinylidène)-3-méthyl-1-formazano]-1, 3-dihydroxyéthylimidazolium.
- Chlorure de 2-[5-(N-hydroxyéthyl-4-pyridinylidène)-3-éthyl-1 -formazano] - 1,3 -dihydroxyéthyl -imidazolium.
- Chlorure de 2-[5-(N-hydroxyéthyl-4-pyridinylidène)-3-phényl-1 -formazano]-1,3-dihydroxyéthyl -imidazolium.
- Chlorure de 2-[5-(N-hydroxyéthyl-4-pyridinylidène)-3-isopropyl-1 -formazano]-1,3-dihydroxyéthyl -imidazolium.
- Chlorure de 2-[5-(N-hydroxyéthyl-4-pyridinylidène)-3-(4'-méthoxyphényl)-1 -formazano] -1,3-dihydroxyéthyl -imidazolium.
- Chlorure de 2-[5-(N-hydroxyéthyl-4-pyridinylidène)-3-(4'-hydroxyphényl)-1-formazano]-1,3-dihydroxyéthyl -imidazolium.
- Chlorure de 2-[5-(N-hydroxyéthyl-4-pyridinylidène)-3-(4'-N,N-dimethylaminophényl)-1-formazano]-1,3-dihydroxyéthyl-imidazolium.
- Chlorure de 2-[5-(N-carboxyéthyl-4-pyridinylidène)-1-formazano]-1,3-dicarboxyéthyl -imidazolium.
- Chlorure de 2-[5-(N-carboxyéthyl-4-pyridinylidène)-3-méthyl-1-formazano]-1,3-dicarboxyéthyl -imidazolium.
- Chlorure de 2-[5-(N-carboxyéthyl-4-pyridinylidène)-3-éthyl-1-formazano]-1,3-dicarboxyéthyl -imidazolium.
- Chlorure de 2-[5-(N-carboxyéthyl-4-pyridinylidène)-3-phényl-1-formazano]-1,3-dicarboxyéthyl -imidazolium.
- Chlorure de 2-[5-(N-carboxyéthyl-4-pyridinylidène)-3-isopropyl-1-formazano]-1,3-dicarboxyéthyl -imidazolium.
- Chlorure de 2-[5-(N-carboxyéthyl-4-pyridinylidène)-3-(4'-méthoxyphényl)- 1 -formazano]-1,3-dicarboxyéthyl -imidazolium.
- Chlorure de 2-[5-(N-carboxyéthyl-4-pyridinylidène)-3-(4'-hydroxyphényl)- 1 -formazano]-1,3-dicarboxyéthyl -imidazolium.
- Chlorure de 2-[5-(N-carboxyéthyl-4-pyridinylidène)-3-(4'-N,N-diméthylaminophényl)-1-formazano]-1,3-dicarboxyéthyl-imidazolium.

18. Procédé de préparation du composé de formule I' selon l'une des revendications 1 à 17 **caractérisé en ce qu'**on fait réagir au moins deux équivalents d'au moins un hydrazone choisie parmi les hydrazones de formule A et les hydrazones de formule B : 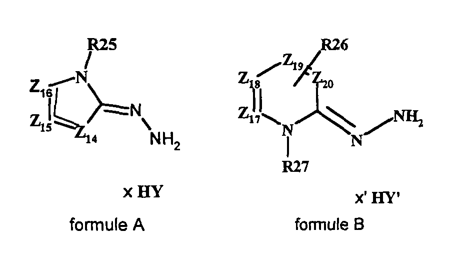 dans lesquelles Z₁₄, Z₁₅, Z₁₆, R₂₅ ont respectivement les mêmes significations que Z'₇, Z'₈, Z'₁₃, R'₅ de la formule IV' définie à la revendication 1,
Y étant un anion organique ou minéral, x étant un entier allant de 1 à 3,
Z₁₇, Z₁₈, Z₁₉, Z₂₀, R₂₆, R₂₇ ont respectivement les mêmes significations que Z'₉, Z'₁₀, Z'₁₁, Z'₁₂, R'₈, R'₇ de la formule V' définie à la revendication 1,
Y' étant un anion organique ou minéral, x' étant un entier allant de 1 à 3,
avec un équivalent d'un aldéhyde de formule R₂₃CHO, R₂₃ ayant la même signification que R'₀ de la formule I' définie à la revendication 1.

19. Procédé de préparation selon la revendication 18 dans lequel l'aldéhyde de formule R₂₃CHO est obtenu à partir d'un précurseur d'aldéhyde de formule R'₂₃CH₂OH en présence d'un système oxydant, R'₂₃ ayant la même signification que R'₀ de la formule I' définie à la revendication 1.

20. Utilisation en tant que colorant direct dans des ou pour la fabrication de compositions tinctoriales pour fibres kératiniques, en particulier pour fibres kératiniques humaines et plus particulièrement des cheveux, d'au moins un composé tétraazapentaméthinique de formule (I) 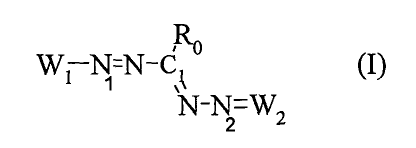 dans laquelle
- W₁ représente un radical hétéroaromatique cationique de formule (II) ou (III) : 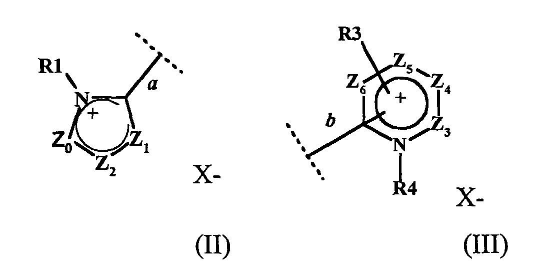
- W₂ représente un radical hétéroaromatique de formule (IV) ou (V): 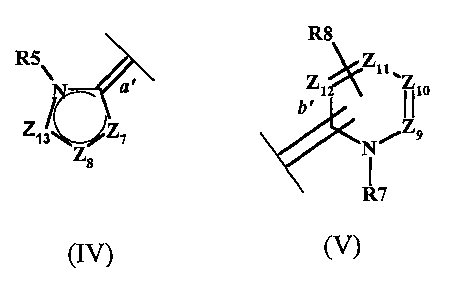
dans lesquelles :
- Z₀ représente un radical CR₂, un atome d'azote ou un radical NR₂₁,
- Z₁ représente un atome d'oxygène, de soufre ou un radical NR₉,
- Z₂ représente un atome d'azote ou un radical CR₁₀,
- Z₃ représente un atome d'azote ou un radical CR₁₁,
- Z₄ représente un atome d'azote ou un radical CR₁₂,
- Z₅ représente un atome d'azote ou un radical CR₁₃,
- Z₆ représente un atome d'azote ou un radical CR₁₄,
- Z₇ représente un atome d'oxygène, de soufre ou un radical NR₁₅,
- Z₈ représente un atome d'azote ou un radical CR₁₆,
- Z₉ représente un atome d'azote ou un radical CR₁₇,
- Z₁₀ représente un atome d'azote ou un radical CR₁₈,
- Z₁₁ représente un atome d'azote ou un radical CR₁₉,
- Z₁₂ représente un atome d'azote ou un radical CR₂₀,
- Z₁₃ représente un radical CR₆, un atome d'azote ou un radical NR₂₂,
- étant entendu que chacun des cycles des formules (II), (III), (IV) et (V) ne comportent pas plus de trois atomes d'azote, et que deux des trois atomes d'azote peuvent être contigus,
- la liaison a du radical hétéroaromatique cationique à 5 chaînons de la formule (II) étant reliée à l'atome d'azote N₁ de la formule (I),
- la liaison b du radical hétéroaromatique cationique à 6 chaînons de la formule (III) étant reliée à l'atome d'azote N₁ de la formule (I).
- la double liaison a' du radical hétéroaromatique à 5 chaînons de la formule (IV) étant reliée à l'atome d'azote N₂ de la formule (I),
- la double liaison b' du radical hétéroaromatique à 6 chaînons de la formule (V) étant reliée à l'atome d'azote N₂ de la formule (I),
- la liaison b, reliant le radical hétéroaromatique cationique de la formule III à l'atome d'azote N₁ de la formule (I), étant située en position ortho ou para de l'atome d'azote portant le radical R₄ lorsque Z₅ représente un radical CR₁₃ ; la liaison b étant située en position ortho de l'atome d'azote portant le radical R₄ lorsque Z₅ représente un atome d'azote,
- la liaison b', reliant le radical hétéroaromatique de la formule V à l'atome d'azote N₂ de la formule (I), étant située en position ortho ou para de l'atome d'azote portant le radical R₇ lorsque Z₁₁ représente un radical CR₁₉ ; la liaison b' étant située en position ortho de l'atome d'azote portant le radical R₇ lorsque Z₁₁ représente un atome d'azote,
- R₂ R₆, R₁₀ et R₁₆ représentent, indépendamment l'un de l'autre, un atome d'hydrogène ; un radical alkyle en C₁-C₄ linéaire ou ramifié, non substitué ou substitué, par un à trois radicaux choisis parmi les radicaux hydroxy, alcoxy en C₁-C₂, (poly)-hydroxyalcoxy en C₂-C₄, amino, (di)alkylamino en C₁-C₂, carboxy ou sulfonique ; un radical phényle non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂ ; un radical carboxy ; un radical sulfonylamino,
- R₁, R₄, R₅, R₇, R₉, R₁₅, R₂₁, et R₂₂ représentent, indépendamment l'un de l'autre, un radical alkyle en C₁-C₈ linéaire ou ramifié, non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂ , carboxy ou sulfonique ;
- R₀, R₃, R₈, R₁₁, R₁₂, R₁₃, R₁₄, R₁₇, R₁₈, R₁₉, et R₂₀ représentent, indépendamment l'un de l'autre, un atome d'hydrogène, une chaîne hydrocarbonée en C₁-C₁₆ linéaire ou ramifiée, cette chaîne pouvant être saturée ou insaturée par une à trois insaturations, cette chaîne étant non substituée ou substituée par un à trois radicaux choisis parmi les radicaux hydroxy, alcoxy en C₁-C₂, (poly)-hydroxyalcoxy en C₂-C₄, amino, (di)alkylamino en C₁-C₂ , carboxy, sulfonique, sulfonylamino, (poly)-hydroxyalkylamino en C₂-C₄ ou un atome d'halogène tel que chlore, fluor ou brome ; un radical phényle non substitué ou substitué par un à trois radicaux choisis parmi les radicaux hydroxy, alcoxy en C₁-C₂, (poly)-hydroxyalcoxy en C₂-C₄, amino, (di)alkylamino en C₁-C₂ , carboxy, sulfonique, sulfonylamino, (poly)-hydroxyalkylamino en C₂-C₄ ou un atome d'halogène tel que chlore, fluor ou brome ; un radical hétéroaryle choisi parmi les radicaux pyrazolyle, pyrrolyle, imidazolyle, thiazolyle, oxazolyle, triazolyle, pyridinyle, pyrimidinyle, triazinyle, pyrazinyle, pyridazinyle, en outre cette chaîne hydrocarbonée peut être interrompue par un ou deux atomes d'oxygène, d'azote, de soufre ou par un radical SO₂,
- étant entendu que R₀, R₃, R₈, R₁₁, R₁₂, R₁₃, R₁₄, R₁₇, R₁₈, R₁₉, et R₂₀ ne comportent pas de liaison peroxyde, ni de radicaux diazo ou nitroso,
- R₂ avec R₁₀, R₁₁ avec R₁₂, R₆ avec R₁₆, et R₁₇ avec R₁₈ pouvant former indépendamment les uns des autres un cycle aromatique carboné à 5 ou 6 chaînons non substitué ou substitué par un ou deux radicaux hydroxy, amino, (di)alkyl C₁-C₂ amino, alcoxy C₁-C₂, (poly)hydroxyalkyl C₂-C₄ amino,
- X est un anion organique ou minéral.

21. Procédé de préparation d'un composé tétraazapentaméthinique de formule (I) tel que défini à la revendication 20, **caractérisé en ce que** l'on fait réagir au moins deux équivalents d'au moins un hydrazone choisie parmi les hydrazones de formule A et les hydrazones de formule B : 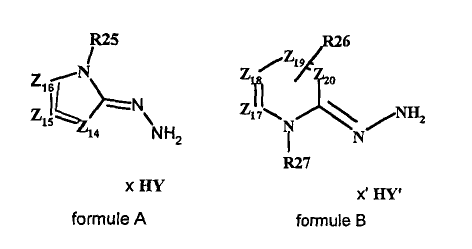 dans lesquelles Z₁₄, Z₁₅, Z₁₆, R₂₅ ont respectivement les mêmes significations que Z₇, Z₈, Z₁₃, R₅ de la formule IV définie à la revendication 20,
Y étant un anion organique ou minéral, x étant un entier allant de 1 à 3,
Z₁₇, Z₁₈, Z₁₉, Z₂₀, R₂₆, R₂₇ ont respectivement les mêmes significations que Z₉, Z₁₀, Z₁₁, Z₁₂, R₈, R₇ de la formule V définie à la revendication 20,
Y' étant un anion organique ou minéral, x' étant un entier allant de 1 à 3,
avec un équivalent d'un précurseur d'aldéhyde de formule R'₂₃CH₂OH, R'₂₃ ayant la même signification que R₀ de la formule (I) définie à la revendication 20, et d'au moins une enzyme capable de générer un aldéhyde à partir du précurseur d'aldéhyde de formule R'₂₃CH₂OH.

22. Composition pour la teinture des fibres kératiniques en particulier des fibres kératiniques humaines et plus particulièrement des cheveux comprenant, dans un milieu approprié pour la teinture, au moins un composé de formule I tel que défini à la revendication 20 ou de formule (I') selon l'une quelconque des revendications 1 à 17 en tant que colorant direct.

23. Composition selon la revendication 22 **caractérisée par le fait que** dans la formule I, W₁ est un radical 2-benzimidazolium et W₂ est un radical 2-benzimidazole.

24. Composition selon la revendication 23, **caractérisée par le fait que** les composés de formule (I) sont choisis parmi :
- Chlorure de 2-[5-(1,3-diméthyl-2-benzimidazolidène)-1-formazano]-1,3-diméthyl-benzimidazolium.
- Chlorure de 2-[5-(1,3-diméthyl-2-benzimidazolidène)-3-méthyl-1-formazano]-1,3-diméthyl-benzimidazolium.
- Chlorure de 2-[5-(1,3-diméthyl-2-benzimidazolidène)-3-éthyl-1-formazano]-1,3-diméthyl-benzimidazolium.
- Chlorure de 2-[5-(1,3-diméthyl-2-benzimidazolidène)-3-isopropyl-1-formazano]-1,3-diméthyl-benzimidazolium.
- Chlorure de 2-[5-(1,3-diméthyl-2-benzimidazolidène)-3-phényl-1-formazano]-1,3-diméthyl-benzimidazolium.
- Chlorure de 2-[5-(1,3-diméthyl-2-benzimidazolidène)-3-(4'-méthoxyphényl)- 1 -formazano] - 1 ,3 -diméthyl-benzimidazolium.
- Chlorure de 2-[5-(1,3-diméthyl-2-benzimidazolidène)-3-(4'-hydroxyphényl)- 1 -formazano] - 1 ,3-diméthyl-benzimidazolium.
- Chlorure de 2-[5-(1,3-diméthyl-2-benzimidazolidène)-3-(4'-N,N-diméthylaminophényl)-1-formazano]-1,3-diméthyl-benzimidazolium.
- Chlorure de 2-[5-(1,3-dihydroxyéthyl-2-benzimidazolidène)-1-formazano]-1,3-dihydroxyéthylbenzimidazolium.
- Chlorure de 2-[5-(1,3-dihydroxyéthyl-2-benzimidazolidène)-3-méthyl- 1 -formazano]- 1,3-dihydroxyéthylbenzimidazolium.
- Chlorure de 2-[5-(1,3-dihydroxyéthyl-2-benzimidazolidène)-3-éthyl-1-formazano]-1,3-dihydroxyéthyl -benzimidazolium.
- Chlorure de 2-[5-(1,3-dihydroxyéthyl -2-benzimidazolidène)-3-phényl-1-formazano]-1,3-dihydroxyéthyl -benzimidazolium.
- Chlorure de 2-[5-(1,3-dihydroxyéthyl -2-benzimidazolidène)-3-isopropyl-1-formazano]-1,3-dihydroxyéthyl -benzimidazolium.
- Chlorure de 2-[5-(1,3-dihydroxyéthyl-2-benzimidazolidène)-3-(4'-méthoxyphényl)-1-formazano]-1,3-dihydroxyéthyl-benzimidazolium.
- Chlorure de 2-[5-(1,3-dihydroxyéthyl-2-benzimidazolidène)-3-(4'-hydroxyphényl)-1-formazano]-1,3-dihydroxyéthyl -benzimidazolium.
- Chlorure de 2-[5-(1,3-dihydroxyéthyl -2-benzimidazolidène)-3-(4'-N,N-diméthylaminophényl)-1-formazano]-1,3-dihydroxyéthyl-benzimidazolium.
- Chlorure de 2-[5-(1,3-dicarboxyéthyl-2-benzimidazolidène)-1-formazano]-1,3-dicarboxyéthyl -benzimidazolium.
- Chlorure de 2-[5-(1,3-dicarboxyéthyl -2-benzimidazolidène)-3-méthyl-1-formazano]-1,3-dicarboxyéthyl -benzimidazolium.
- Chlorure de 2-[5-(1,3-dicarboxyéthyl -2-benzimidazolidène)-3-éthyl- 1 -formazano]-1,3-dicarboxyéthyl -benzimidazolium.
- Chlorure de 2-[5-(1,3-dicarboxyéthyl -2-benzimidazolidène)-3-phényl- 1 -formazano]-1,3-dicarboxyéthyl -benzimidazolium.
- Chlorure de 2-[5-(1,3-dicarboxyéthyl -2-benzimidazolidène)-3-isopropyl-1-formazano]-1,3-dicarboxyéthyl-benzimidazolium.
- Chlorure de 2-[5-(1,3-dicarboxyéthyl-2-benzimidazolidène)-3-(4'-méthoxyphényl)-1-formazano]-1,3-dicarboxyéthyl-benzimidazolium.
- Chlorure de 2-[5-(1,3-dicarboxyéthyl-2-benzimidazolidène)-3-(4'-hydroxyphényl)- 1 -formazano]- 1 ,3-dicarboxyéthyl -benzimidazolium.
- Chlorure de 2-[5-(1,3-dicarboxyéthyl -2-benzimidazolidène)-3-(4'-N,N-dimethylaminophényl)-1-formazano]-1,3-dicarboxyéthyl-benzimidazolium.

25. Composition selon l'une des revendications 22 à 24, **caractérisée par le fait que** le ou les composés de formule 1 sont présents dans une concentration allant de 0,001 à 5% et de préférence de 0,05 à 2% en poids par rapport au poids total de la composition.

26. Composition selon l'une des revendications 22 à 25, **caractérisée par le fait qu'**elle comprend au moins une base d'oxydation choisie dans le groupe formé par les paraphénylènediamines, les bis-phénylalkylènediamines, les para-aminophénols, les ortho-aminophénols, les bases hétérocycliques et leurs sels d'addition.

27. Composition selon la revendication 26, **caractérisée par le fait que** la ou les bases d'oxydation sont présentes à une concentration allant de 0,001 à 10% en poids par rapport au poids total de la composition.

28. Composition selon l'une des revendications 22 à 27, **caractérisée par le fait qu'**elle comprend au moins un coupleur choisi dans le groupe formé par les métaphénylènediamines, les métaaminophénols, les métadiphénols, les coupleurs naphtaléniques, les coupleurs hétérocycliques ainsi que leurs sels d'addition.

29. Composition selon la revendication 28, **caractérisée par le fait que** le ou les coupleurs sont présents à une concentration allant de 0,001 à 10 % en poids par rapport au poids total de la composition.

30. Composition selon l'une des revendications 22 à 29, **caractérisée par le fait qu'**elle comprend au moins un colorant direct différents des composés de formule I.

31. Composition selon la revendication 30 **caractérisée, par le fait que** le ou les colorants directs additionnels sont présents à une concentration allant de 0,001 à 20% en poids par rapport au poids total de la composition.

32. Composition selon l'une quelconque des revendications 22 à 31, **caractérisée par le fait qu'**elle comprend en outre un agent oxydant.

33. Procédé de teinture des fibres kératiniques et en particulier des fibres kératiniques humaines et plus particulièrement les cheveux, **caractérisée par le fait qu'**on applique sur lesdites fibres au moins une composition selon l'une des revendications 22 à 31, pendant une durée suffisante pour développer la coloration désirée.

34. Procédé selon la revendication 33, **caractérisé par le fait que** la composition tinctoriale contient un agent oxydant.

35. Procédé selon la revendication 34, **caractérisé par le fait que** l'agent oxydant est mélangé au moment de l'emploi à la composition tinctoriale.

36. Procédé selon l'une quelconque des revendications 34 ou 35, **caractérisé par le fait que** l'agent oxydant est appliqué sur les fibres, simultanément ou séquentiellement à la composition tinctoriale.

37. Procédé de teinture d'oxydation des fibres kératiniques, en particulier des fibres kératiniques humaines et plus particulièrement des cheveux, **caractérisé par le fait qu'**on applique sur les fibres au moins une composition tinctoriale telle que définie à l'une quelconque des revendications 22 à 31, comprenant en outre au moins une base d'oxydation et optionnellement au moins un coupleur, en présence d'un agent oxydant.

38. Procédé selon la revendication 37, **caractérisé par le fait que** l'agent oxydant est mélangé au moment de l'emploi à la composition tinctoriale.

39. Procédé selon la revendication 37, **caractérisé par le fait que** l'agent oxydant est appliqué sur les fibres, simultanément ou séquentiellement à la composition tinctoriale.

40. Procédé de décoloration d'un composé de formule (I) selon la revendication 20, ou d'un composé (I') selon l'une des revendications 1 à 17, **caractérisé en ce qu'**une composition de décoloration comprenant au moins un agent réducteur choisi parmi les réducteurs comprenant du soufre est appliquée sur le composé de formule (I) ou (I').

41. Procédé de décoloration des fibres kératiniques colorées, en particulier des fibres kératiniques humaines et plus particulièrement des cheveux, **caractérisé par le fait qu'**il comprend les étapes suivantes :
a)on applique sur lesdites fibres colorées par une composition tinctoriale telle que définie à l'une quelconque des revendications 22 à 31, une composition de décoloration, comprenant, dans un milieu approprié, au moins un agent réducteur choisi parmi les réducteurs soufrés,
b) on laisse pauser pendant une durée suffisante pour obtenir la décoloration,
c) on rince éventuellement les fibres,
d) on lave lesdites fibres, on les rince, puis on les sèche.

42. Procédé selon la revendication 40 ou 41, **caractérisé par le fait que** les agents réducteurs soufrés sont choisis parmi les composés portant une fonction thiol, sulfure ou sulfite.

43. Procédé selon l'une quelconque des revendications 40 à 42, **caractérisé par le fait que** les thiols sont choisis parmi l'acide thioglycolique, l'acide thiolactique, leurs sels de métal alcalin ou alcalino-terreux et leurs esters ; le (β-mercaptoéthanol ; la cystéine, la cystéamine et leurs dérivés ; l'homocystéine et l'un de ses sels ; les mercaptoaldéhydes ; la pénicillamine ; le glutathion ; seuls ou en mélanges.

44. Procédé selon l'une quelconque des revendications 40 à 43, **caractérisé par le fait que** les sulfites sont choisis parmi les sulfites, métabisulfites ou hydrosulfites de métaux alcalins, alcalino-terreux ou d'ammonium, ainsi que leurs mélanges.

45. Procédé selon l'une quelconque des revendications 40 à 44, **caractérisé par le fait que** le sulfite de sodium et le métabisulfite de sodium sont utilisés.

46. Procédé selon l'une quelconque des revendications 40 à 45, **caractérisé par le fait que** les sulfures sont choisis parmi les sulfures ou les disulfures telle que la cystine.

47. Procédé selon l'une quelconque des revendications 40 à 46, **caractérisé par le fait que** la teneur en agent réducteur dans la composition est comprise entre 0,01 et 10 % en poids de la composition de décoloration, de préférence entre 0,1 et 5 % en poids de la composition de décoloration.
